(19) Europäisches Patentamt
European Patent Office
Office européen des brevets

(11) **EP 2 176 359 B1**

(12) **EUROPÄISCHE PATENTSCHRIFT**

(45) Veröffentlichungstag und Bekanntmachung des Hinweises auf die Patenterteilung:
**30.11.2011 Patentblatt 2011/48**

(51) Int Cl.:
***C09C 1/00*** (2006.01)     ***C09C 1/62*** (2006.01)
***C09C 1/64*** (2006.01)     ***A61K 8/00*** (2006.01)

(21) Anmeldenummer: **08785037.6**

(22) Anmeldetag: **24.07.2008**

(86) Internationale Anmeldenummer:
**PCT/EP2008/006084**

(87) Internationale Veröffentlichungsnummer:
**WO 2009/012995 (29.01.2009 Gazette 2009/05)**

(54) **MEHRSCHICHTIGE METALLISCHE EFFEKTPIGMENTE, VERFAHREN ZU DEREN HERSTELLUNG UND VERWENDUNG**

MULTI-LAYER METALLIC EFFECT PIGMENTS, PROCESS FOR THEIR PREPARATION AND USE

PIGMENTS A EFFET METALLIQUES MULTICOUCHES, LEUR PROCEDE DE PRODUCTION ET LEUR UTILISATION

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **24.07.2007 DE 102007034928**

(43) Veröffentlichungstag der Anmeldung:
**21.04.2010 Patentblatt 2010/16**

(73) Patentinhaber: **Eckart GmbH**
**91235 Hartenstein (DE)**

(72) Erfinder:
• **FISCHER, Martin**
**92281 Königstein (DE)**
• **SCHNEIDER, Ralph**
**91207 Lauf (DE)**
• **GEISSLER, Bernhard**
**90592 Schwarzenbruck (DE)**
• **HERZING, Wolfgang**
**91233 Neunkirchen am Sand (DE)**
• **BLEISTEINER, Jasmin**
**91241 Kirchensittenbach (DE)**

(74) Vertreter: **Walcher, Armin**
**Louis, Pöhlau, Lohrentz & Segeth**
**Postfach 3055**
**90014 Nürnberg (DE)**

(56) Entgegenhaltungen:
**EP-A- 0 747 453     EP-A- 1 522 606**
**WO-A-2007/093401     DE-A1-102004 063 433**
**US-A- 5 766 827**

**Beschreibung**

Die vorliegende Erfindung betrifft ein mehrschichtiges, spiegelartiges, metallisch glänzendes Effektpigment sowie Verfahren zur Herstellung derselben. Die Erfindung betrifft ferner die Verwendung dieser Effektpigmente in verschiedenen Anwendungsbereichen.

Metalleffektpigmente werden seit vielen Jahren in Beschichtungen zur Erzeugung eines metallischen Effektes eingesetzt.

Klassische Metalleffektpigmente bestehen aus plättchenförmigen Metallpigmenten, deren Wirkung auf der gerichteten Reflexion von einfallendem Licht an flächig ausgebildeten und im jeweiligen Anwendungsmedium parallel ausgerichteten Metallteilchen beruht.

Typische Anwendungsgebiete der Metalleffektpigmente sind die Lackindustrie, insbesondere die Automobilindustrie, die Druckindustrie und die Kunststoffindustrie.

Der Metallic-Effekt wird durch bestimmte Größen beschrieben. Diese sind u.a. die Brillanz (Sparkle und metallischer Glanz), die Helligkeit sowie der Flop (Helligkeitsänderung in Abhängigkeit vom Einfalls- und/oder Betrachtungswinkel) und die Deckfähigkeit. Bei farbigen Metallicbeschichtungen kommt noch die Bunttonsättigung und der Farbflop ("two-tone") hinzu.

Der Glanz wird nach dem Verhältnis von reflektiertem zu gestreutem Licht in Relation zu einem Standard bestimmt.

Der Metalleffekt wird wesentlich u.a. von der Teilchenform und dem Formfaktor (Verhältnis mittlerer Teilchendurchmesser zu mittlerer Teilchendicke) der Pigmente, der Teilchendicke sowie von deren Oberflächenrauhigkeit, der Partikelgröße, der Teilchengrößenverteilung und von der Pigmentorientierung parallel zur Oberfläche des Lackes oder Kunststoffes beeinflusst.

Während bei Pigmentteilchen mit größeren Durchmessern mit gleichmäßiger Form eine stärkere Reflexion auftritt, die sich in einer hohen metallischen Brillanz, einer verbesserten Helligkeit und starkem Flop äußert, ist der Streuanteil bei Pigmenten mit geringeren Teilchendurchmessern sehr hoch, was zu einer guten Deckung führt.

Die Deckfähigkeit wird jedoch vor allem durch die Dicke der Metallpigmente bestimmt. Je dünner die Metallpigmente sind, desto besser wird ihre spezifische Deckfähigkeit, also die auf das Gewicht bezogene Deckfähigkeit.

Es besteht ein großes Interesse seitens der Druck-, Lack-, Kunststoff- und Kosmetikindustrie an farbigen und insbesondere an goldglänzenden Metallpigmenten. Goldähnliche Produkte besitzen eine hohe ästhetische Qualität und verleihen den entsprechend beschichteten, bedruckten oder eingefärbten Materialien ein wertvolles Aussehen.

Jedoch auch schwarze Metalleffekte erfreuen sich in letzter Zeit zunehmend an Beliebtheit. So ist die Farbe schwarz derzeit zur Modefarbe im Automobilbereich geworden. Dabei handelt es sich um Mischungen von Schwarzpigmenten mit klassischen Aluminiumpigmenten. Metallpigmente, die von sich aus schwarz sind und noch einen hohen Glanz aufweisen, sind kommerziell noch nicht bekannt geworden.

Seit langem bekannt sind die so genannten Goldbronzepulver, die überwiegend aus Kupfer/Zink-Legierungen bestehen und je nach Zusammensetzung unterschiedliche Farbtöne von Rotgold bis Reichgold aufweisen können (Pigment Handbook, Vol. 1, 1973, S. 807 ff, Wiley-Interscience). Goldbronzepigmente werden durch Verdüsen einer schmelzflüssigen Kupfer/Zink-Legierung und anschließendem Vermahlen des bei der Verdüsung entstandenen Grießes hergestellt. Beim Mahlprozess werden die Legierungspartikel plättchenförmig verformt und zerkleinert. In der Praxis werden Goldbronzepigmente überwiegend durch trockene Vermahlung erhalten. Um Kaltverschweißungen zu vermeiden, wird dem eingesetzten Grieß Schmiermittel wie beispielsweise Stearinsäure zugegeben. Unregelmäßigkeiten der Oberfläche und der Kanten der Metallplättchen wirken glanzmindernd. Diese auf klassischem Weg hergestellten Metalleffektpigmente besitzen neben einer ausgeprägten Teilchendickenverteilung auch Teilchendicken von weit über 100 nm.

Für höherwertige Anwendungen wurden besonders dünne Aluminiumpigmente entwickelt, welche über PVD-Verfahren hergestellt werden.

Seit längerer Zeit sind Metallpigmente, die durch PVD-Verfahren hergestellt werden, bekannt. Sie zeichnen sich durch extrem hohen Glanz, einem enormen Deckvermögen und einzigartige optische Eigenschaften aus. Bedingt durch ihre geringe Dicke von ca. 30 bis 70 nm und ihre extrem glatten Oberflächen neigen sie dazu, sich nach ihrer Applikation sehr dicht an den Untergrund anzuschmiegen. Dies führt bei einem sehr glatten Untergrund nahezu zu einem spiegelähnlichen Erscheinungsbild.

Kommerziell sind bisher von den reinen Metallpigmenten lediglich Aluminiumpigmente bekannt geworden. Beispiele hierfür sind Metalure® (hergestellt von der Fa. Avery Dennison, vertrieben von Fa. ECKART), Decomet® (Fa. Schlenk) oder Metasheen® (Fa. Ciba).

Derartige Pigmente stehen für den Farbton "Silber" in seiner höchsten Ausführungsform.

Hochwertige farbige Metalleffekte erhält man in der Regel durch Abmischung von PVD-Aluminiumpigmenten mit Farbstoffen und/oder Farbpigmenten. So können beispielsweise hochwertige Goldtöne durch Abmischung der PVD-Aluminiumpigmente mit gelben Farbstoffen oder Farbpigmenten erzeugt werden. Derartige Abmischungen weisen jedoch Nachteile auf: so lassen sich diese Abmischungen insbesondere nicht auf saugenden Untergründen applizieren, da hier eine Trennung von Metallpigment und Farbstoff erfolgt. Bei Anwendungen, bei denen hohe Lichtechtheiten gefragt sind, versagen diese Systeme oft aufgrund der mangelnden Lichtechtheit des Farbpigmentes oder des Farbstoffes.

Über PVD-Verfahren hergestellte Pigmente auf Basis metallischer Schichten werden in der US 2,839,378 näher beschrieben.

Dort wird die Herstellung spiegelähnlicher Pigmente mit extrem dünnen Schichtdicken beschrieben, die auf ein Substrat, welches mit einem "release layer" versehen ist, aufgedampft werden. Nach dem Aufbringen der Metallschichten und Ablösung des Films werden die Pigmente mittels mechanischer Beanspruchung auf die gewünschte Teilchengröße zerkleinert.

Die Anwendung derartig hergestellter Pigmente in Coating-Formulierungen wird detailliert in US 2,941,894 beschrieben. Dort werden die hohen Reflektivitäten, die niedrige Pigmentierungshöhe und das hohe Deckvermögen bzw. die Deckfähigkeit der Pigmente hervorgehoben.

Der Prozess zur Herstellung von metallischen Pigmenten mittels Aufdampfverfahren mit einer Dicke von 35 bis 45 nm wird in US 4,321,087 genauer beschrieben und beinhaltet das Aufbringen eines Releasecoats, den Metallisierungsprozess, den Ablöseprozess in einem Lösemittelbad, das Konzentrieren der Partikel und die Ultraschallzerkleinerung auf die gewünschte Pigmentgröße.

Diese einschichtigen Metallpigmente weisen eine begrenzte Farbtonvielfalt auf. Es besteht ein Bedarf an neuen Farbeffekten mit optisch hochwertigen Metallpigmenten.

Die WO2004/026971 und WO2004/026972 betreffen einschichtige hochglänzende goldfarbene Metalleffektpigmente, die aus einer kupferbasierenden Legierung und weiteren metallischen Legierungsbestandteilen bestehen und durch Ablösen und Zerkleinern von im Vakuum abgeschiedenen Metallfilmen hergestellt werden. Die Nachteile derartiger Pigmente sind die begrenzte Farbtonvielfalt. Die Herstellung von Pigmenten unter Verwendung von Cu bzw. Zn als Schwermetall führt zu Pigmenten mit einer hohen Dichte und damit verbunden zu einer relativ geringen Deckfähigkeit sowie zu Absetzproblemen in gewissen Formulierungen. Ein weiterer Nachteil besteht in der starken Korrosionsempfindlichkeit dieser Legierungspigmente.

Des weiteren sind mittels PVD-Verfahren (Physical Vapour Deposition) hergestellte mehrschichtige Effektpigmente seit langem bekannt. Sie wurden erstmals in US 3,438,796 beschrieben. Hier sind fünfschichtige Interferenzpigmente mit einer zentralen, reflektierenden Aluminiumschicht, beidseitig flankiert von einer $SiO_2$-Schicht mit einer Schichtdicke von 100 bis 600 nm und abschließend semitransparenten Absorberschichten aus Aluminium beansprucht. Die zentrale Aluminiumschicht soll reflektierend wirken, d.h. hierzu sind Schichtdicken von über 60 nm nötig. Die äußeren Absorberschichten aus Aluminium hingegen müssen, um semitransparente Eigenschaften zu besitzen, Schichtdicken von unter 40 nm besitzen. Weiterhin wurde in diesem Patent ein Interferenzpigment mit einem dreischichtigen Aufbau beschrieben, bei dem eine zentrale $SiO_2$-Schicht von zwei dünnen, semitransparenten Aluminiumschichten flankiert ist.

In der US 5,571,624 wird eine Farbe beansprucht, die mehrfarbige Interferenzpigmente enthält. Diese besitzen eine zentrale metallische reflektierende Schicht, die beidseitig von Schichtpaketen flankiert wird, die wiederum aus einem Dielektrikum und einer semiopaken Metallschicht besteht, wobei die Dielektrikumsschicht dem Reflektorkern zugewandt ist. Auch hier ist für die zentrale metallische reflektierende Schicht, um wirklich opak zu sein, eine Mindestschichtdicke von 35 bis 40 nm erforderlich. Die Dielektrikumsschichten sollten mindestens eine optische Schichtdicke von zwei Viertel einer ausgewählten Wellenlänge von 400 nm besitzen. Dies entspricht beispielsweise für eine $SiO_2$-Schicht mit einem Brechungsindex von 1,55 einer geometrischen Mindestschichtdicke von 310 nm.

Goldfarbige Metallpigmente hoher Qualität sind in der DE 10 2004 063433 A1 offenbart. Hier werden mehrschichtige PVD-Pigmente beschrieben, die eine zentrale Metallschicht aufweisen, die so dünn ist, dass sie nicht mehr opak reflektierend wirkt. Beidseitig ist diese Schicht mit Dielektrikaschichten beschichtet. Derartige Pigmente sind nur kostenaufwendig herzustellen, da zur Herstellung einer Pigmentschicht auf der Ablösefolie diese dreimal bzw. fünfmal beschichtet werden muss. Die Herstellung der absorbierenden Zentralschicht ist nicht einfach unter Produktionsbedingungen zu reproduzieren.

Ähnliche Pigmente werden in der WO 2004/052999 A2 offenbart. Sie weisen die gleichen Nachteile auf.

Diese mehrschichtigen Effektpigmente weisen alle den Nachteil auf, dass die Dielektrikaschichten, im Vergleich zu Metallschichten, nur mit sehr langsamen Raten aufgedampft werden können. Daher sind mehrschichtige, durch Verdampfungsverfahren hergestellte Effektpigmente, bei denen Dielektrika verdampft bzw. aufgedampft werden, nur sehr kostenintensiv zu produzieren. Zudem muß eine Folie mehrmals bedampft werden, um den Multilayeraufbau realisieren zu können, was die Herstellungskosten weiter in die Höhe treibt.

In der EP 1 522 606 A1 wird die Herstellung einer Folie mit schwarzem Aluminiumoxid beschrieben. Hierbei werden weder Effektpigmente noch mehrschichtige Strukturen offenbart. Die hier offenbarten Folien weisen keinen nennenswerten Metalleffekt mit Glanz und Flop auf.

In der US 4,430,366 wird die Herstellung von Folien, die ein Gemisch aus Metall und Metalloxid enthalten, beschrieben. Hier sind ebenfalls keine Effektpigmente erwähnt. Die Folien besitzen eine inhomogene Zusammensetzung mit einem Gradienten von Metall und Metalloxid über die Schichtdicke, wobei der Metallkonzentrationsgradient und der Metalloxidgradient konträr zueinander sind.

Aufgabe der Erfindung ist es, hochbrillante, farbige oder schwarze, spiegelartige Metalleffektpigmente mit höchsten optischen Qualitäten und sehr hohem Hell-Dunkel-Flop bereitzustellen.

Weiterhin ist es Aufgabe der Erfindung, insbesondere hochbrillante, goldene, spiegelartige Metalleffektpigmente mit höchster ästhetischer Qualität ohne Farbausblutung bereitzustellen.

Eine weitere Aufgabe besteht darin, kostengünstige Herstellungsverfahren zur Bereitstellung derartiger Metallpigmente zu finden.

Die der Erfindung zugrunde liegende Aufgabe wird durch Bereitstellung eines mehrschichtigen PVD-Effektpigments gelöst, wobei das metallische Effektpigment plättchenförmig ist und wenigstens drei Schichten umfasst:

A) eine Schicht A, welche wenigstens ein Metall $M_A$ und einen durchschnittlichen Gehalt an Sauerstoff $O_A$, bezogen auf den Gesamtgehalt an $M_A$ und $O_A$ in der Schicht A aufweist,

B) eine Schicht B mit wenigstens einem Metall $M_B$ und einem durchschnittlichen Gehalt an Sauerstoff $O_B$ von 0 bis 77 Atom-%, vorzugsweise von 0 bis 66 Atom-%, weiter vorzugsweise von 0 bis 58 Atom-%, bezogen auf den Gesamtgehalt an $M_B$ und $O_B$ in der Schicht B,

C) eine Schicht C, welche wenigstens ein Metall $M_C$ und einen durchschnittlichen Gehalt an Sauerstoff $O_C$, bezogen auf den Gesamtgehalt an $M_C$ und $O_C$ in der Schicht C, aufweist,

wobei der durchschnittliche Sauerstoffgehalt $O_{AC}$ in den Schichten A und C gemäß der Formel (I)

$$O_{AC} = \frac{1}{2}\left(\frac{O_A}{M_A + O_A} + \frac{O_C}{M_C + O_C}\right) \qquad (I)$$

bestimmt wird und in einem Bereich von 2 bis 77 Atom-%, vorzugsweise von 4 bis 66 Atom-%, weiter vorzugsweise von 25 bis 58 Atom-%, liegt, wobei die Schichten A und C keine reinen stöchiometrischen Oxidschichten sind.

Ferner wird die Aufgabe durch Bereitstellung eines Verfahrens zur Herstellung eines metallischen Effektpigmentes nach einem der Ansprüche 1 bis 9 gelöst, bei dem die einzelnen Schichten A, B und C durch PVD-Verfahren nacheinander durch Aufdampfen von $M_A$, $M_B$ und $M_C$ angeordnet werden, wobei zumindest die Schichten A und C in Gegenwart wenigstens einer Sauerstoff-abgebenden Sauerstoffquelle aufgedampft werden.

Bevorzugte Weiterbildungen sind in den jeweiligen Unteransprüchen angegeben.

Die Angaben der Gehalte von Metallatomen M bzw. von Sauerstoffatomen O beziehen sich auf die Anzahl an den jeweiligen Atomen. In der Formel (I) bedeutet mithin die Angabe $M_A$ die Anzahl der Atome eines Metalles A in der Schicht A, die Angabe $M_B$ die Anzahl der Atome eines Metalles in der Schicht B, die Angabe $M_C$ die Anzahl der Atome eines Metalles in der Schicht C, die Angabe $O_A$ die Anzahl der Sauerstoffatome in der Schicht A, die Angabe $O_B$ die Anzahl der Sauerstoffatome in der Schicht B, die Angabe Oc die Anzahl der Sauerstoffatome in der Schicht C.

Dabei können die Metallatome in den Schichten A, B und C unabhängig voneinander verschieden oder gleich sein. Beispielsweise können die Metalle $M_A$ und $M_C$ gleich sein. Bei einer weiteren erfindungsgemäßen Ausführungsform sind die Metalle $M_A$, $M_B$ und $M_C$ gleich.

Bei einer bevorzugten Weiterbildung der Erfindung sind die Schichten A, B und C voneinander unterscheidbar, entweder im Hinblick auf die verwendeten Metalle oder im Hinblick auf den Sauerstoffgehalt.

Bei einer bevorzugten Ausführungsform weisen die erfindungsgemäßen metallischen Effektpigmente einen symmetrischen Aufbau auf, wobei die verwendeten Metalle $M_A$ und $M_C$ oder der Sauerstoffgehalt $O_A$ und $O_C$ jeweils gleich sind, wobei das Metall $M_B$ verschieden von den Metallen $M_A$ und $M_C$ bzw. der Sauerstoffgehalt $O_B$ verschieden von $O_A$ und $O_C$ ist. Bei einer Variante der Erfindung sind sowohl die Metalle $M_A$ und $M_C$ als auch die Sauerstoffgehalte $O_A$ und $O_C$ jeweils gleich, wobei das Metall $M_B$ verschieden von den Metallen $M_A$ und $M_C$ bzw. $O_B$ ist.

Gemäß einer weiteren Variante der Erfindung können die erfindungsgemäßen metallischen Effektpigmente auch einen asymmetrischen Aufbau haben, wobei die Metalle $M_A$, $M_B$ und Mc jeweils voneinander verschieden sein können und/oder die Sauerstoffgehalte $O_A$, $O_B$ und $O_C$ jeweils gleich oder voneinander verschieden sein können. Alternativ können die Metalle $M_A$, $M_B$ und $M_C$ jeweils gleich oder voneinander verschieden sein und/oder die Sauerstoffgehalte $O_A$, $O_B$ und $O_C$ jeweils voneinander verschieden sein.

Die vorliegende Erfindung betrifft gemäß einer Ausführungsform mehrschichtige Effektpigmente mit einem zentralen metallischen Kern, der mit wenigstens zwei optisch wirksamen Schichten A und C beschichtet ist.

Gemäß einer Variante der Erfindung ist der Gehalt an Sauerstoff in den Schichten A und C, also $O_A$ und $O_C$, höher als der Sauerstoffgehalt $O_B$ in der Schicht B.

Bei einer weiteren Variante der Erfindung ist der Sauerstoffgehalt $O_B$ in der Schicht B größer als der Gehalt an Sauerstoff in den Schichten A und C, von also $O_A$ und Oc.

EP 2 176 359 B1

Gemäß einer bevorzugten Ausführungsform umfassen die erfindungsgemäßen metallischen Effektpigmente einen Schichtaufbau aus drei metallischen Schichten A, B und C, wobei der Gehalt an Sauerstoff in den Schichten A, B und C wie oben angegeben ist.

Erfindungsgemäß kann auf die zentrale Schicht B, sowie auf den beiden flankierenden Schichten A und C, wenigstens eine weitere äußere Schicht A' bzw. C' aufgebracht sein, wobei die Schicht A' auf der Schicht A und die Schicht C' auf der Schicht C angeordnet ist, und das erfindungsgemäße Pigment somit einen fünfschichtigen Aufbau aufweist. Bei den Schichten A' und C' kann es sich um Schichten mit wenigstens einem Metall und einem Gehalt an Sauerstoff handeln, die von der Schicht A bzw. C verschieden sind, wobei jedoch der durchschnittliche Sauerstoffgehalt ebenfalls in einem Bereich von 2 bis 77 Atom-%, vorzugsweise von 4 bis 66 Atom%, weiter vorzugsweise von 25 bis 58 Atom-%, liegt. Sämtliche vor- oder nachstehenden Ausführungen bezüglich der Schichten A und/oder C gelten entsprechend für die Schichten A' und/oder C'.

Es hat sich jedoch überraschend gezeigt, dass bereits bei einem dreischichtigen Aufbau des Metallpigmentes mit den Schichten A, B und C ein breites Spektrum an Farb- und Flopeffekten erhalten werden kann.

Nachfolgend wird im Wesentlichen ein dreischichtiger Aufbau beschrieben, wobei das erfindungsgemäße Verfahren auch zur Herstellung von metallischen Effektpigmenten mit vier, fünf, sechs oder sieben Schichten, etc. verwendet werden kann. Unter Effektpigmenten wird im Sinne der Erfindung verstanden, dass die Pigmente plättchenartig sind, so dass sie in einem Anwendungsmedium, beispielsweise einem Lack, einer Farbe, einem Nagellack, etc., als eine Vielzahl kleiner Spiegel wirken und sich im Anwendungsmedium hervorragend orientieren bzw. ausrichten können. Die Effektpigmente sind mithin nicht sphärisch, sondern flächig ausgebildet. Das an den verschiedenen Schichten des Schichtaufbaus der erfindungsgemäßen Effektpigmente reflektierte Licht wird aufgrund der flächigen Struktur der Effektpigmente gerichtet reflektiert. Um bei einem Betrachter einen angenehmen visuellen Eindruck hervorzurufen, ist es wesentlich, dass sich die Pigmente in etwa planparallel zur Substratoberfläche ausrichten, damit das einfallende Licht von allen Pigmenten gerichtet reflektiert, d.h. nicht in die verschiedenen Richtungen gestreut wird.

Mit Effektpigmenten beschichtete Gegenstände weisen immer einen optischen Eindruck auf, der vom Beobachtungs- und/oder Einfallswinkel abhängt. Typisch für Metalleffektpigmente ist neben dem hohen Glanz u.a. der sogenannte Helligkeitsflop, d.h. eine Verringerung der Helligkeit vom Glanzwinkel hin zu steileren Einfalls- und/oder Beobachtungswinkeln.

Der Helligkeitsflop bei hochglänzenden spiegelartigen Beschichtungen ist geprägt durch eine sehr starke Abnahme der Helligkeit vom Glanzwinkel hin zu steileren Einfalls- und/oder Beobachtungswinkeln. Bei steileren Beobachtungswinkeln zeigen spiegelartige Beschichtungen ein sehr dunkles Erscheinungsbild, das durch die sehr glatten Oberflächen bedingt wird.

So ist beispielsweise der starke Helligkeitsflop eines spiegelartigen Goldschmucks "von Gold ins dunkle" auf eine sehr glatte Oberfläche des Schmuckgegenstandes zurückzuführen.

Im Folgenden wird die Erfindung anhand der detaillierten Beschreibung, Ausführungsbeispielen und der Bezugnahme auf Zeichnungen näher erläutert.

## Abbildungen

Abbildung 1 zeigt den schematischen Aufbau eines erfindungsgemäßen mehrschichtigen Pigments.

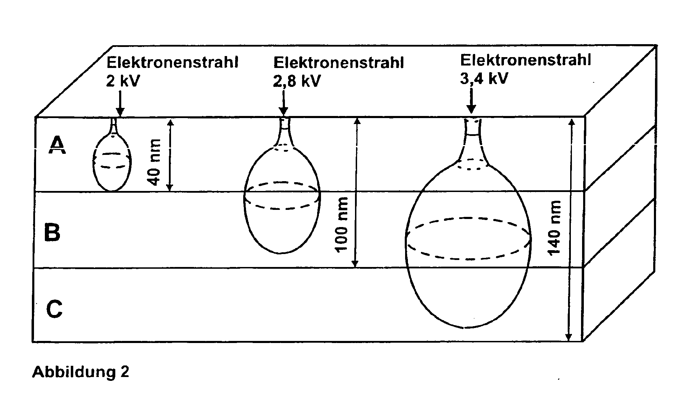

Abbildung 2 skizziert schematisch die Tiefenwirkung bei der EDX-Methode. Hier werden mittels eingestrahlter Röntgenstrahlung Elektronen aus der K- oder L-Schale der Elemente der Messprobe herausgeschlagen. Diese Elektronen besitzen jedoch nur eine gewisse Reichweite. Die Abbildung skizziert die Reichweite des Elektronenstrahls (auch "Anregungsbirne" genannt) in das erfindungsgemäße dreischichtige Pigment. Den jeweiligen Eindringtiefen entsprechend, lässt sich der atomare Sauerstoffgehalt, bezogen auf den Gesamtgehalt des zur Beschichtung aufgedampften Metalls und des Sauerstoffs, in Atom-%, bestimmen.

Abbildung 3 beschreibt schematisch die geometrische Anordnung in einer Drehtellerverdampfungsanlage in einer Vakuumkammer.

Abbildung 4a zeigt die schematische Darstellung einer Bandanlage mit einer Verdampfungsquelle, die in der Vakuumkammer von Abbildung 3 (ohne Drehtellervorrichtung) angeordnet ist.

Abbildung 4b zeigt die schematische Darstellung einer Bandanlage bei der Beschichtung eines bandförmigen Substrats mit drei Verdampfungsquellen.

Abbildung 5 zeigt, wie sich prinzipiell die drei Schichten A, B und C durch schrittweises Bedampfen darstellen lassen. Das Band wird nach den Beschichtungen A und B jeweils zurückgespult, um sowohl die einzelnen Schichten, als auch die komplette Schichtabfolge A-B-C realisieren zu können. Diese Vorgehensweise empfiehlt sich vor allem bei Verwendung unterschiedlicher Metalle für A oder B oder C.

Abbildung 6 zeigt den charakteristischen Verlauf des Sauerstoffgehalts in Abhängigkeit der Schichtdicke, der über EDX-Methodik ermittelt wurde.

Abbildung 7 stellt beispielhaft das Schichtdicken- bzw. Sauerstoffprofil, ermittelt mit der ESCA-Methode, für

EP 2 176 359 B1

den Fall dar, dass die zu verdampfenden Metalle für die Schichten A, B und C identisch sind.

Abbildung 8 stellt exemplarisch das Schichtdicken- bzw. Sauerstoffprofil, ermittelt mit der ESCA-Methode, für den Fall dar, dass das verwendete Metall für die Schichten A und C gleich ist, aber ungleich B ist.

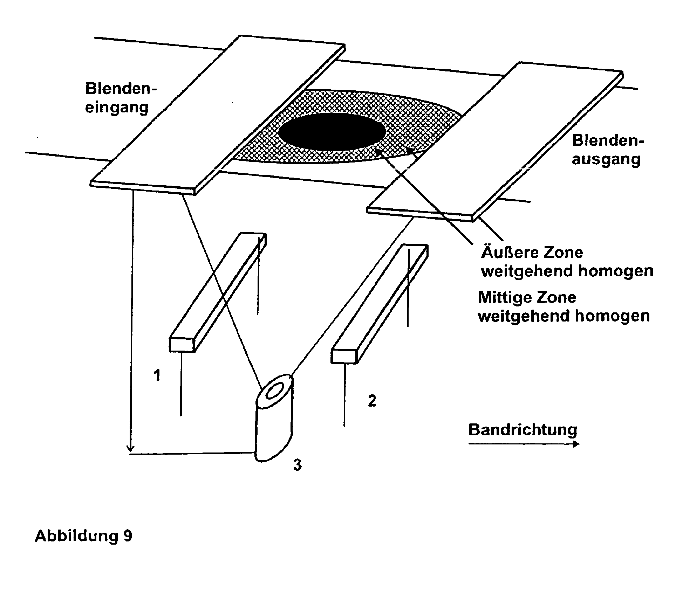

Abbildung 9 zeigt schematisch die Abbildung der Grundfläche eines bandförmigen Substrates und die Intensitätsverteilung eines Verdampferkegels auf diesem bandförmigen Substrat mit zwei Konzentrationsbereichen, wobei bei diesem Beispiel der Bereich mit der höchsten Konzentration an Metallatomen und der geringsten Konzentration an Sauerstoffatomen im Zentrum liegt und schwarz dargestellt ist. Dieser innere Bereich ist von einem äußeren, schraffiert kariert dargestellten Bereich umgeben, in dem die Konzentration an Metallatomen geringer und die Konzentration an Sauerstoffatomen höher ist als in dem inneren Bereich. Die relative Ausdehnung dieser beiden Bereiche zueinander kann durch die Anordnung der Sauerstoff-abgebenden Sauerstoffquelle seitlich von der Metallverdampferquelle beeinflußt werden.

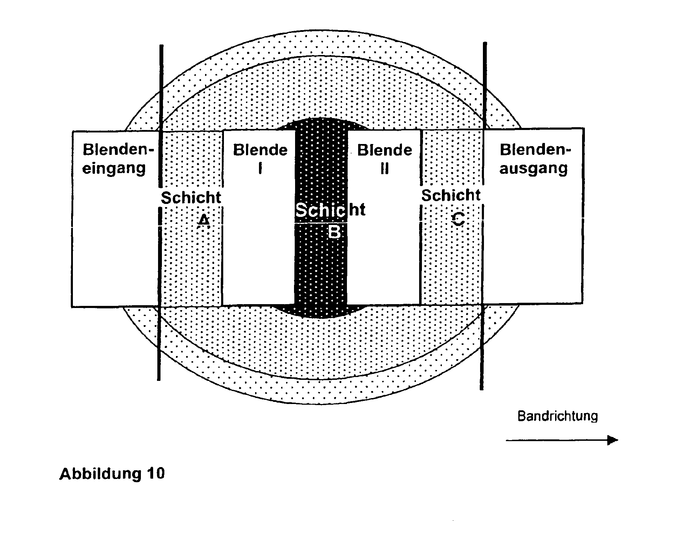

Abbildung 10 zeigt, wie ausgehend von der in Abbildung 9 dargestellten Intensitätsverteilung durch Anordnung von Abdeckvorrichtungen, wie den dargestellten Blenden I und II, über dem sich in die gezeigte Richtung bewegenden Band Schichten mit definierten und weitgehend homogenen Gehalten an Metall- und Sauerstoffatomen übereinander auf dem Band aufgebracht werden können. Bei dem erhaltenen Schichtenaufbau mit drei Schichten A, B und C weisen bei dieser beispielhaften Variante die Schichten A und C die gleiche Zusammensetzung auf und sind weitgehend oxidisch. Schicht B ist weitgehend metallisch.

Abbildung 11 zeigt die präparative Abschirmung eines bandförmigen Substrats durch horizontale und vertikale Blenden I und II, die letztlich die Beschichtung der einzelnen Schichten A, B und C sowie der gesamten Schichtabfolge A-B-C simultan bei der Metallisierung erzeugen. Die Schichten A und C sind weitgehend oxidisch und die Schicht B ist weitgehend metallisch.

Abbildung 12 zeigt beispielhaft, die farbmetrische Darstellung erfindungsgemäßer metallischer goldfarbener, blauer, violetter und roter Effektpigmente im a\*, b\*-Farbraum und die farbsättigende Wirkung der oxidhaltigen Schichten A und C im vergleich zu Schichten A und C mit geringerem Oxidanteil.

Abbildung 13 zeigt beispielhaft die Verstärkung des Chromas bei goldfarbenen und blauen metallischen Effektpigmenten durch den höheren Oxidanteil in den Schichten A und C.

Abbildung 14 sind die Helligkeitswerte über verschiedene Winkel graphisch dargestellt. Diese Darstellung soll die Spiegelartigkeit der erfindungsgemäßen Effektpigmente unterstreichen.

Abbildung 15 zeigt eine alternative Anordnung zu Abbildung 9. Im Unterschied zur Anordnung gemäß Abbildung 9 ist der Gaseinlass zentral über der Verdampfungsquelle 2 angeordnet.

Abbildung 16 zeigt eine inverse Anordnung der aufgedampften Schichten zur Anordnung der Schichten gemäß Abbildung 10, wobei in Schicht A und C der Gehalt an Sauerstoff geringer ist als in Schicht B. Die Schichten A und C sind dabei weitgehend metallisch und die Schicht B weitgehend oxidisch.

Abbildung 17 zeigt eine inverse Anordnung der aufgedampften Schichten zur Anordnung gemäß Abbildung 11, wobei in Schicht A und C der Gehalt an Sauerstoff geringer ist als in Schicht B. Die Schichten A und C sind dabei weitgehend metallisch und die Schicht B weitgehend oxidisch.

Die Erfindung betrifft mithin die Bereitstellung von mehrschichtigen metallischen PVD-Effektpigmenten, die folgende Schichtenfolge umfassen:

A) eine weitgehend metallische Schicht A mit einer weitgehend homogenen chemischen Zusammensetzung, welche wenigstens ein Metall $M_A$ und einen dazugehörigen durchschnittlichen Gehalt an Sauerstoff $O_A$, bezogen auf den Gesamtgehalt an $M_A$ und Sauerstoff in dieser Schicht, aufweist,

B) eine Schicht B mit wenigstens einem Metall $M_B$ und einem dazugehörigen durchschnittlichen Gehalt an Sauerstoff $O_B$, von 0 bis 77 Atom-%, vorzugsweise von 0 bis 66 Atom-%, weiter bevorzugt von 0 bis 58 Atom-%, bezogen auf den Gesamtgehalt an $M_B$ und Sauerstoff $O_B$ in dieser Schicht, aufweist, wobei $M_B$ gleich oder verschieden von $M_A$ ist,

C) eine weitgehend metallische Schicht C, welche wenigstens ein Metall $M_C$ und einen dazugehörigen durchschnittlichen Gehalt an Sauerstoff $O_C$, bezogen auf den Gesamtgehalt an $M_C$ und Sauerstoff $O_C$ in dieser Schicht, aufweist, wobei der durchschnittliche Sauerstoffgehalt $O_{AC}$ gemäß der Formel (I)

$$O_{AC} = \frac{1}{2}\left( \frac{O_A}{M_A + O_A} + \frac{O_C}{M_C + O_C} \right) \qquad (I)$$

bestimmt wird und in einem Bereich von 2 bis 77 Atom-%, weiter bevorzugt von 4 bis 66 Atom-%, noch weiter bevorzugt von 25 bis 58 Atom-%, liegt.

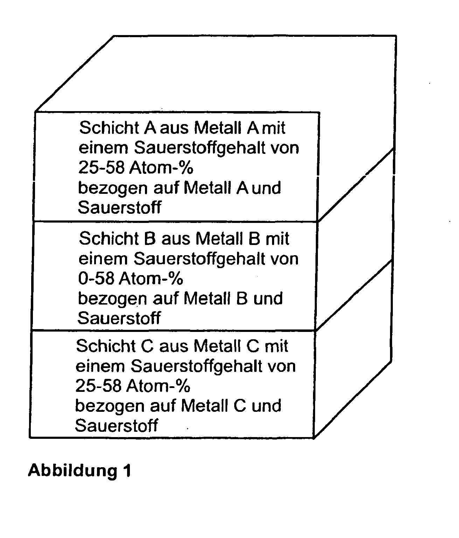

Der Schichtaufbau von einer erfindungsgemäßen Ausführungsform der erfindungsgemäßen Pigmente ist in Abbildung 1 näher illustriert. Der Gehalt an Sauerstoff in den Schichten A, B und/oder C liegt dabei innerhalb der in Anspruch 1 angegebenen Grenzen. Die nachstehenden Ausführungen gelten mithin entsprechend für höhere bzw. niedrigere Gehalte an Sauerstoff in den Schichten A, B und/oder C, wie in Anspruch 1 jeweils angegeben.

Sämtliche Schichten zeichnen sich vorzugsweise jeweils dadurch aus, dass sie eine weitgehend homogene, vorzugsweise homogene, Verteilung von Metallatomen und Sauerstoffatomen aufweisen. Es liegt somit vorzugsweise innerhalb einer jeden Schicht über die Schichtdicke und -breite kein mit den weiter unten beschriebenen Meßmethoden messbarer Gradient an Metall- bzw. Sauerstoffatomen vor. Der hohe Sauerstoffgehalt ist weitgehend auch nicht auf die Ausbildung oberflächliche Metalloxidschichten zurückzuführen, die bei einigen Metallen spontan beim Kontakt mit Luft oder einer anderen Sauerstoffquelle entstehen können.

Die erfindungsgemäß bereitgestellten metallischen Effektpigmente sind und wirken für einen Betrachter metallisch.

Die Schichten A und/oder C der erfindungsgemäßen Pigmente sind bei der dargestellten erfindungsgemäßen Variante so ausgebildet, dass sie durch einen gesteigerten Oxidgehalt von 25 bis 58 Atom-% Sauerstoff ein transparenteres Erscheinungsbild zeigen als reine Metallschichten bei gleicher Schichtstärke. Bei diesen Schichten handelt es sich jedoch nicht um reine Oxide, die in der Regel vollkommen transparent sind. Gleiches gilt für einen Sauerstoffgehalt bei Schicht B in einem Bereich von 0 bis 77 Atom-% oder von 0 bis 66 Atom-%.

Im Stand der Technik sind in der WO99/35194 Pigmente mit einem dreischichtigen Aufbau beschrieben, bei welchen die Eigenfarbe einer intermediär liegenden Metallschicht durch zwei außenliegende dielektrische Stützschichten nicht verändert wird.

Die Schichten A und/oder C enthalten unabhängig voneinander vorzugsweise wenigstens ein Metall $M_A$ und/oder $M_C$, das aus der Gruppe, bestehend aus Aluminium, Magnesium, Chrom, Silber, Kupfer, Gold, Zink, Zinn, Mangan, Eisen, Kobalt, Nickel, Titan, Tantal, Molybdän, deren Mischungen und deren Legierungen, ausgewählt wird.

Bevorzugte Metalle für $M_A$ und/oder $M_C$ sind Aluminium, Silber, Kupfer, Chrom, Eisen oder Mischungen oder Legierungen davon. Als sehr geeignet haben sich insbesondere Aluminium und/oder Chrom erwiesen.

Die Schichten A und/oder C der erfindungsgemäßen metallischen Effektpigmente besitzen vorzugsweise einen weitgehend metallischen Charakter.

Wenn der Sauerstoffgehalt $O_B$ der Schicht B höher ist als der Sauerstoffgehalt der Schichten A und/oder C, also $O_A$ und/oder $O_C$, weist die Schicht B vorzugsweise einen weitgehend oxidischen Charakter auf. Bei dieser Variante kann dann der metallische Charakter der Schichten A und/oder C stärker ausgeprägt sein.

Sind in einem dreischichtigen Aufbau eines Effektpigmentes die beiden äußeren Schichten farblose Dielektrika, so sind diese i.d.R. optisch nicht wirksam. Im Gegensatz hierzu sind die Schichten A und/oder C der erfindungsgemäßen Effektpigmente optisch wirksam. In Abhängigkeit von der Zusammensetzung der Schichten A und/oder C, d.h. in Abhängigkeit von dem Anteil an Metall und Sauerstoff in den Schichten A und/oder C wird der Farbeindruck für einen Betrachter verändert. Es kommt mithin bei den erfindungsgemäßen metallischen Effektpigmenten zu einer optischen Wechselwirkung zwischen der zentralen Schicht B und den äußeren Schichten A und/oder C, wodurch beim Betrachter der Farbeindruck erzeugt wird.

Der hohe Sauerstoffgehalt in den Schichten A und/oder C der erfindungsgemäßem metallischen Effektpigmente ist auf die Bildung von Oxiden und/oder Suboxiden neben rein metallischen Anteilen zurückzuführen. Es ist jedoch noch nicht im letzten Detail geklärt, welchen strukturellen Aufbau die Schichten A und/oder C besitzen.

Untersuchungen mit Elektronenbeugung zeigen zwei deutlich unterscheidbare diffuse Ringe, die Metall und Metalloxid in der Schicht A und/oder C zugewiesen werden können. Diese Ergebnisse zeigen, dass die Schicht A und/oder C unterschiedliche Phasen von Metall und Metalloxid in einer sehr feinteiligen Form, bevorzugt im Nanometerbereich, enthält. Dabei liegt die mittlere Größe dieser Nanoteilchen bei unter ca. 40 nm, bevorzugt bei unter 30 nm, besonders bevorzugt bei 20 nm und weiterhin besonders bevorzugt bei kleiner als 10 nm. Eine Schicht mit derartigen Strukturen wird im Rahmen dieser Erfindung als weitgehend metallisch bezeichnet.

Derartig feinstrukturierte Phasen Können nicht zuverlässig in REM-Aufnahmen oder mittels der oben erwähnten EDAX- oder XPS-Spektroskopie detektiert werden. Daher sind die Schichten A und/oder C der erfindungsgemäßen metallischen Effektpigmente im Sinne dieser Erfindung weitgehend homogen zusammengesetzt. Auf keinen Fall jedoch handelt es sich bei den Schichten A und C um reine, stöchiometrische Oxidschichten.

Wenn der Sauerstoffgehalt $O_B$ der Schicht B höher ist als der Sauerstoffgehalt der Schichten A und/oder C, also $O_A$ und/oder $O_C$, enthält die Schicht B entsprechend Metall und Metalloxid in feinteiliger Form. Die vorstehend für die Schichten A und/oder C gemachten Ausführungen gelten dann entsprechend für die Schicht B.

Es hat sich nun völlig überraschend gezeigt, dass die erfindungsgemäßen metallischen Effektpigmente neuartige optische Eigenschaften aufweisen. So nimmt gegenüber einem rein metallischen dreischichtigen PVD-Pigment mit steigendem Sauerstoffgehalt in den Schichten A und/oder C die Farbintensität der Effektpigmente zu. Weiterhin können sehr hohe Hell-Dunkel-Flops realisiert werden, die in dieser Stärke und Farbintensität bislang nicht mit einem Effektpigment zugänglich waren.

Es hat sich ferner überraschend gezeigt, dass die vorzugsweise homogen aus Sauerstoff und Metall aufgebauten Schichten der erfindungsgemäßen metallischen Effektpigmente eine gute Wetterstabilität sowie gute UV- und Schwitzwasserstabilitäten etc. aufweisen.

Bei einer bevorzugten Ausführungsform besitzen die beiden optisch wirksamen Schichten A und C der erfindungsgemäßen Effektpigmente zusammen einen durchschnittlichen Gehalt an Sauerstoff von 30 bis 57 Atom-% und besonders bevorzugt 35 bis 56 Atom-%. Der durchschnittliche Sauerstoffgehalt wird gemäß der Formel (I)

$$O_{AC} = \frac{1}{2}\left(\frac{O_A}{M_A + O_A} + \frac{O_C}{M_C + O_C}\right) \qquad (I)$$

bestimmt.

Unterhalb eines Sauerstoffgehaltes von 25 Atom-%, bezogen auf den Gesamtgehalt an $M_A$ und/oder $M_C$ und Sauerstoff scheiden sich beispielsweise Metalle mit der Oxidationsstufe +III, wie z.B. Aluminium oder Chrom, weitgehend in ihrer jeweiligen Eigenfarbe auf metallischen Oberflächen ab. Oberhalb von 58 Atom-%, bezogen auf den Gesamtgehalt an $M_A$ und/oder $M_C$ und Sauerstoff, scheiden sich die Metalle mit der Oxidationsstufe +III überwiegend als Metalloxide ab und verlieren ihren weitgehend metallischen Charakter.

Gemäß einer weiteren besonders bevorzugten Ausführungsform der Erfindung liegt der durchschnittliche Gehalt an Sauerstoff $O_A$, bezogen auf den Gesamtgehalt an $M_A$ und $O_A$ in der Schicht A, sowie der durchschnittliche Gehalt an Sauerstoff $O_C$, bezogen auf den Gesamtgehalt an $M_C$ und $O_C$ in der Schicht C, unabhängig voneinander jeweils in einem Bereich von 25 bis 58 Atom-%, bevorzugt 30 bis 57 Atom-% und besonders bevorzugt von 35 bis 56 Atom-%.

Bei dieser Ausführungsform besitzen bevorzugt die beiden Schichten beide einen weitgehend metallischen Charakter und die damit verbundenen Vorteile kommen insgesamt bei diesem erfindungsgemäßen Effektpigment besonders gut zutage.

Um den Sauerstoffgehalt innerhalb einer einzigen Schicht A und/oder C bestimmen zu können, muss durch geeignete Fokussierung jeweils ein einziges Effektpigment mit den weiter unten angegebenen Methoden analysiert werden. Um einen durchschnittlichen Wert für alle Effektpigmente zu ermitteln, sollten diese Messungen an mindestens 5, bevorzugt mindestens 10, einzelnen Pigmenten durchgeführt und anschließend ein Mittelwert gebildet werden.

Bei weiteren bevorzugten Ausführungsformen besitzt nur eine der beiden Schichten A oder C einen durchschnittlichen Sauerstoffgehalt $O_{A/C}$, bezogen auf den Gesamtgehalt an $M_{A/C}$ und $O_{A/C}$, im Bereich von 0 bis 58 Atom-%. Der Sauerstoffgehalt der jeweils anderen Schicht kann höher oder niedriger als dieser Bereich sein, jedoch ist der Mittelwert des Sauerstoffgehaltes beider Schichten im erfindungsgemäßen Bereich. Mithin besitzt eine der beiden Schichten bei dieser Ausführungsform einen eher metallischen oder eher oxidischen Charakter als die andere. Sollte eine Schicht einen Sauerstoffgehalt von unter 25 Atom-% aufweisen, so ist die Schichtdicke so zu wählen, dass die Schicht optisch noch teildurchlässig ist, um auf diese Weise an Interferenzphänomenen teilnehmen zu können.

Die zentrale Schicht B des erfindungsgemäßen mehrschichtigen metallischen Effektpigmentes kann sehr unterschiedliche Sauerstoffgehalte aufweisen. Der mittlere Sauerstoffgehalt beträgt 0 bis 77 Atom-%, vorzugsweise 0 bis 66 Atom-%, vorzugsweise 0 bis 58 Atom-%, bezogen auf den Gesamtgehalt an $M_B$ und Sauerstoff in der Schicht B.

Bei einer besonders bevorzugten Ausführungsform ist die Schicht B eine metallische Schicht mit einem mittleren Sauerstoffgehalt von 0 bis 25 Atom-%, bevorzugt von 0 bis 15 Atom-% und besonders bevorzugt von 0 bis 10 Atom-%.

Bei dieser Ausführungsform besitzt die Schicht B einen metallischen Charakter. Sie wirkt daher i.d.R. als metallischer Reflektor. Einfallendes Licht kann durch Wechselwirkung der planparallel orientierten erfindungsgemäßen Effektpigmente durch die optische Wechselwirkung der Schichten A und/oder C mit der Schicht B durch Interferenzphänomene zu reizvollen optischen Eindrücken führen.

Vorzugsweise liegt die Schichtdicke der Schicht B im Fall metallischer Schichten in einem Bereich von 10 bis 200 nm, bevorzugt von 20 bis 150 nm, weiter bevorzugt von 40 bis 125 nm, noch weiter bevorzugt von 50 bis 100 nm.

Oberhalb von ca. 40 nm Schichtdicke sind diese metallischen Schichten optisch opak und metallisch reflektierend. Das optische Erscheinungsbild der Pigmente wird durch das Zusammenspiel der Schicht A und/oder Schicht C und Schicht B bewirkt. Unterhalb von 40 nm zeigen solche Schichten ein zunehmend transparentes Erscheinungsbild mit einer zu Weilen dunklen Färbung. Hier wirkt die Metallschicht noch stark absorbierend, kann jedoch nicht die hohe Reflektivität eines Metallreflektors entfalten. Das optische Erscheinungsbild der Pigmente wird in diesem Fall durch das optische Zusammenspiel aller Schichten A, B und C bewirkt.

Unterhalb von 10 nm ist das Erscheinungsbild dieser Schichten weitgehend oxidisch, da der Einfluss der sich an der Oberfläche von Metallen natürlich bildenden Oxidschichten stark zunimmt, und die Schicht verliert ihren metallischen Charakter.

Oberhalb von 200 nm ändern sich die optischen Eigenschaften der Schicht in keiner Weise und daher würden dickere Schichten nur unnötige Materialverschwendungen mit sich bringen.

Bei weiteren erfindungsgemäßen Ausführungsformen beträgt der mittlere Sauerstoffgehalt der Schicht B 25 bis 58 Atom-%, bevorzugt 30 bis 57 Atom-% und besonders bevorzugt 35 bis 56 Atom-%. Vorzugsweise besitzt die Schicht B in diesem Fall einen weitgehend metallischen oder oxidischen Charakter und weist vorzugsweise eine Schichtdicke von 50-2000 nm auf.

Bei einer bevorzugten Ausführungsform besitzt hierbei die Schicht B analog zu den Schichten A und/oder C einen weitgehend metallischen Charakter.

In diesem Fall liegt die Schichtdicke der Schicht B vorzugsweise in einem Bereich von 10 bis 200 nm, bevorzugt von 20 bis 150 nm, weiter bevorzugt von 40 bis 125 nm, noch weiter bevorzugt von 50 bis 100 nm.

Mit derartigen Schichtabfolgen lassen sich besonders bevorzugt sehr dunkle bis schwarze metallische Effektpigmente herstellen. Dies kann durch die Verwendung von Luft und/oder Wasser als Sauerstoffquelle unterstützt werden (s. Beispiel 13).

Bei einer weiteren bevorzugten Ausführungsform können die Schichten A, B und/oder C voneinander verschieden sein. Vorzugsweise sind die Schichten A und B entweder im Hinblick auf das Metall und/oder im Hinblick auf den Sauerstoffgehalt unterschiedlich, wie oben ausgeführt.

Es ist gemäß einer Ausführungsform der Erfindung bevorzugt, dass die zentrale Schicht B im wesentlichen oxidisch ist und die auf der zentralen Schicht B aufgebrachten Schichten A und C im wesentlichen metallisch sind.

Es ist gemäß einer weiteren Ausführungsform der Erfindung bevorzugt, dass die zentrale Schicht B im wesentlichen metallisch ist und die auf der zentralen Schicht B aufgebrachten Schichten A und C im wesentlichen oxidisch sind.

Bei einer weiteren Ausführungsform kann die Schicht B jedoch auch einen weitgehend oxidischen Charakter annehmen. In diesem Fall ist nun weiter bevorzugt, dass beide Schichten A und C einen weitgehend metallischen Charakter mit jeweils einem durchschnittlichen Sauerstoffgehalt von 25 bis 58 Atom-%, bevorzugt von 0 bis 25 Atom-%, weiterhin bevorzugt 0 bis 15 Atom-%, besonders bevorzugt 0 bis 10 Atom% aufweisen. In diesem Fall handelt es sich um Effektpigmente mit einem Fabry-Perot-Aufbau.

Die Schichtdicke der Schicht B kann im Fall von weitgehend oxidischen Schichten von 50 bis 2000 nm, bevorzugt von 100 bis 1000 nm und besonders bevorzugt von 150 bis 800 nm variiert werden. Bei niedrigen Schichtdicken werden dabei weitgehend einfarbige metallische Effektpigmente erhalten und bei höheren Schichtdicken Effektpigmente mit Farbflops.

Im Gegensatz zu den im Stand der Technik bekannten Pigmenten mit ähnlichem Aufbau weisen die erfindungsgemäßen Effektpigmente eine stärkere Farbsättigung auf. Diese ist vermutlich auf die besonderen Eigenschaften der äußeren Schichten A und C zurückzuführen.

Die zentrale metallische Schicht B enthält vorzugsweise wenigstens ein Metall, das aus der Gruppe, bestehend aus Aluminium, Chrom, Silber, Kupfer, Gold, Zink, Zinn, Mangan, Eisen, Kobalt, Nickel, Titan, deren Mischungen und deren Legierungen, ausgewählt ist.

Bevorzugte Metalle sind dabei Aluminium, Silber, Chrom oder Mischungen oder Legierungen davon.

Gemäß einer bevorzugten Ausführungsform ist das Metall $M_A$ und/oder $M_C$ im wesentlichen Chrom und in der Schicht A und/oder C liegt unabhängig voneinander der durchschnittliche Gehalt an Sauerstoff $O_A$ bzw. $O_C$ im Bereich von 35 bis 48 Atom-%, bezogen auf den jeweiligen Gesamtgehalt an Chrom und Sauerstoff in der Schicht A bzw. C. Es hat sich gezeigt, dass Pigmente mit außenliegenden Chrom/Chromoxidschichten als Schicht A und C korrosionsstabile Effektpigmente sind. Wenn gemäß weiterer Ausführungsformen die mittlere Schicht B silbrig reflektierend ist (z.B. aus Ag oder Al in entsprechenden Schichtdicken), so erhält man in Abhängigkeit von der Schichtdicke der Schichten A und C einen Farbeindruck von hellgold, gold, braun, rot, violett, blau bis türkis.

Gemäß einer weiteren bevorzugten Ausführungsform ist das Metall $M_A$ und/oder $M_C$ im wesentlichen Aluminium und in der Schicht A und/oder C liegt unabhängig voneinander der durchschnittliche Gehalt an Sauerstoff $O_A$ bzw. $O_C$ im Bereich von 30 bis 55 Atom-%, bezogen auf den jeweiligen Gesamtgehalt an Aluminium und Sauerstoff in der Schicht A bzw. C. Es hat sich gezeigt, dass Pigmente mit außenliegende Aluminium/Aluminiumoxidschichten als Schicht A und C preisgünstig herzustellende Effektpigmente sind, die in Abhängigkeit von der Schichtdicke der Schichten A und C einen außerordentlich starken Helligkeitsflop aufweisen. Wenn gemäß weiterer Ausführungsformen die mittlere Schicht B silbrig reflektierend ist (z.B. aus Ag oder Al in entsprechenden Schichtdicken), so erhält man ebenfalls in Abhängigkeit von der Schichtdicke der Schichten A und C einen Farbeindruck von hellgold, gold, braun, rot, violett, blau bis türkis.

Das Metall kann in jeder Schicht, beispielsweise in den vorgenannten Schichten A, B und/oder C, als Legierung oder Mischung verschiedener Metalle vorliegen. Gemäß einer bevorzugten Ausführungsform wird in jeder Schicht A, B und/oder C ein Metall mit einer Reinheit oberhalb von 99,0 Gew-% verwendet. Die Schichten A, B und/oder C können dabei im Hinblick auf das verwendete Metall oder die verwendeten Metalle gleich oder unabhängig voneinander verschieden sein. Die Schichten A, B und/oder C können sich dann beispielsweise im Hinblick auf den Anteil an Sauerstoff in Atom-% und/oder der Schichtdicke voneinander unterscheiden. Vorzugsweise weisen die Schichten A und C eine vergleichbare, weiter bevorzugt eine identische, chemische Zusammensetzung auf, wobei weiter bevorzugt die Schichten A und C zusätzlich eine identische Schichtdicke aufweisen. Weiterhin ist bevorzugt, dass die chemische Zusammensetzung der Schicht B von den Schichten A und/oder C verschieden ist.

Vorzugsweise sind die Schichten A, B und C unmittelbar aufeinander folgend angeordnet. Es können sich jedoch auch zwischen den Schichten, beispielsweise zwischen Schicht A und Schicht B, eine oder mehrere weitere Schichten befinden.

Die optische Wirkungsweise der Schichten A und/oder C ist vor allem von der Art des Metalls, der Schichtdicke und dem Sauerstoffgehalt abhängig. Bevorzugt beträgt die Schichtdicke der Schichten A und/oder C unabhängig voneinander 10 bis 250 nm und besonders bevorzugt 40 bis 150 nm.

Unterhalb von 10 nm Schichtdicke ist der durch diese Schichten hervorgerufene optische Effekt zu gering und oberhalb von 250 nm ist die Transparenz zu gering, wodurch die erfindungsgemäßen Vorteile des vorzugsweise dreischichtigen Aufbaus nicht mehr sichtbar sein können.

Vorzugsweise sind die mittleren Schichtdicken von A und C im Wesentlichen gleich dick.

Um verschiedenste optische Effekte zu erzeugen, kann sowohl die Schichtdicke der zentralen Schicht B und/oder die Schichtdicke der Schichten A und/oder C variiert werden. Beispielsweise kann nur die Schichtdicke der zentralen Schicht B des erfindungsgemäßen Effektpigments verändert, beispielsweise in einem Bereich von 10 bis 200 nm, und die Schichtdicke der Schichten A und/oder C konstant gehalten werden.

Die erfindungsgemäßen farbigen metallischen Effektpigmente besitzen bevorzugt eine durchschnittliche mittlere Pigmentdicke von 30 bis 550 nm und besonders bevorzugt von 50 bis 300 nm.

Die Pigmentdicke wird maßgeblich von den Schichtdicken der farbgebenden Schichten A und/oder C und der, vorzugsweise im Bereich von 10 bis 200 nm liegenden, Schichtdicke für B bestimmt. Die Schichtstärke B wirkt oberhalb von 20 nm praktisch nicht farbgebend. Oberhalb von ca. 40 nm Schichtdicke und einem Sauerstoffgehalt unter 15 Atom-% wirkt die Schicht B als opaker metallischer Reflektor. In diesem Fall wird die Farbgebung des metallischen Effektpigmentes nur durch die Schicht A bzw. C und die Schicht B bewirkt.

Die Schichtdicken der einzelnen Schichten des erfindungsgemäßen Effektpigmentes können beispielsweise durch den Fachmann mittels REM-Aufnahmen anhand von Querschliffaufnahmen bestimmt werden. Hierbei ist darauf zu achten, dass die Querschliffe an Effektpigmenten, die im Wesentlichen parallel zu einer definierten Ebene orientiert sind, durchgeführt werden. Dies ist erforderlich, um Meßfehler durch "gekippte" Pigmente zu vermeiden.

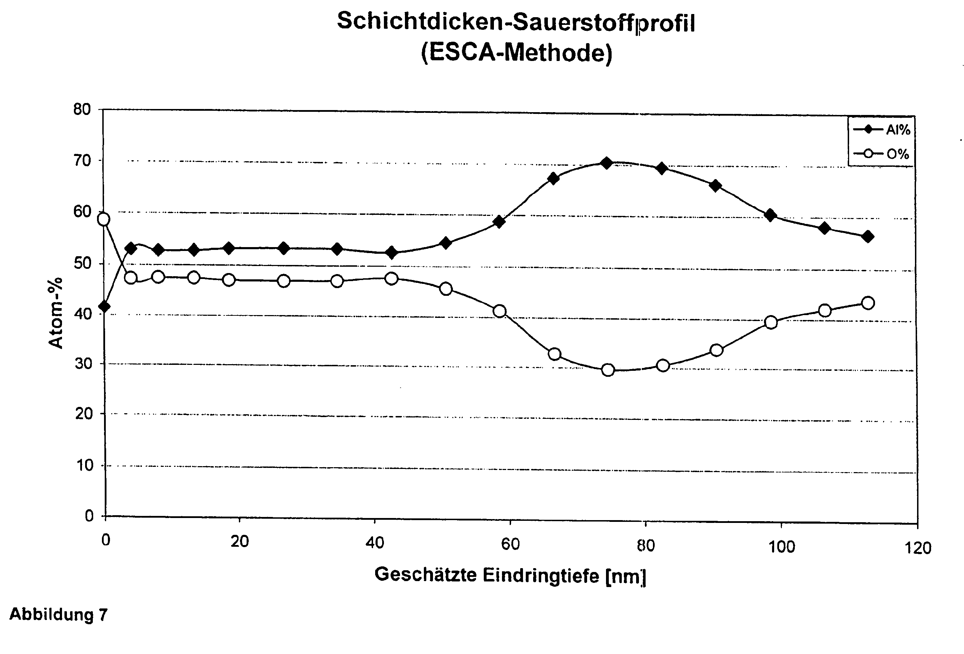

Die im Rahmen dieser Erfindung bestimmten Sauerstoffgehalte der erfindungsgemäßen Effektpigmente werden bevorzugt mittels der ESCA-Methode (<u>e</u>lectron <u>s</u>pectroscopy for <u>c</u>hemical <u>a</u>nalysis) oder der Auger-Spektroskopie jeweils in Kombination mit Sputterverfahren bestimmt. Hier kann für die Effektpigmente ein Tiefenprofil der mittleren Elementzusammensetzung entlang der Dicke der Pigmente erstellt werden. Es kann dabei zwischen dem Sauerstoffgehalt der Oberfläche und dem des Pigmentinneren differenziert werden. Durch diese Methode kann ebenfalls das Schichtdicken- bzw. Sauerstoffprofil besonders gut dargestellt werden. Um dies zu veranschaulichen wird auf die Abbildung 7 hingewiesen.

Abbildung 7 stellt das Schichtdicken-Sauerstoffprofil des gemäß dem erfindungsgemäßen Beispiel 9 hergestellten metallischen Effektpigmentes dar, das durch Messung mit der ESCA-Methode und Absputtern erhalten wurde. Bei diesem Effektpigment handelt es sich um ein dreischichtiges Pigment mit folgender Schichtfolge: A: oxidisches Al; B: metallisches Al; C: oxidisches Al. Während der ersten ca. 50 nm Schichtdicke erkennt man eine konstante Zusammensetzung mit einem Al-Anteil von 53 Atom-% und einem Sauerstoffanteil von 47 Atom-%. Nach ca. 50 nm Sputtertiefe steigt der Aluminiumanteil kontinuierlich bis zu einem Maximum von 71 Atom-%. Der Sauerstoffanteil sinkt hierzu proportional ab.

Die Messung wurde an mehreren Pigmenten durchgeführt. Hierzu wurde das Pigment zunächst in Aceton dispergiert und anschließend wurde diese Dispersion auf einen Glasträger aufgebracht. Nach dem Verdampfen des Lösemittels wurde die Probe untersucht. Naturgemäß liegen die Pigmente, die weitgehend parallel zum Glassubstrat orientiert sind, in einer statistischen Verteilung vor, d.h. in weitgehend gleichen Anteilen liegen Pigmente vor, bei denen die Schicht A bzw. die Schicht C nach oben zeigen. Daher entspricht der Sauerstoffgehalt der ersten Messungen einem Mittelwert gemäß Formel I der beiden Schichten A und C. Der am Anfang der Messung bestimmte Sauerstoffgehalt ist etwas höher und ist durch die sich natürlich bildende äußere Oxidschicht bedingt und wird nicht bei der Bestimmung des Sauerstoffgehaltes in den äußeren Schichten A und C berücksichtigt. Vielmehr wird in diesem Fall der durchschnittliche Wert des sich einstellenden Plateaus zur Bestimmung des Sauerstoffgehaltes herangezogen. Für die Schicht B ist das Maximum des Metallgehaltes zur Bestimmung des Metallgehaltes heranzuziehen. Dies erweist sich als nötig, da sich das Signal des Metallgehaltes nur langsam verändert. Die starke Verbreiterung dieses Signals an den Übergängen von den Schichten A und C zur mittleren Schicht B, welche an und für sich von scharf definierte Grenzflächen sind, ist durch mehrere Faktoren bedingt:

a) Die austretenden Elektronen der analysierten Schicht kommen jeweils aus einem Tiefenbereich von ca. 10 nm.

b) Die Messung wird jeweils an mehreren Pigmenten gleichzeitig durchgeführt. Damit gehen die vorhandenen Schichtdickenverteilungen aller einzelnen Schichten A, B und C sämtlicher Pigmente in die Messung mit ein.

c) Insbesondere nach sehr starkem Absputtern können einzelne Pigmente bereits völlig weggesputtert sein und man erreicht bereits darunter liegende Pigmente.

Aus diesen Gründen wird zur Bestimmung des Metallgehaltes nach dieser Methode stets das Maximum innerhalb eines einer Schicht zuzuordnenden Bereiches gewählt.

Stellt sich ein maximales Plateau ein, so wird dieses zur Bestimmung des Sauerstoff- bzw. des Metallgehaltes herangezogen. Der Fachmann ist hier in der Lage, die richtige Zuordnung zu treffen. In diesem Zusammenhang ist es empfehlenswert, die Messungen eines derartigen Sputterprofils mit der Schichtdickenanalyse aus REM-Bildern zu kombinieren. Auf diese Weise ist der Fachmann bereits über den zu erwartenden Schichtaufbau informiert. Dies gilt insbesondere vor dem Hintergrund, dass die mittels Sputtern ermittelte Tiefenskala mit einem großen Fehler behaftet sein kann und dann nur sehr eingeschränkt zur Ermittlung der Schichtdicken herangezogen werden kann.

Diese Methode lässt sich auch an einzelnen Pigmenten durchführen. Hierbei wird das einstrahlende UV-Licht auf ein einzelnes Pigmentplättchen fokussiert und entsprechend vermessen. Die Pigmente können zuvor durch Übersichtsaufnahmen mittels der Elektronenemission charakterisiert, und die Fokussierung entsprechend kontrolliert werden. Hierbei sind mindestens fünf einzelne Pigmente zu analysieren, um einen repräsentativen Mittelwert zu erhalten.

Werden bei der Messung an einzelnen Pigmenten innerhalb einer Schicht, vorzugsweise der äußeren Schicht, eine konstante Metall-/Sauerstoffzusammensetzung ermittelt, so handelt es sich im Rahmen dieser Erfindung um eine weitgehend homogene Zusammensetzung dieser Schicht. Dies schließt jedoch etwaige Inhomogenitäten, die nicht mittels dieser Meßmethode ermittelt werden können, mit ein.

Der Sauerstoffgehalt der Schichten lässt sich auch durch weitere Methoden ermitteln. Beispielsweise kann er mittels EDX-Analyse (energy dispersive X-ray analysis) bestimmt werden. Vorzugsweise verwendet man hier ein Gerät, welches in ein Elektronmikroskop integriert ist, beispielsweise das EDAX Genisis, Version 3.60, Fa. EDAX.

Im Folgenden wird die prinzipielle Vorgehensweise zur Bestimmung der Elementzusammensetzung der Pigmente mit dieser Methode erläutert:

Bei der EDX-Analysemethode dringt der abbildende Elektronenstrahl des Elektronenmikroskops, abhängig von seiner Energie und dem Material, ein Stück weit in die Probenoberfläche ein und gibt seine Energie an die dort befindlichen Atome ab. Aufgrund der hohen Energie der Strahlelektronen werden Elektronen aus den kernnahen Schalen (K- oder L-Schale) der angeregten Atome herausgeschlagen. Bei diesem Vorgang entsteht durch zweierlei Mechanismen Röntgenstrahlung. Die starke Abbremsung der Elektronen erzeugt eine kontinuierlich verteilte Röntgenstrahlung, die Bremsstrahlung, und die Wiederauffüllung der Schalen durch äußere Elektronen erzeugt aufgrund damit verbundener Emissionsvorgänge ein diskretes Röntgenspektrum. Dieses ergibt das charakteristische Linienspektrum des Atoms, welches eine eindeutige Identifizierung der Elemente ermöglicht.

Das von der zu untersuchenden Probe emittierte Röntgenstrahlungsspektrum wird mittels eines energiedispersiven Röntgenspektrometers gemessen. Es besteht aus dem Bremsstrahlungsuntergrund und einer Reihe von Röntgenspektrallinien. Anhand der Lage der Linien können die emittierenden Elemente bestimmt werden, die Höhe der Linien ist ein Maß für ihre relativen Gehalte in der Probe.

Bei der EDX-Elementanalyse sind für eine korrekte Messung der Elementgehalte einige wichtige Randbedingungen zu beachten. Die zu untersuchenden Proben müssen:

a) in ihrer Zusammensetzung homogen sein,
b) sie müssen so dick sein, dass der abbildende Elektronenstrahl vollständig in der Probe absorbiert wird und
c) dem Elektronenstrahl ohne störende Einflüsse einer Matrix und/oder eines Untergrundes frei zugänglich sein.

Je höher die Ordnungszahl der Elemente ist, desto stärker sind auch die kernnahen Elektronen gebunden. Die zur Ionisation erforderliche Energie steigt daher mit der Ordnungszahl an. Die kinetische Energie des Elektronenstrahls muss an die zu analysierenden Elemente angepasst werden. Die Eindringtiefe des Elektronenstrahls in das zu untersuchende Material hängt jedoch von seiner Energie ab. Der Elektronenstrahl dringt in die Probe in einer Intensitätsverteilung, die eine birnenförmige Struktur aufweist und auch als Anregungsbirne bezeichnet wird. Bei der Analyse dünner Schichten ist zu beachten, dass diese von hochenergetischen Elektronen leicht durchschlagen werden können. Sollen dünne Schichten, also Schichten in einem Bereich von unter 250 nm vermessen werden, darf die kinetische Energie nur wenige keV betragen. Daher muss konsequenterweise bei schwereren Elementen auf die Anregung der höheren Schalen ausgewichen werden. Die Analyse muss dann über die Auswertung der L- oder M-Linien der Elemente erfolgen.

Im Einzelnen geht man bei der Analyse dünnschichtiger, plättchenförmiger Pigmente folgendermaßen vor: Die EDX-Messeinheit wird vor der Analyse mit Hilfe geeigneter, kommerziell erhältlicher Standards gemäß Herstellerangaben kalibriert.

Mittels elektronenmikroskopischer Abbildungen ist die Schichtdicke der zu untersuchenden Schicht zu bestimmen. Eine Elementaranalyse bei höherer Spannung von ca. 10 bis 20 kV gibt Aufschluss über alle in der untersuchten Probe vorhandenen Elemente sowie zusätzlich über weitere im darunter liegenden Substrat befindliche Elemente. Anhand der Stärke und der Elementzusammensetzung der Schicht wird mit Hilfe einer Monte-Carlo-Simulation (bevorzugt: Programm: EDAX Flight-E, Version 3.1-E) die Elektronenenergie ermittelt, bei der das Schichtvolumen vom eindringenden Elektronenstrahl vollständig ausgefüllt, aber noch nicht durchschlagen wird. In diesem Fall hat die Anregungsbirne das größte Volumen.

Im nächsten Schritt ist zu ermitteln, ob und wenn ja welche Röntgenlinien bei dieser Strahlenenergie angeregt werden. Eventuell muss die kinetische Anregungsenergie noch etwas an die Spektrallinien angepasst werden.

Eine erste Probemessung mit den so ermittelten Parametern ist durchzuführen und zu analysieren. Sind im Spektrum Röntgenlinien von Substratelementen zu erkennen, so ist die eingestellte Strahlenenergie zu hoch und muss korrigiert werden.

Nun werden mehrere Messungen an der Schicht mit schrittweise steigender Strahlenspannung durchgeführt und ausgewertet. Die ermittelten Elementgehalte sollten nur geringfügig schwanken. Wenn der Anteil leichter Elemente in der Analyse mit steigender Spannung merkbar abzufallen beginnt, ist die Strahlenenergie zu hoch und muss verringert werden.

Mit den so ermittelten optimalen Parametern werden an mehreren Stellen der Schicht Messungen durchgeführt und die Elementgehalte ermittelt. Die Messergebnisse sind auf Plausibilität zu prüfen und die Streuung der Messwerte sollte nicht mehr als ca. 5 % betragen.

Um das Schichtdicken/Sauerstoffprofil an der gesamten Schichtabfolge des erfindungsgemäßen Pigmente analysieren zu können, kann die Eindringtiefe in die Schicht des Pigments durch schrittweise Erhöhung der Anregungsenergie des Elektronenstrahls (der "Anregungsbirne"), wie in Abbildung 2 schematisch dargestellt, erreicht werden. Die Eindringtiefe des Elektronenstrahls wird dabei jeweils vorgegeben und der Oxidgehalt dazu bestimmt.

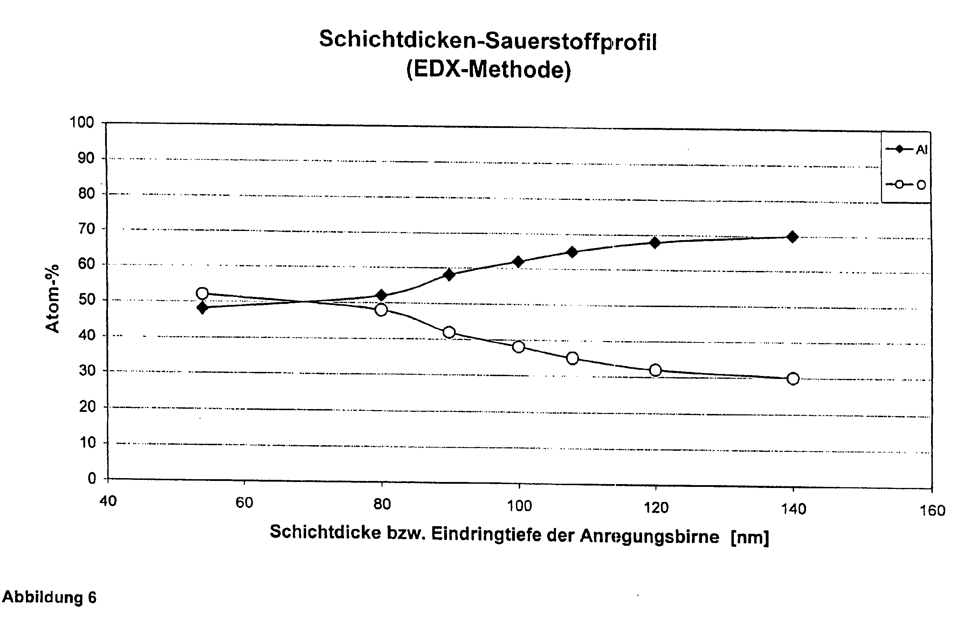

So können auch die tiefer liegenden Schichten in die Analyse des Sauerstoff- bzw. Metallgehaltes mit einbezogen werden. In Abbildung 6 ist das mit dieser Methode erstellte Schichtdicken- und Sauerstoffprofil von gemäß dem erfindungsgemäßen Beispiel 10 hergestellten Effektpigmenten dargestellt.

Diese Methode kann sehr gut zur Bestimmung des Sauerstoffgehaltes in den Schichten A und/oder C herangezogen werden. Jedoch ist es schwierig, die mittlere Schicht B zu analysieren, da hier das Signal immer auch die oberste Schicht erfasst. Daher erhält man entsprechend dem Intensitätsprofil der "Anregungsbirne" des Elektronenstrahls gefaltete Werte der beiden ersten Schichten.

Die Analyse einzelner Schichten kann jedoch durchgeführt werden, wenn diese Methode ebenfalls mit Sputtertechniken kombiniert wird. Hierbei muss, wie oben beschrieben, die Anregungsenergie des Elektronenstrahls jeweils auf jede einzelne Schicht optimiert werden.

Auch kann man durch entsprechende Fokussierung des anregenden Elektronenstrahls auf einzelne Pigmente diese im einzeln analysieren. Auf diese Weise kann die Analyse der Zusammensetzung der Schichten A und C erfolgen. Hierbei sind ebenfalls mindestens fünf einzelne Pigmente zu analysieren, um einen repräsentativen Mittelwert zu erhalten.

Der dreischichtige Aufbau mit den äußeren Schichten mit einem hohen Sauerstoffgehalt bewirkt, dass sich die erfindungsgemäßen metallischen Effektpigmente hinsichtlich ihrer mechanischen Eigenschaften insgesamt eher spröde, vergleichbar mit Oxiden oder Gläsern verhalten.

Im Vergleich zu den bekannten PVD-Metallpigmenten besitzen sie damit anwendungstechnische Vorteile. Konventionelle PVD-Metallpigmente neigen aufgrund ihrer Dünnheit und der Duktilität des Metalls dazu, sich aufzurollen. Sie können sich aufgrund ihrer Flexibilität zwar hervorragend an einen Untergrund "anschmiegen", jedoch kann in einer Applikation nach Aushärtung des Bindemittels immer ein kleiner Anteil der Pigmente verformt, im Extremfall gar "aufgerollt" sein. Mit diesem Phänomen gehen Verluste der optischen Qualität einher. Die Fähigkeit zum Aufrollen ist insbesondere beim Herstellungsprozess der Pigmente nach Ablösen von der Folie störend. Nach dem nachfolgenden Zerkleinern der Pigmente sind zwar weniger Pigmente aufgerollt, jedoch ist der Effekt teilweise noch vorhanden und kann später in der Applikation stören. Weiterhin sind diese Pigmente mechanisch labil, was sich beispielsweise in einer Empfindlichkeit gegenüber der Einwirkung starker Scherkräfte äußert.

In der WO 99/35194 sind metallische PVD-Pigmente beschrieben, die zur Verbesserung ihrer mechanischen Eigenschaften beidseitige Stützschichten aus einem Dielektrikum, wie beispielsweise $SiO_2$, tragen. Die Dielektrikaschichten werden ebenfalls durch PVD-Verfahren auf die Metallschicht aufgebracht. Dies ist jedoch ein sehr umständliches Verfahren, wobei die Wirtschaftlichkeit insbesondere darunter leidet, dass Metalloxide wesentlich langsamere Verdampfungsgeschwindigkeiten besitzen als Metalle.

Bei den erfindungsgemäßen farbigen metallischen Effektpigmenten ist die gewünschte mechanische Steifheit durch den mehrschichtigen Aufbau und auch den bei einer erfindungsgemäßen Ausführungsform hohen Sauerstoffgehalt der äußeren Schichten A und C realisiert. Die zerkleinerten Pigmente rollen sich nicht auf und zeigen entlang ihrer Längsausdehnung ein sehr homogenes Erscheinungsbild mit glatten Oberflächen ohne Wellen oder Ausbeulungen. Der hohe Sauerstoffgehalt der Schichten A und C sowie optional auch B bewirkt hinsichtlich der mechanischen Eigenschaften eine weitgehend glas- bzw. keramikartig Steifheit dieser Schichten. Daher lassen sich diese Pigmente nach Ablösen von der Folie hervorragend zerkleinern. Es entstehen Effektpigmente mit sehr glatten Bruchkanten. Dies wirkt sich vorteilhaft auf die optischen Eigenschaften der Pigmente aus, da aufgrund der glatten Bruchkanten geringere Streuzentren für das einfallende Licht vorhanden sind.

Die erfindungsgemäßen Effektpigmente sind stets plättchenförmig. Ihre Längsausdehnung, ausgedrückt durch

den $D_{50}$-Wert der Summendurchgangsverteilung, liegt in den üblichen Bereichen von 2 bis 250 μm, bevorzugt von 5 bis 150 μm und besonders bevorzugt von 7 bis 50 μm. Gemessen wird die Größenverteilung üblicherweise durch Laserbeugungsmethoden.

Die erfindungsgemäßen metallischen Effektpigmente weisen Formfaktoren von 4 bis 8500, bevorzugt von bis 10 bis 5000 und besonders bevorzugt von 20 bis 1700 auf. Der Formfaktor ist definiert als das Verhältnis des $d_{50}$-Wertes der Summendurchgangskurve der Größenverteilung zur mittleren Dicken der plättchenförmigen Pigmente.

Die erfindungsgemäßen Effektpigmente weisen überraschenderweise eine außerordentlich hohe Farbsättigung bzw. ein starkes Chroma und in vielen Ausführungsformen einen starken Helligkeitsflop auf. Dieses kann anhand von Rakelabzügen bestimmt werden. Die Rakelabzüge werden durchgeführt in vorzugsweise konventionellen, d.h. wasserfreien Lacksystemen ohne Zusatz weiterer Effekt- und/oder Farbpigmente oder Mattierungsmittel. Besonders bevorzugt verwendet man Nitrolacke, wie beispielsweise der kommerziell erhältliche Nitrolack Dr. Renger Erco Bronzemischlack 2615e; Fa. Morton. Die Konzentration des Effektpigmentes sollte hoch genug sein, um einen deckenden Rakelabzug zu erhalten und ist abhängig von der Art des Metalls, des Metallgehaltes und der Schichtdicke (siehe auch Tabelle 5).

Weiterhin zeigen die Pigmente gemäß den erfindungsgemäßen Beispielen und im Besonderen die goldfarbenen Pigmente durch den oxidischen Einbau in den äußeren Schichten einen sehr starken Helligkeitsflop.

Der Helligkeitsflop ist dabei von DuPont nach folgender Formel (II) festgelegt (A.B.J. Rodriguez, JOCCA, (1992 (4)) S. 150 - 153):

$$Flopindex = 2{,}69 \times \frac{(L_{15°}^{\bullet} - L_{110°}^{\bullet})^{1{,}11}}{(L_{45°}^{\bullet})^{0{,}86}} \qquad (II)$$

Die Flopwerte bzw. Flopindizes der erfindungsgemäßen Effektpigmente liegen im Bereich von 30 bis 70, bevorzugt bei 35 bis 65 und besonders bevorzugt bei 40 bis 60.

Die erfindungsgemäßen Effektpigmente erscheinen nahe dem Glanzwinkel noch relativ hell, vergleichbar mit durch konventionelle Mahlverfahren hergestellte Aluminiumpigmente. Bei steileren Beobachtungswinkeln fallen die Helligkeitswerte jedoch sehr schnell ab und erreichen bei 45° bereits Werte, die nahe an schwarzen Beschichtungen heranreichen. Dadurch ergeben sich derart außerordentlich hohe Flopwerte.

Von hochglänzenden Aluminiumpigmenten, die durch PVD-Verfahren gewonnen werden, und die die bislang brillantesten Effektpigmente darstellen, sind in entsprechenden Applikationen hingegen Flopwerte im Bereich von 20 bis ca. 30 bekannt.

Somit liegen die Flopwerte der erfindungsgemäßen Effektpigmente im Allgemeinen deutlich höher, überlappen sich jedoch teilweise mit denen von aus dem Stand der Technik bekannten Pigmenten. Zur weiteren Charakterisierung der optischen Eigenschaften der erfindungsgemäßen Pigmente werden als Maßzahl die Helligkeiten $L^{*}_{45°}$ herangezogen. Die $L^{*}_{45°}$-Werte der erfindungsgemäßen Effektpigmente liegen im Bereich von 1 bis 25, bevorzugt von 1,4 bis 20 und besonders bevorzugt von 1,7 bis 12 Helligkeitseinheiten. Diese sehr geringen Werte belegen den starken Flop ins Dunkle. Bekannte metallische PVD-Pigmente besitzen $L^{*}_{45°}$-Werte von über 35.

Weiterhin besitzen die erfindungsgemäßen metallischen Effektpigmente eine hohe Farbsättigung, was sich farbmetrisch in einem Chroma-Wert widerspiegelt. Das Chroma gemessen bei einem Differenzwinkel von 15° beträgt mehr als 20, bevorzugt mehr als 30 und besonders bevorzugt mehr als 40 Einheiten.

Bei einer besonders bevorzugten Ausführungsform ist das Metall $M_A$ der Schicht A und das Metall $M_C$ der Schicht C von der gleichen Art. Derartige Pigmente lassen sich besonders einfach herstellen, wie untenstehend näher erläutert wird.

Bei einer weiterhin bevorzugten Ausführungsform sind die Metalle $M_A$ der Schicht A, $M_B$ der Schicht B und $M_C$ der Schicht C von der gleichen Art.

Weiterhin ist bevorzugt, dass die mittleren Schichtdicken der Schichten A und C im wesentlichen gleich sind. Hierbei sind Abweichungen der mittleren Schichtdicken von A und C von bis zu 10 % gemeint. Besitzen diese Schichten eine annähernd gleiche Schichtdicke und sind darüber hinaus von der gleichen Art, so handelt es sich um symmetrisch aufgebaute metallische Effektpigmente. Diese bewirken in einer Applikation über besonders reine und deutliche Farbeffekte. Metallische Effektpigmente mit einem symmetrischen Aufbau sind erfindungsgemäß bevorzugt.

Nachfolgend werden weitere bevorzugte Ausführungsformen der Erfindung beschrieben.

Beispielhafte Ausführungsform A

Aufbau: Oxidisches-Cr/Ag/Oxidisches-Cr oder Oxidisches-Cr/Al/Oxidisches-Cr

Bei diesen bevorzugten Ausführungsformen ist das farbige metallische Effektpigment dadurch gekennzeichnet, dass das Metall $M_A$ und/oder $M_C$ im wesentlichen aus Chrom besteht und einen durchschnittlichen Gehalt an Sauerstoff von 35 bis 48 Atom-%, bezogen auf den Gesamtgehalt an Chrom und Sauerstoff, besitzt. Das Metall $M_B$ besteht bei diesen Ausführungsformen bevorzugt aus Aluminium und/oder Silber.

In beiden Fällen können äußerst attraktive goldfarbene metallische Effektpigmente durch Einstellung der Schichtdicke der oxidischen Cr-Schichten erzeugt werden. In einem Schichtdickenbereich von 10 nm - 35 nm für die oxidischen Cr-Schichten werden Goldtöne von Hellgold (Schicht B: Aluminium) bzw. Silberbeige (Schicht B: Silber) bis sehr intensives rötliches Gold erzeugt. Auch kann beispielsweise der Farbton von Echtgold erreicht werden. Vorzugsweise werden bei Verwendung von Silber oder Al als Schicht B Schichtdicken in einem Bereich von 50 - 100 nm, besonders bevorzugt von 15 - 40 nm, verwandt.

Beispielhafte Ausführungsform B

Aufbau: Oxidisches-Al/Ag/Oxidisches-Al oder Oxidisches-Al/Al/Oxidisches-Al

Bei diesen bevorzugten Ausführungsform ist das farbige metallische Effektpigment dadurch gekennzeichnet, dass das Metall $M_A$ und/oder $M_C$ im wesentlichen aus Aluminium besteht und einen durchschnittlichen Gehalt an Sauerstoff von 30 bis 55 Atom-%, bezogen auf den Gesamtgehalt an Aluminium und Sauerstoff, besitzt. Das Metall $M_B$ besteht bei diesen Ausführungsformen bevorzugt aus Aluminium oder Silber.

Eine weitere bevorzugte Ausführungsform der farbigen metallischen Effektpigmente ist hierbei durch die Schichtabfolge Oxidisches-Al/Al/Oxidisches-Al gegeben, die sich dadurch auszeichnet, dass das Metall $M_A$ identisch mit Metall $M_B$ und identisch mit Metall $M_C$ ist.

Die Wirkungsweise der äußeren oxidischen Aluminiumschichten ist der der oxidischen Cr-Schichten der oben beschriebenen Variante A ähnlich. Es können die gleichen Farbtöne erzielt werden.

In einem Schichtdickenbereich von 10 nm - 60 nm für die oxidischen Al-Schichten werden Goldtöne von Hellgold (Schicht B: Aluminium) bzw. Silberbeige (Schicht B: Silber) bis sehr intensives rötliches Gold erzeugt.

Die intermediär liegende metallische Schicht B weist bei den beiden oben beschriebenen Varianten A und B jeweils einen Metallgehalt von 70 bis 100 Atom-%, bezogen auf den Gehalt an Metall und Sauerstoff in der Schicht B, auf, wobei $M_B$ gleich oder verschieden von $M_A$ sein kann.

Als Metall $M_B$ in der Schicht B wird bei den bevorzugten Ausführungsformen Aluminium und/oder Silber verwendet, wobei der Sauerstoffgehalt vorzugsweise in einem Bereich von 30 bis 0 Atom-%, weiter bevorzugt von weniger als 25 bis 0 Atom%, noch weiter bevorzugt von weniger als 20 bis 0 Atom-%, noch weiter bevorzugt von weniger als 10 bis 0 Atom-%, jeweils bezogen auf den Metall- und Sauerstoffgehalt in der Schicht B, beträgt.

Der Metallgehalt der Schicht B liegt für Aluminium und/oder Silber vorzugsweise bei 70-100 Atom-%, bezogen auf das Aluminium bzw. das Silber sowie etwaig vorhandenen Sauerstoff in der Schicht B. Unterhalb des genannten Metallgehalts für Silber oder auch Aluminium kann die zentrale Schicht B zu transparent wirken. Bei Verwendung von Silber und/oder Aluminium in der Schicht B wird bevorzugt eine Schichtdicke im Bereich von 50 -100 nm, besonders bevorzugt von 20 - 40 nm, verwandt.

Um die erfindungsgemäßen Effektpigmente insbesondere für den Einsatz in Wasserlacken oder wässrigen Druckfarben zu stabilisieren, können sie optional mit einer Korrosionsschutzschicht, vorzugsweise umhüllend, beschichtet werden.

Hier sind die gängigen Methoden anwendbar wie die Behandlung mit organisch modifizierten Phosphorsäuren und/oder Phosphonsäuren und/oder deren Derivaten. Für den Fall, dass die äußeren Schichten A und C aus Aluminium bestehen, kann eine Chromatierung in Anlehnung an das in der EP 0 259 592 B1 offenbarte Verfahren durchgeführt werden. Weiterhin kann eine Behandlung der Pigmentoberflächen mit Vanadium- und/oder Molybdänverbindungen sowie eine Kombination dieser Methoden durchgeführt werden. Ferner können die erfindungsgemäßen Pigmente auch mit Polymeren oder Metalloxiden beschichtet werden. Die Metalloxide umfassen bevorzugt $SiO_2$, Boroxide, Aluminiumoxide, Molybdate, Vanadate und schließen deren Hydroxide und Oxidhydrate oder Mischungen derselben ein.

Bei einer besonders bevorzugten Ausführungsform umfasst die, vorzugsweise umhüllende, Korrosionsschutzschicht $SiO_2$ oder besteht aus $SiO_2$. Besonders bevorzugt wird die $SiO_2$-Schicht mit Sol-Gel-Methoden auf das Effektpigment umhüllend aufgebracht.

Bei weiteren bevorzugten Ausführungsformen können die mit einer Korrosionsschicht vor Korrosion geschützten erfindungsgemäßen Effektpigmente auch zusätzlich organisch-chemische Oberflächenmodifikationen, wie beispielsweise Silane, Titanate oder Aluminate aufweisen. Die organischchemischen Oberflächenmodifikationen können eine Kompatibilisierung mit dem umgebenden Applikationsmedium, beispielsweise das Bindemittelsystem eines Lackes oder einer Farbe, bewirken. Beispielsweise kann eine solche organisch-chemische Nachbeschichtung eine chemische Anbindung an Bindemittel von Lacken oder Farben ermöglichen, wodurch eine kovalente Anbindung der erfindungsgemäßen Effektpigmente ermöglicht wird. Eine kovalente Anbindung der Effektpigmente an das Bindemittelsystem erhöht die Schwitzwasserbeständigkeit und mechanische Beständigkeit des Beschichtungsmediums, beispielsweise von Farben und Lacken, nach Aushärtung.

Im Folgenden werden verschiedene Verfahren zur Bereitstellung der erfindungsgemäßen Effektpigmente beschrieben.

Bei dem zur Herstellung der erfindungsgemäßen Effektpigmente verwendeten Verfahren handelt es sich immer um PVD-Verfahren, d.h. dass die einzelnen Schichten, im Falle eines dreischichtigen Systems die Schichten A, B und C, durch PVD-Verfahren nacheinander und vorzugsweise aufeinander aufgebracht werden.

Vorzugsweise umfasst ein Verfahren zur Herstellung der erfindungsgemäßen metallischen Effektpigmente dabei folgende Schritte:

a) Bedampfen eines, vorzugsweise beweglichen, Substrates in einer Vakuumkammer mittels physical vapour deposition (PVD) mit wenigstens einem Metall $M_A$ in Gegenwart von Sauerstoff unter Bildung der Schicht A auf dem Substrat,

b) Bedampfen der Schicht A in einer Vakuumkammer mittels physical vapour deposition (PVD) mit wenigstens einem Metall $M_B$ in Gegenwart oder Abwesenheit von Sauerstoff unter Bildung der Schicht B,

c) Bedampfen der Schicht B in einer Vakuumkammer mittels physical vapour deposition (PVD) mit wenigstens einem Metall $M_C$ in Gegenwart von Sauerstoff unter Bildung der Schicht C,

d) Ablösen des metallischen Schichtpaketes vom Substrat,

e) Zerkleinern des metallischen Schichtpaketes zu metallischen Effektpigmenten,

f) optional Überführen der metallischen Effektpigmente in eine Dispersion oder Paste.

Die Dispersion liegt vorzugsweise ebenfalls in einem organischen Lösemittel vor. Bei einer Variante beträgt der Anteil an organischem Lösemittel in der Dispersion mindestens 70 Gew.-%.

Die erfindungsgemäßen Effektpigmente können aber auch in konzentrierterer Form vorliegen, beispielsweise als Paste. Hierbei beträgt der Lösemittelgehalt bis zu 60 Gew.-%, bevorzugt bis zu 50 Gew.-%, bezogen auf die Metalleffektpigmentpaste.

Die erfindungsgemäßen metallischen Effektpigmente können auch in einer kompaktierten Form als Pellets, Granulat, Tabletten, Würstchen, Briketts, etc. vorliegen, wobei der Lösemittelgehalt vorzugsweise weniger als 10 Gew.-%, weiter bevorzugt zwischen 3 und 8 Gew.-%, jeweils bezogen auf das Gewicht der kompaktierten Form vorliegt.

Das Bedampfen mit Metall kann durch bekannte Verfahren wie beispielsweise Elektronenstrahltechnologie oder widerstands- bzw. strahlungsbeheizte Verfahren erfolgen. Bei diesen bekannten Verfahren werden die Metalle in entsprechenden Verdampfern angeordnet und aus diesen verdampft, wobei sich der Metalldampf auf einem beweglichen oder unbeweglichen Substrat niederschlägt.

Die Dicke der außenliegenden Metallschichten A und/oder C kann während des Aufbringens durch Transmissionsmessungen kontrolliert werden. Die Transmissionen sind meist aufgrund des teilweise oxidischen Charakters der Folienschicht geringer als bei der Verdampfung reiner Metalle. Die Kontrolle der Schichtdicke der zentralen Schicht B kann beispielsweise über Online-Schichtwiderstandsmessungen erfolgen.

Wird statt eines Metalls M eine Legierung verwendet, so wird diese Legierung entweder aus separaten Verdampfern, beispielsweise durch Flashverdampfung, Springstrahlverdampfern, o.ä., durch simultane Kondensation der Metalle oder mittels geeigneter Verdampfungsmethoden wie beispielsweise Sputtern oder Elektronenstrahltechnologie hergestellt.

Das bewegliche Substrat kann aus Polymerfolien, wie beispielsweise Polyterephthalat oder aus einem Metallband, welches bevorzugt ein umlaufendes Metallband ist, bestehen.

Der Sauerstoff beim Bedampfungsschritt kann durch eine Vielzahl möglicher Verfahrensvarianten zur Verfügung gestellt werden.

Diese können die Bereitstellung von molekularem Sauerstoff aus atmosphärischem Sauerstoff oder auch die kontrollierte Zudosierung von Sauerstoffgas in die Vakuumkammer umfassen. Im letzteren Fall könnte man von einer reaktiven PVD-Bedampfung sprechen.

Vorzugsweise wird reiner Sauerstoff in die Vakuumkammer zugeführt, da sich feinere metallische Phasen bzw. feinere Metalloxidphasen ausbilden, wodurch der Farbeindruck der metallischen Effektpigmente feiner gesteuert werden kann.

Als Sauerstoffquelle kommt auch Wasser in Frage, welches noch in der Vakuumkammer vorhanden ist oder gezielt eingebracht wird. Das Wasser kann dabei atmosphärischen Ursprung haben oder aus einem Wasserspeicher stammen. Als Wasserspeicher kommen beispielsweise Hydratverbindungen in Frage. Die Hydratverbindung muss für sich genommen hinreichend schwer flüchtig sein, um unter den vorhandenen Vakua nicht zu sublimieren. Vorteilhafterweise gibt die Hydratverbindung jedoch, ggfs. unter Wärmezufuhr, Wasser ab. Weiterhin kann während der Bedampfung Wasserdampf in die Vakuumkammer kontrolliert zudosiert werden.

Hierbei ist zu bemerken, dass Wasser unter den niedrigen Drücken einer Vakuumbeschichtungskammer in fester Form vorliegt. Daher muss das Wasser durch eine geeignete Heizquelle sublimiert werden. Diese kann beispielsweise in den Verdampferquellen bei einer widerstandsbeheizten Metallverdampfung liegen, durch die eine nicht unbeträchtliche Wärme erzeugt wird.

Ferner kann Sauerstoff aus dem vorzugsweise beweglichen Substrat, beispielsweise einer beweglichen Folie, auf die zunächst ein Releasecoat und anschließend das Metall aufgedampft wird, stammen. Selbstverständlich kann der Sauerstoff auch durch eine beliebige Kombination dieser Verfahrensmöglichkeiten zur Verfügung gestellt werden.

Vorzugsweise ist der Vakuumdruck bei Schritt a) relativ hoch. Bevorzugt liegt der Vakuumdruck bei $2 \times 10^{-4}$ bis $1 \times 10^{-1}$ mbar, besonders bevorzugt bei $3 \times 10^{-4}$ bis $1 \times 10^{-2}$ mbar und weiterhin besonders bevorzugt bei $4 \times 10^{-4}$ bis $3 \times 10^{-3}$ mbar. Dunkle Schichten werden bevorzugt bei höheren Drücken erhalten.

Herkömmlicherweise werden bei PVD-Verfahren zur Herstellung von Metallpigmenten Drücke von $1 \times 10^{-4}$ mbar und weniger eingestellt. Des Weiteren werden keine Sauerstoff-abgebenden Sauerstoffquellen in der Vakuumkammer angeordnet oder in diese eingebracht.

Gemäß einem bevorzugten Verfahren wird die Beschichtung mittels in Reihe geschalteter Verdampfungsquellen aufgebracht.

Diese Verfahrensvariante zur Herstellung der erfindungsgemäßem metallischen Effektpigmente umfasst folgende Schritte:

a) Bedampfen eines, vorzugsweise beweglichen, Substrates, vorzugsweise eines umlaufenden oder sich bewegenden Bandes, in einer Vakuumkammer mit wenigstens dem Metall $M_A$ aus einer Verdampferquelle $VQ_A$ in Gegenwart einer Sauerstoff-abgebenden Sauerstoffquelle unter Bildung der Schicht A,

b) Bedampfen der Schicht A auf dem, vorzugsweise beweglichen, Substrat, vorzugsweise eines umlaufenden oder sich bewegenden Bandes, in einer Vakuumkammer mit wenigstens dem Metall $M_B$ aus einer Verdampferquelle $VQ_B$ in Gegenwart oder Abwesenheit einer Sauerstoffquelle unter Bildung der Schicht B,

c) Bedampfen der Schicht B auf dem, vorzugsweise beweglichen, Substrat, vorzugsweise eines umlaufenden oder sich bewegenden Bandes, in einer Vakuumkammer mit wenigstens dem Metall $M_C$ aus einer Verdampferquelle $VQ_C$ in Gegenwart einer Sauerstoffquelle unter Bildung der Schicht C,

d) Ablösen des metallischen Schichtpaketes von dem, vorzugsweise beweglichen, Substrat, vorzugsweise eines umlaufenden oder sich bewegenden Bandes,

e) Zerkleinern des metallischen Schichtpaketes zu metallischen Effektpigmenten,

f) optional Überführen der metallischen Effektpigmente in eine Dispersion oder Paste.

Gemäß einer bevorzugten Weiterbildung des erfindungsgemäßen Verfahrens sind die einzelnen Verdampferquellen $VQ_A$, $VQ_B$ und $VQ_C$ voneinander oder jeweils paarweise voneinander abgetrennt.

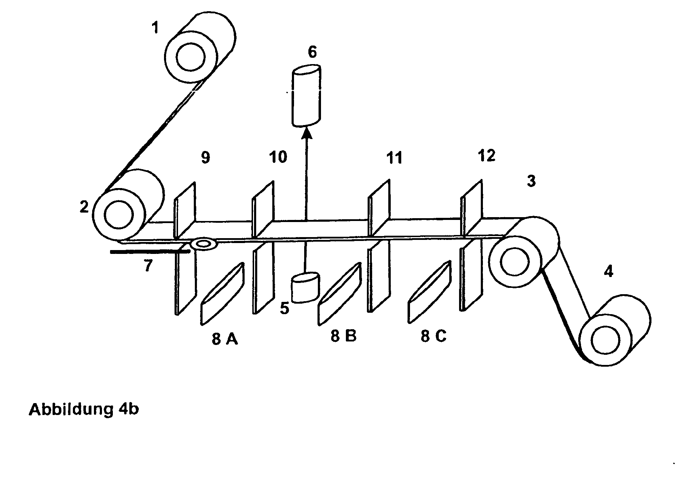

Optional kann das Verfahren zur Herstellung eines metallischen Effektpigmentes, wie in Abbildung 4b beispielhaft dargestellt ist, weiter modifiziert werden, indem die einzelnen Verdampferquellen 8A, 8B und 8C zur Verdampfung der Metalle durch Trenneinrichtungen wie Blenden oder Wände voneinander getrennt sind. Abbildung 4b zeigt die schematische Darstellung bei der Beschichtung eines bandförmigen Substrats über drei Verdampferquellen 8A, 8B und 8C. Dabei wird das bewegliche und mit einem Releasecoat versehene Substrat, beispielsweise eine mit Releasecoat versehene Folie, von der Quellrolle 1 über die Umlenkrollen 2 und 3 zu dem Rollenaufnehmer 4 geführt. Die Anordnung ist innerhalb einer Vakuumkammer (nicht dargestellt) angeordnet. Unterhalb des beweglichen Substrates sind die Verdampferquellen 8A, 8B und 8C angeordnet, aus denen das Metall oder die Metalle in Richtung des beweglichen Substrats verdampft werden. Die Vakuumkammer ist bei dieser Ausführungsform durch die Trennwände 9-12 unterteilt. Die Sauerstoff-abgebenden Sauerstoffquellen können dabei beispielsweise beabstandet zu den Verdampferquellen 8A innerhalb der Trennwände 9 und 10 und zu 8C innerhalb der Trennwände 11 und 12 angeordnet werden. Falles gewünscht können auch Sauerstoffquellen beabstandet zur Verdampferquelle 8B angeordnet werden. Hierbei ist im einzelnen der Abstand der Sauerstoffquellen zu dem jeweiligen Verdampfer und zum beweglichen Substrat ein wichtiger Parameter.

Es ist selbstverständlich auch möglich, Zuführungen in der Vakuumkammer vorzusehen, über die Sauerstoffhaltige Verbindungen, beispielsweise Sauerstoff oder Wasser, zugeführt werden. Über die Schwingquarzmessung 7 kann die Verdampfungsrate und über die Transmissionsmessung die Schichtdicke des aufgedampften Films kontrolliert werden.

Das in Abbildung 4b dargestellte Verfahren ist kontinuierlich; das bewegliche Substrat, vorzugsweise eine mit einem Releasecoat versehene Bandfolie, wird mit konstanter Geschwindigkeit durch die Vakuumkammer geführt. Die Schichtdicken der Schichten A, B und C können hierbei durch die Verdampfungsrate und/oder den Abstand der Verdampfungsquelle zu dem beweglichen Substrat eingestellt werden.

Die für die farbigen metallischen Effektpigmente benötigten Schichten A und C können beispielsweise durch einen größeren Abstand der Verdampferquellen 8A und 8C zum beweglichen Substrat, beispielsweise umlaufenden oder bewegten Band, ggf. in Kombination mit der gewählten Blendenanordnung realisiert werden, um geringere Schichtdicken zu erhalten. Auch können die Heizleistungen und damit die Ab- bzw. Aufdampfraten der Verdampferquellen individuell eingestellt werden.

Zur Erzeugung der metallischen Schicht B kann dann vorzugsweise der Abstand von der Verdampferquelle 8B geringer eingestellt werden, als dies bei den Verdampferquellen 8A und 8C der Fall ist, um eine möglichst hohe Metalldichte oder Schichtdicke zu erzeugen. Ebenso kann eine höhere Schichtdicke bei der metallischen Schicht B erhalten werden, wenn die Heizleistung bei der Verdampferquelle 8B erhöht bzw. die Abdampfrate verstärkt wird.

Der große Vorteil dieser besonders bevorzugten Verfahrensvariante liegt darin, dass die komplette Schichtabfolge des erfindungsgemäßen Pigments in einem einzigen Beschichtungsvorgang aufgebracht wird.

Eine weitere bevorzugte Verfahrensvariante ist dadurch gekennzeichnet, dass $M_A$, $M_B$ und $M_C$ gleich, paarweise gleich oder verschieden voneinander sind und aus einer Metallverdampfungsquelle oder mehreren Metallverdampfungsquellen ein bewegliches, Substrat, vorzugsweise ein umlaufendes oder sich bewegendes Band, in einer Vakuumkammer in Gegenwart von einer oder mehreren Sauerstoff-abgebenden Sauerstoffquellen mit Metall bedampft wird, wobei sich zwischen der Metallverdampfungsquelle, der Sauerstoffquelle und dem beweglichen Substrat dreidimensionale Konzentrationsbereiche aus Metalldampf und Sauerstoff in der Vakuumkammer ausbilden, wodurch auf dem beweglichen Substrat durch physikalische Dampfabscheidung die wenigstens drei Schichten A, B und C mit voneinander unterscheidbaren Metall- und Sauerstoffgehalten nacheinander abgeschieden werden (s. Abbildung 9).

Bei dieser weiteren bevorzugten Verfahrensvariante wird der Umstand ausgenutzt, dass sich bei der Verdampfung von Metallen, ausgehend von einer Verdampferquelle, jeweils annähernd konzentrische Kreise mit unterschiedlichen Metallkonzentrationen auf einem nicht beweglichen Substrat abscheiden lassen. Diese unterschiedlichen auf dem Substrat aufgedampften Metallkonzentrationen sind darauf zurückzuführen, dass die aus der Verdampferquelle abgegebenen Metallatome in dem Raum zwischen Verdampferquelle und Substrat aufgrund der unterschiedlichen Wegstrecken dreidimensionale Konzentrationsbereiche in Form von Verdampfungskegeln ausbilden.

Abbildung 9 zeigt schematisch einen Bereich mit hoher Metallkonzentration in Form einer kreisförmigen schwarz dargestellten Fläche und einen diesen Bereich konzentrisch umgebenden Bereich mit niedrigerer Metallkonzentration in Form einer ringförmigen schraffiert kariert dargestellten Fläche. Dieses konzentrische Konzentrationsprofil wird erhalten, wenn ein unbewegliches Substrat aus einer unter dem Substrat angeordneten Verdampferquelle mit Metall bedampft wird.

Gemäß einer bevorzugten Verfahrensvariante werden zwischen Metaliverdampfungsquelle, Sauerstoffquelle und beweglichem Substrat jeweils eine Abdeckvorrichtung, wie z.B. eine Blende, oder mehrere Abdeckvorrichtungen, wie z.B. Blenden, angeordnet, durch die die mögliche Ausbildung von Übergangsschichten zwischen den Schichten A, B und C unterdrückt werden, so dass die wenigstens drei aufeinander folgenden Schichten A, B und C mit jeweils voneinander unterscheidbaren Gehalten an Metall und Sauerstoff abscheiden können.

Durch Anordnung von Abdeckvorrichtungen, wie beispielsweise Blenden, können konzentriertere Metallbereiche von weniger konzentrierteren Metallbereichen getrennt werden. In Abbildung 10 ist eine beispielhafte Anordnung von Abdeckvorrichtungen in Form von Blenden gezeigt, wodurch definierte Bereiche mit geringer Metallkonzentration, als Schicht A gekennzeichnet, von einem Bereich mit hoher Metallkonzentration, als Schicht B bezeichnet, und wiederum von einem Bereich mit geringer Metallkonzentration, als Schicht C bezeichnet, auf dem Substrat voneinander abgetrennt ist.

Bei einem beweglichen Substrat, beispielsweise einer Bandfolie, die vom Blendeneingang I zum Blendenausgang II (siehe Abbildung 10) geführt wird, wird zunächst die Schicht A, darüber dann die Schicht B und schließlich darauf die Schicht C in einem kontinuierlichen Verfahren aufgebracht. Durch die Blenden I und II werden die Übergangskonzentrationen ausgeblendet, so dass die Schichten A, B und C scharfe Übergänge haben.

Gemäß einer weiteren bevorzugten Verfahrensvariante ist die wenigstens eine Sauerstoffquelle in Form von Wasser, Wasser-abgebenden Substanzen, Sauerstoff-abgebenden Substanzen und/oder Sauerstoffgas in der Vakuumkammer angeordnet oder die Vakuumkammer weist eine oder mehrere Zuführungen für Sauerstoff oder Sauerstoffabgebende Substanzen, beispielsweise Wasser oder Wasserdampf, auf.

Bevorzugt ist, dass während der Bildung der Schichten A und/oder C und optional während Schicht B eine kontrollierte Zudosierung von Sauerstoffgas in die Vakuumkammer erfolgt.

Abhängig von der gewünschten Zusammensetzung der jeweiligen Schicht sollte die Sauerstoffquelle in definiertem Abstand zum beweglichen Substrat und zur jeweiligen Verdampferquelle positioniert werden.

In die Randbereiche des Verdampfungskegels (siehe Abbildung 9) können Sauerstoff-haltige Verbindungen, wie Sauerstoff oder Wasser, eingebracht werden, so dass in den Schichten A und C der gewünschte Anteil an Sauerstoff realisiert wird. In Abhängigkeit von dem Ort der Zugabe der Sauerstoff-haltigen Verbindung kann die Schicht B ebenfalls Sauerstoff in unterschiedlichen, definierten Mengen enthalten. Positioniert man eine Sauerstoffquelle sehr nahe an die Verdampferquelle, so kann die Schicht B auch nahezu vollständig oxidisch gestaltet werden.

Es ist Sauerstoff auch bei sehr niedrigem Druck als Restgas in Form von Wasser in der Vakuumkammer vorhanden. Bei der Verdampfung eines Metalls kann in die äußeren, bereits mit Sauerstoff verdünnten Bereiche des Verdampfungskegels (Abbildung 9) kontrolliert Luft zudosiert werden, so daß diese Verdampfungsbereiche mit bereits geringerer Metallkonzentration zusätzlich mit Sauerstoff angereichert werden. Die mittige Schicht B verliert dabei ihre metallische Reflektoreigenschaft nicht, solange im Verfahren darauf geachtet wird, dass nur die äußeren Bereiche des Verdampfungskegels innerhalb der angegebenen Grenzen mit Sauerstoff "verdünnt" werden.

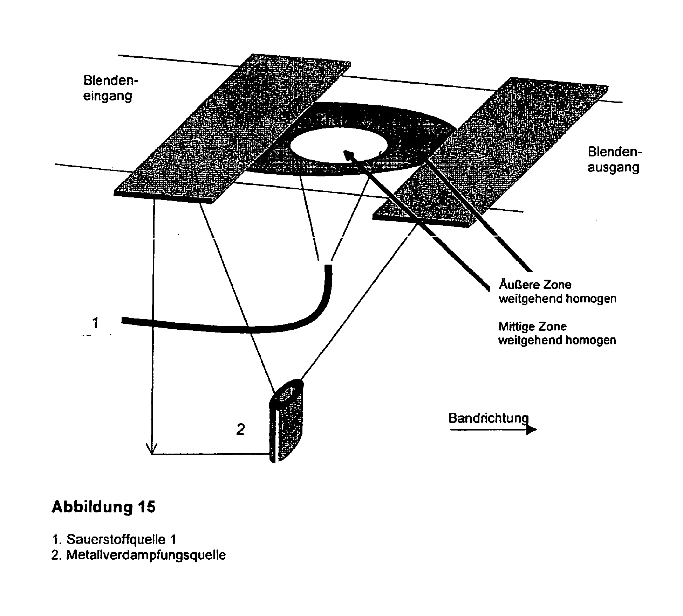

Eine alternative Variante wird in Abbildung 15 beschrieben. Hier wird eine mittige weitgehend oxidische Phase durch die zentrale Positionierung der Sauerstoffquelle erhalten. Bereiche des äußeren Verdampfungskegels werden weitgehend metallisch abgeschieden.

Ein großer Vorteil dieser Verfahrensvarianten liegt darin, dass ein dreischichtiges metallisches Effektpigment in nur einem einzigen Banddurchlauf durch das Verdampfen von Metall mit unterschiedlichen Sauerstoffkonzentrationen in unterschiedlichen Bereichen des Verdampfungskegels hergestellt werden kann.

Mit den erfindungsgemäßen Verfahrensvarianten können somit die Schichtdicken und der Sauerstoffgehalt der einzelnen Schichten in einem kontinuierlichen Verfahren in einem Schritt auf überraschend einfache Art und Weise effizient eingestellt werden.

Völlig überraschend war dabei, dass es genügt, in die bei der Verdampfung eines Metalls äußeren Bereiche des sich bildenden Verdampfungskegels Sauerstoff, Wasser oder Sauerstoff-haltige Verbindungen einzubringen, um einen mehrschichtigen Filmaufbau zu erhalten, bei dem die äußeren Schichten A und C einen größeren Anteil an Sauerstoff, verglichen mit der mittleren metallischen Schicht B, äufweisen, und nach der Ablösung und Zerkieinerung dieses Films optisch äußerst interessante metallische Effektpigmente erhalten werden können.

Der auf das Substrat, beispielsweise eine bandförmige Kunststofffolie, aufgebrachte dreischichtige Metallfilm kann in dieser Form aufbewahrt und transportiert werden. Um die erfindungsgemäßen metallischen Effektpigmente herzustellen, wird der dreischichtige Metallfilm von einem vorab aufgebrachten Releaselayer (Trennschicht) entfernt oder abgelöst und dabei oder nachfolgend, beispielsweise in einem chemisch nicht reaktiven organischen Lösemittel, zerkleinert. Danach können die so erhaltenen erfindungsgemäßen metallischen Effektpigmente konzentriert und/oder gewaschen werden, um beispielsweise Reste des Releaselayers zu entfernen. Gemäß einer weiteren bevorzugten Ausführungsform werden die so erhaltenen erfindungsgemäßen metallischen Effektpigmente mit einer Korrosionsschutzschicht, beispielsweise aus Kunststoff oder Metalloxid(en) wie $SiO_2$ versehen und/oder einer organischen Nachbeschichtung, um funktionelle Gruppen aufzubringen, die beispielsweise mit Bindemitteln eines Lacksystems oder einer Farbe unter Umsetzung regieren können, versehen.

Die erfindungsgemäßen Metallpigmente finden Verwendung in Coatings, Lacken, Automobillacken, Pulver-lakken, Druckfarben, Digitaldruckfarben, Kunststoffen oder kosmetischen Formulierungen, insbesondere in Nagellacken.

Die der Erfindung zugrunde liegende Aufgabe wird auch durch eine Beschichtungsmittel gelöst, das metallische Effektpigmente nach einem der Ansprüche 1 bis 25 enthält.

Gemäß einer bevorzugten Ausführungsform wird das Beschichtungsmittel aus der Gruppe, die aus Coatings, Lacken, Automobillacken, Pulverlacken, Druckfarben, Digitaldruckfarben, Kunststoffen und kosmetischen Formulierungen besteht, ausgewählt.

Die der Erfindung zugrunde liegende Aufgabe wird auch durch einen beschichteten Gegenstand, der mit metallischen Effektpigmenten nach einem der Ansprüche 1 bis 25 oder mit einem Beschichtungsmittel nach Anspruch 35 oder 36 versehen ist, gelöst.

Beispielsweise kann der Gegenstand eine Fahrzeugkarosserie, ein Fassadenelement, ein bedrucktes Substrat, beispielsweise Papier, Pappe, Folie, etc, oder ein künstlicher Fingernagel sein.

Im Folgenden wird die Herstellung der erfindungsgemäßen Effektpigmente anhand von Beispielen beschrieben, ohne die Erfindung zu beschränken.

**Beispiele 1-4:**

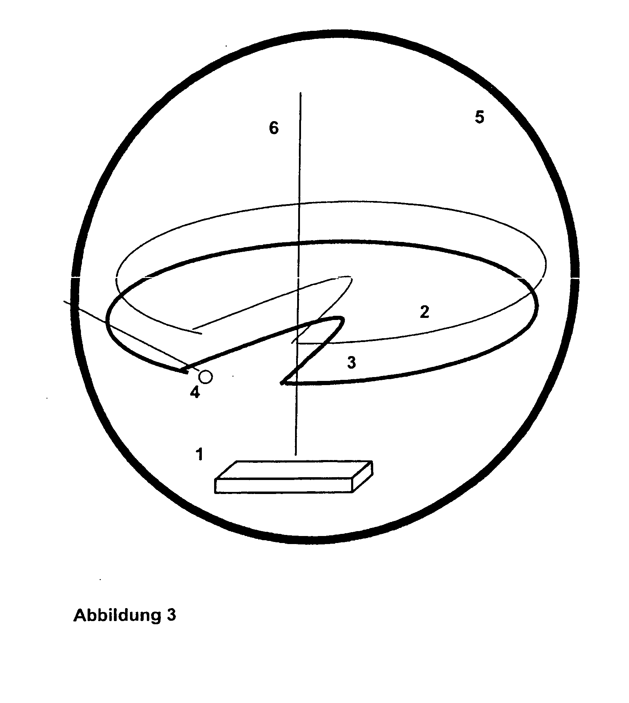

Die Herstellung der Effektpigmente der erfindungsgemäßen Beispiele 1- 4 und der erfindungsgemäßen Beispiele 5 und 6 erfolgte mit einer Drehtelleranlage, wie in Abbildung 3 gezeigt, in drei separaten Beschichtungsschritten:

Allgemeine Vorgehensweise:

Ein um eine Drehachse 6 drehbarer Drehteller 2 mit einem V-förmigen Ausschnitt wird mit einer Polyethylenterephthalat-(PET)-Folie von 23 μm Dicke, welche mit einem Releasecoat beschichtet ist, versehen und in der Vakuumkammer 5, wie in Abbildung 3 gezeigt, angeordnet. Der Releasecoat besteht aus acetonlöslichem Methylmethacrylatharz und wurde in einem separaten Arbeitsschritt vorab aufgebracht. Zwischen der Verdampferquelle 1 und dem mit der PET-Folie versehenen Drehteller 2 ist die feststehende Blende 3, die ebenfalls einen V-förmigen Ausschnitt aufweist, angeordnet. Die PET-Folie ist auf der der Verdampferquelle 1 zugewandten Seite mit dem Releasecoat (Trennschicht) versehen. Der in den V-förmigen Ausschnitt der Blende 4 ragende Schwingquarz 4 wurde zur Bestimmung der aufgedampften Mengen an Metall verwendet.

Mit der in Abbildung 3 gezeigten PVD-Laboranlage wurde in drei Durchgängen nacheinander eine erste Schicht A aus oxidhaltigem Chrom, eine zweite Schicht B aus Silber oder Aluminium und abschließend eine dritte Schicht C aus oxidhaltigem Cr aufgedampft. Die Metalle wurden dabei jeweils in der passenden Verdampferquelle 1 (siehe Abbildung 3) angeordnet.

Das Vakuum wurde durch zwei Rotationspumpen (Firma Edwards) und eine Diffusionspumpe (Firma Varian) erzeugt.

Die verwendeten Metalle wurden jeweils widerstandsbeheizt verdampft.

Die Massenbelegung wurde am Schwingquarz (Modell FTM7, Fa. Edwards), über die benötigte Heizleistung des zu verdampfenden Metalls gesteuert.

Der Verdampfungsvorgang wurde so kontrolliert, dass sich eine konstante Massenbelegung am Schwingquarz 4 (s. Abbildung 3) während der Beschichtung ergab.

Beschichtungsdauer und Kammervakuum wurden während den Beschichtungen konstant gehalten.

Bei den oxidischen Metallbeschichtungen der Schichten A und C der erfindungsgemäßen Beispiele dienten als Sauerstoffquelle mit Wasser getränkte Kartonstücke, die vor dem Evakuieren in die Vakuumkammer gelegt wurden.

Der Sauerstoffpartialdruck für die jeweiligen Schichten wurde über das konstant eingestellte Kammervakuum kontrolliert, welches der Tabelle 1 entnommen werden kann.

Über ein Datenerfassungsprogramm ließen sich die Prozessdaten (Massenänderung am Schwingquarz, Kammervakuum) während der Beschichtung aufzeichnen.

Die pro Sekunde aufgezeichneten Aufdampfraten am Schwingquarz wurden je nach Beschichtungszeit im Blendenausschnitt aufaddiert. Über einen Umrechnungsfaktor, der die Dichte des aufgedampften Materials berücksichtigt, lassen sich Schichtdicke bzw. Massenbelegung der jeweiligen Schichten berechnen.

Nach abgeschlossener Beschichtung wurde die Vakuumkammer belüftet, die metallisierte PET-Folie entnommen und die Beschichtung von der PET-Folie in einer separaten Ablöseeinheit mit Aceton abgelöst.

Hierbei wurde durch Auflösen der Releasecoatschicht die Beschichtung von der Folie abgetrennt. Die abgetrennten Schichtpakete wurden abfiltriert, der entstandene Filterkuchen wurde mit Aceton vom Releasecoat vollständig gewaschen, in eine Pigmentsuspension überführt und auf die gewünschte Partikelgröße zerkleinert.

Die Versuchsparameter und die mittels des Schwingquarzes berechneten Schichtdicken der einzelnen Schichten sind in der Tabelle 1 angegeben.

**Erfindungsgemäße Beispiele 5 und 6:**

Die erfindungsgemäßen Beispiele 5 und 6 wurden nach dem gleichen Verfahren hergestellt. Im Unterschied zu den erfindungsgemäßen Beispielen 1-4 wurde jedoch für die Schicht B statt Silber Aluminium verwendet. Weiterhin wurde auf eine Sauerstoffquelle verzichtet, was zu einem geringeren Oxidanteil in den Schichten A und C führte. Bei diesen Beispielen wurde in einem geringeren Maße in die außenliegenden Cr-Schichten Sauerstoff eingebaut, was durch den in der Drehtelleranlage gegebenen Abstand von Substrat 2 (s. Abbildung 3) zur Verdampfungsquelle 1 (45 cm) begünstigt wurde.

**Tabelle 1: Versuchs parameter für die Beispiele 1 bis 6 (Drehtelleranlage) widerstandsbeheiztes Verdampfungsverfahren**

| Probe | Schichtenfolge / aufgedampfte Stoffe | KV [$1*10^{-4}$ mbar] | Massenbelegung [$\mu g*cm^{-2}$] | Schichtdicke über den Schwingquarz berechnet [nm] |
|---|---|---|---|---|
| Beispiel 1 | A: Oxidhaltiges Cr | 3,44 | 9 | 23 |
| | B: Ag | 1,32 | 53 | 51 |
| | C: Oxidhaltiges Cr | 3,84 | 9 | 23 |
| Beispiel 2 | A: Oxidhaltiges Cr | 2,84 | 21 | 41 |
| | B: Ag | 0,99 | 54 | 51 |
| | C: Oxidhaltiges Cr | 3,12 | 21 | 41 |
| Beispiel 3 | A: Oxidhaltiges Cr | 3,17 | 13 | 32 |
| | B: Ag | 1,2 | 53 | 50 |

(fortgesetzt)

| Probe | Schichtenfolge / aufgedampfte Stoffe | KV [$1*10^{-4}$ mbar] | Massenbelegung [$\mu$g*cm$^{-2}$] | Schichtdicke über den Schwingquarz berechnet [nm] |
|---|---|---|---|---|
| | C: Oxidhaltiges Cr | 2,61 | 13 | 32 |
| Beispiel 4 | A: Oxidhaltiges Cr | 3,63 | 15 | 38 |
| | B. Ag | 1,2 | 53 | 51 |
| | C. Oxidhaltiges Cr | 2,39 | 15 | 38 |
| Beispiel 5 | A: Oxidhaltiges Cr | 1,69 | 9 | 24 |
| | B: Al | 0,82 | 16,6 | 61 |
| | C: Oxidhaltiges Cr | 0,97 | 9 | 24 |
| Beispiel 6 | A: Oxidhaltiges Cr | 1,21 | 20 | 40 |
| | B: Al | 0,75 | 13 | 49 |
| | C: Oxidhaltiges Cr | 0,86 | 20 | 40 |
| KV: Kammervakuum | | | | |

**Beispiele 7 bis 9 und 20 bis 21** (mehrstufiges Bandverfahren):

Beispiele 7-9 widerstandsbeheiztes Verdampfungsverfahren

Beispiele 20-21 Verdampfung mittels Elektronenstrahlverdampfung

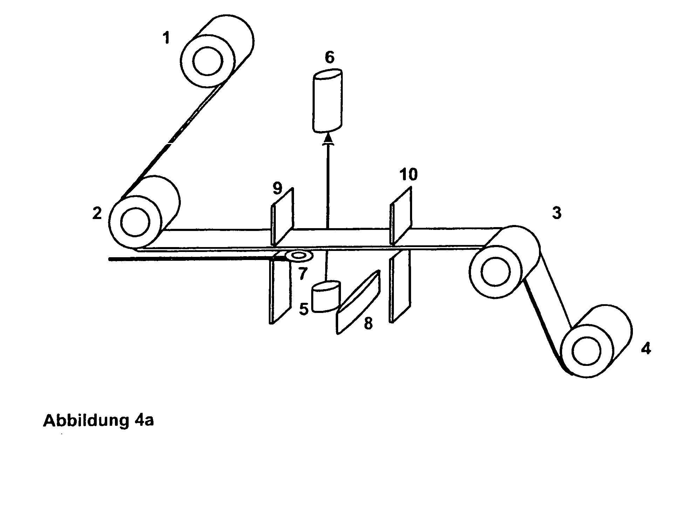

Die erfindungsgemäßen Beispiele 7 und 8 wurden ebenfalls in drei Beschichtungsschritten, jedoch mit einer PVD-Bandanlage erstellt, wie sie in Abbildung 4a schematisch dargestellt ist. Es wurde in drei Durchgängen nacheinander mit einer ersten Schicht A aus oxidhaltigem Cr, mit einer zweiten Schicht B aus Aluminium (Beispiel 7) oder Silber (Beispiel 8) und abschließend mit einer dritten Schicht C aus oxidhaltigem Cr beschichtet. Nach den Beschichtungen A bzw. B wurde jeweils das beschichtete Band zurückgespult, um die gewünschte Beschichtungssequenz zu erhalten.

Auf gleiche Weise wurde das Vergleichsbeispiel 9 durchgeführt. Im Unterschied zu den erfindungsgemäßen Beispielen 7 und 8 wurde als zu verdampfendes Metall für die Schichten A, B und C jeweils Aluminium verwendet.

Auf gleiche Weise wurde das Vergleichsbeispiel 20 durchgeführt. Im Unterschied zu den Beschichtungen der erfindungsgemäßen Beispielen 7 und 8 wurde als zu verdampfendes Metall für die Schichten A und C eine Eisen-Chrom-Legierung mittels Elektronenstrahlverdampfer erzeugt. Der Sauerstoffeinbau in die Schichten A und C erfolgte mit einem Gasflussregler (Mass Flow Controller) der Firma MKS gemäß Abbildung 15. Für die Erzeugung der Schicht B wurde Aluminium mittels Elektronenstrahlverdampfung verdampft.

Die Beschichtung für Beispiel 21 erfolgte in gleicher Weise wie Beispiel 20. Für die beiden äußeren Schichten wurde jedoch Titan mittels Elektronenstrahlverdampfung verdampft.

Als Beschichtungssubstrat wurde eine 30 cm breite Polyethylenterephthalat-(PET)-Folie von 23 $\mu$m Dicke, welche mit einem Releasecoat beschichtet war, verwendet.

Der Releasecoat bestand aus acetonlöslichem Methylmethacrylatharz und wurde in einem separaten Arbeitsschritt vorab aufgebracht.

Das Vakuum wurde, wie oben ausgeführt, ebenfalls mit Hilfe zweier Vorpumpen und einer Diffusionspumpe erzeugt. Die Verfahrensparameter sind in Tabelle 2 bzw. Tabelle 3 aufgeführt.

Die Folie wurde mit den Bandgeschwindigkeiten, die in den Tabellen 2 bzw. 3 angegeben sind, bewegt. Auf die bewegte Folie wurden die Schichten A, B und C der angegebenen Metalle durch Aufdampfen abgeschieden. Das Verdampfen der Metalle erfolgte unter Verwendung von Widerstandsbeheizung für die Beispiele 7-9 und mittels Elektronenstrahlverfahren (Elektronenstrahlverdampfer der Fa. Telemark Modell 272) für die Beispiele 20-21. Die Massenbelegung wurde mittels einer Schwingquarzmessung, wie in Abbildung 4a veranschaulicht (Modell FTM7, Fa. Edwards), kontrolliert und die Schichtdicke über die Heizleistung und Bandgeschwindigkeit gesteuert.

Während der oxidischen Metallbeschichtungen A und C der erfindungsgemäßen Beispiele diente als Sauerstoffquelle mit Wasser getränkte Kartonstücke, die vor dem Evakuieren in die Vakuumkammer gelegt wurden. Der Sauerstoffpartialdruck für die jeweiligen Schichten wurde über das konstant eingestellte Kammervakuum (s. Tabelle 2) kontrolliert.

Die Positionierung (gemäß Abbildung 15) der geregelten Sauerstoffzudosierung für die Schichten der Beispiele 20, 21 wurde mit einem Mass-Flow-Controller der Fa. MKS bewerkstelligt (s. Tabelle 3).

Bei Aufbringung der Schicht B aus Al (Beispiel 7, 9, 20 und 21) bzw. der Schicht B aus Ag (Beispiel 8) wurde in gleicher Weise verfahren. Im Unterschied zu den Beschichtungen A und C wurde aber auf die Zufuhr von Sauerstoff verzichtet.

Die genauen Parameter und die mittels des Schwingquarzes berechneten Schichtdicken und oder die daraus resultierenden Massenbelegungen der einzelnen Schichten A, B und C der erfindungsgemäßen Pigmente sind der Tabelle 2 bzw. Tabelle 3 zu entnehmen.

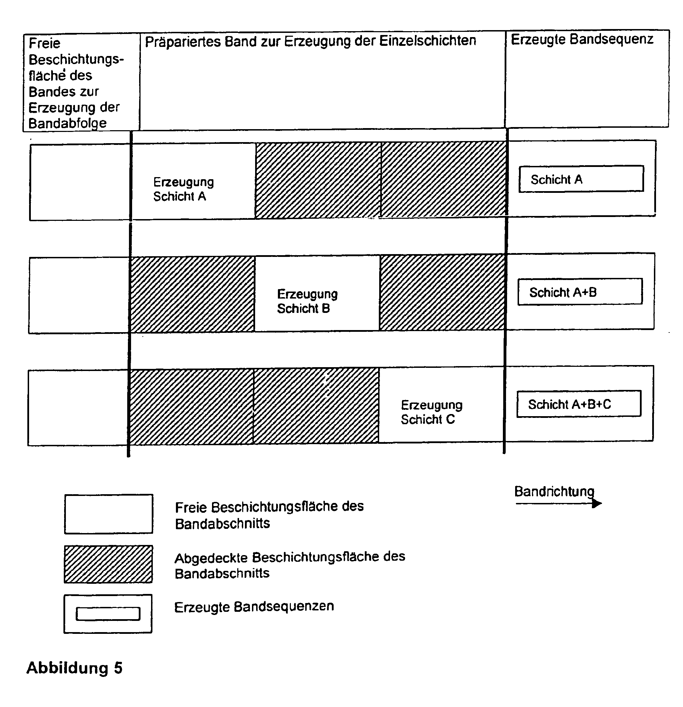

Um zusätzlich den Sauerstoffgehalt der einzelnen Schichten A, B, C und der kompletten Schichtabfolge A-B-C besser bestimmen zu können, wurde beim Beispiel 7, 20 und 21 die zu beschichtende Folie, wie in Abbildung 5 schematisch dargestellt, durch geeignet platzierte Blenden während der Beschichtungsvorgänge präpariert. Hierdurch wurden bestimmte Folienabschnitte ausschließlich mit nur jeweils einer Schicht A, B oder C beschichtet und ließen sich später separat analysieren. In dem Abschnitt ohne Blenden wurden alle drei Schichten abgeschieden.

Nach abgeschlossener Beschichtung wurde die Vakuumkammer belüftet, die metallisierte PET-Folie entnommen und die Beschichtung von der PET-Folie in einer separaten Ablöseeinheit mit Aceton abgelöst.

Hierbei wurde durch Auflösen der Releasecoatschicht die Beschichtung von der Folie abgetrennt. Die abgetrennten Schichtpakete wurden abfiltriert, der entstandene Filterkuchen wurde mit Aceton vom Releasecoat vollständig gewaschen, in eine Pigmentsuspension überführt und auf die gewünschte Partikelgröße zerkleinert.

**Beispiele 10, 11 und 13:** (einstufiges Bandverfahren):

Beispiele 10, 11 und 13 widerstandsbeheiztes Verdampfungsverfahren

Beispiele 22, 23 und 24 Verdampfung mittels Elektronenstrahlverdampfung

Zur Herstellung der erfindungsgemäßen farbigen metallischen Effektpigmente der erfindungsgemäßen Beispiele 10, 11, 13 und 22, 23, 24 wurde ein erfindungsgemäßes PVD-Bandverfahren verwendet, bei dem in nur einem Durchgang ein dreischichtiges Metallpigment erzeugt wurde. Hierbei wurde die gesamte Vakuumkammer jeweils mit folgenden Sauerstoffquellen ausgestattet: für Beispiel 10 wurde durch eine konstante Luftzudosierung in die Vakuumkammer ein konstanter Sauerstoffpartialdruck erzeugt. Bei Beispiel 11 wurden feuchte Kartons links und rechts des Blendeneingangs bzw. des Blendenausgangs (siehe Abbildung 9) positioniert.

Für die Positionierung bei Beispiel 13 wurden zwei als Sauerstoffquelle getränkte Kartons dicht an den Verdampfungskegel herangeführt. Durch die vom Verdampfer ausgehende Strahlungswärme wurde ein hoher Sublimationsstrom des Wassers aus den beiden wassergetränkten Kartons in den Metallkegel hinein erzeugt.

Die Positionierung (gemäß Abbildung 15) der geregelte Sauerstoffzudosierung für die Beispiele 22, 23, 24 wurde mit einem Mass-Flow-Controller der Firma MKS bewerkstelligt.

In Abbildung 4a ist die Beschichtungsapparatur schematisch dargestellt. Entscheidend ist hierbei die Tatsache, dass sich in Abhängigkeit vom Abstand der Verdampferquelle zum Beschichtungsband auf diesem zwei relativ scharf unterscheidbare Bereiche mit Schichten unterschiedlichen Sauerstoffgehaltes abbilden lassen (s. Abbildung 9 bzw. Abbildung 15).

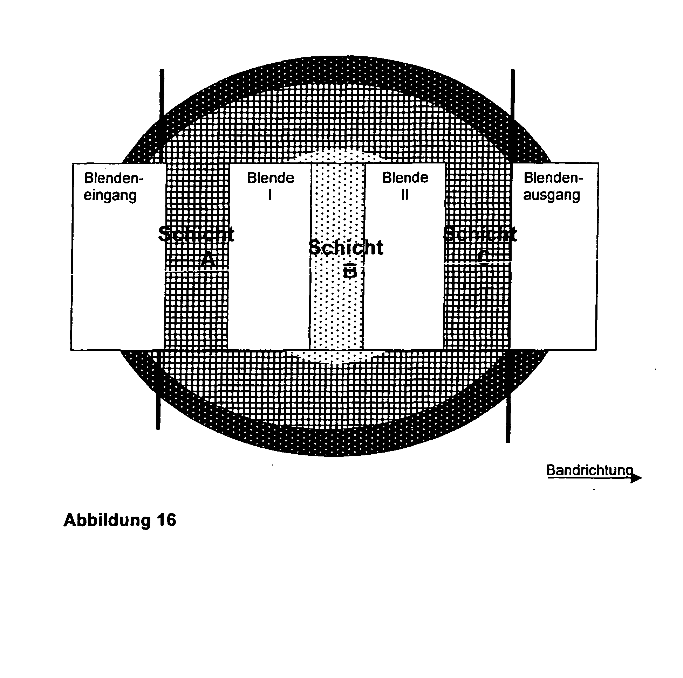

Abbildung 10 bzw. Abbildung 16 zeigt in einer schematischen Draufsicht (aus Sicht der Verdampferquelle) eine Blendenanordnung zwischen Verdampferquelle und Beschichtungsband. Durch Auswahl geeigneter Blenden (Breite, Länge) und der genauen Positionierung der Blenden ließ sich das Beschichtungsband in drei separate Beschichtungszonen unterteilen, bei denen jeweils nur die Schichten A, B und C entstanden.

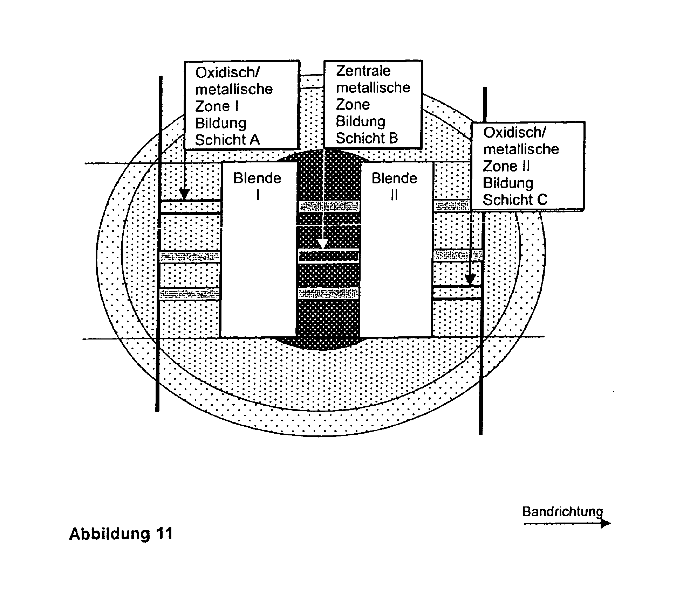

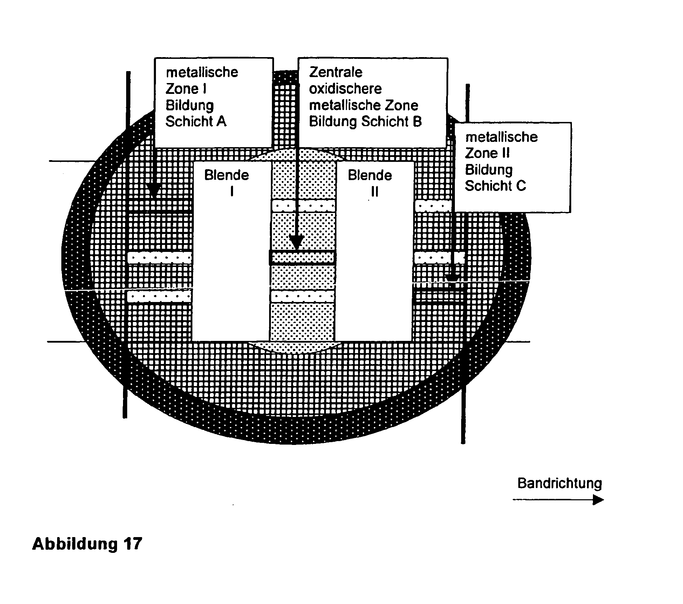

Um eine genaue Bestimmung der Schichtdicken und der Sauerstoffgehalte der einzelnen Schichten A, B und C in ihren jeweiligen Zonen, sowie der kompletten Schichtabfolge A-B-C zu ermöglichen, wurden bei den Beispielen 10, 22, 23, 24 zusätzlich sehr schmale, parallel zur Bandrichtung laufende Blenden (Längsblenden) eingebaut (s. Abbildung 11 bzw. Abbildung 17), wodurch nach dem Durchlauf des Bandsubstrates auf dem Bandsubstrat Bereiche mit den Schichten A, B und C jeweils separat sowie den vollständigen Schichtaufbau A-B-C aufgebracht wurden. Die geometrische Anordnung der Quer- und Längsblenden gemäß den Abbildungen 11 bzw. 17 ist Tabelle 4 zu entnehmen.

Bei Beispiel 11 und 13 wurde im Unterschied zu den Beispielen 10, 22, 23, 24 (siehe Draufsicht schematische Abbildung 10) auf eine Abtrennung durch Blenden in drei Beschichtungsbereiche verzichtet.

Zur Beschichtung wurde eine 30 cm breite Polyethylenterephthalat-(PET)-Folie von 23 μm Dicke, welche mit einem Releasecoat beschichtet ist, verwendet.

Der Releasecoat bestand aus acetonlöslichem Methylmethacrylatharz und wurde in einem separaten Arbeitsschritt vorab aufgebracht.

Das Vakuum wurde jeweils mit Hilfe zweier Vorpumpen und einer Diffusionspumpe erzeugt, wie vorstehend beschrieben.

Für die Beispiele 10, 11 und 13 wurde eine einzige Verdampferquelle aus widerstandsbeheiztem Aluminium verwendet. Die Schichtdicke der mittigen Al-Schicht A wurde über die von außen in die Vakuumkammer geführte Al-Drahtmenge, die Größe des mittigen Blendenausschnitts, die Heizleistung der verwendeten Verdampferkeramik und der Bandgeschwindigkeit gesteuert.

Für die Beispiele 22, 23, 24 wurde mittels eines Elektronenstrahlverdampfers der Fa. Telemark Modell 272 gemäß den Verfahrensparametern der Tabelle 3 Cr (Beispiel 22), Ag (Beispiel 23) und Cu (Beispiel 24) verdampft. Die Schichtdicke der mittigen Schichten B wurde über die jeweiligen Emissionsströme bei konstanter Beschleunigungsspannung, die Größen der jeweils für die Beispiele 22, 23 und 24 mittigen Blendenausschnitte und der jeweiligen Bandgeschwindigkeiten gesteuert.

Die Schichtstärke für die Beispiele 10, 11, 13 und 22, 23, 24 und der Oxidgehalt der Schichten A und C wurde durch die Blendenanordnung sowie die zugeführte Sauerstoffmenge kontrolliert. Die Versuchsparameter sind in Tabelle 2 bzw. Tabelle 3 aufgeführt.

Nach abgeschlossener Beschichtung wurde die Vakuumkammer belüftet und die metallisierte PET-Folie entnommen.

Die beschichteten Folien der Beispiele 10, 22, 23 und 24 zeigten jeweils vier Längsstreifen der Schichten A, B und C, die sich farblich deutlich von der kompletten Schichtabfolge A-B-C abhoben. Die Folienabschnitte der Beschichtungen A, B und C sowie die komplette Schichtabfolge A-B-C wurden voneinander abgeschnitten und von der PET-Folie jeweils in separaten Ablöseeinheiten mit Aceton abgelöst.

Die Pigmente gemäß den Beispielen 11 und 13 wurden gemäß der schematischen Darstellung des Verfahrens in Abbildung 9 und der vorstehend beschriebenen Vorgehensweise nach den Verfahrensparametern in Tabelle 2 hergestellt, jedoch ohne die zwei schmalen zusätzlichen Blenden I und II und ohne die Längsblenden.

### Analytische Sauerstoffbestimmung

EDX-Messungen:

Von den Pigmenten gemäß den Beispielen 7, 10, und 20-24 wurden die Zusammensetzung von Sauerstoff und Metall mit der oben beschriebenen Meßmethodik mittels EDX (Gerät: EDAX Gemini; Fa. EDAX Incorp., USA) an den einzelnen Schichten ermittelt.

Probenvorbereitung:

Einige Tropfen der Pigmentdispersionen wurden auf den Probenteller aufgegeben und das Lösemittel bei Raumtemperatur langsam verdampft. Dabei orientieren sich die Pigmente weitgehend parallel zur Telleroberfläche.

Mit den Pigmenten des Beispiels 10 wurde ein Tiefenprofil des erfindungsgemäßen Effektpigments (s. Abbildung 6) erstellt. Außerdem wurden von Einzeischichten des erfindungsgemäßen Effektpigments aus Beispiel 10 das atomare Verhältnis von Sauerstoff zu Metall bestimmt (s. Tabelle 5).

Die Anregungsenergie des Elektronenstrahls bestimmte die Eindringtiefe in das Pigment (vgl. Abbildung 2). Es wurden jeweils für Sauerstoff die $K_\alpha$-Linie (Anregungsenergie: ca. 0,5 keV), für Chrom die $L_\alpha$-Linie (Anregungsenergie: ca. 0,6 keV) und für Aluminium die $K_\alpha$-Linie (Anregungsenergie: ca. 1,5 keV) angeregt.

Im Einzelnen wurde folgendermaßen vorgegangen:

Durch eine schrittweise Erhöhung der eingestrahlen Elektronenenergie wurden die Eindringtiefe des Elektronenstrahls in der Probe fortlaufend erhöht. Gleichzeitig wurde mittels der EDX-Analyse untersucht, welche Elemente angeregt wurden. Auf diese Weise konnte exakt festgestellt werden, ab welcher Schichtdicke (bzw. Eindringtiefe) die Elemente der jeweils 2. bzw. 3 Schicht nachweisbar sind. Zur Analyse wurden dann nur diejenigen Spektren herangezogen, die die Signale der ersten Schicht, der zweiten Schicht sowie schließlich der dritten Schicht repräsentieren. Bei der dritten Schicht jedoch wurden wieder Signale der ersten Schicht erhalten, die von darunter liegenden Pigmenten stammten. Die Signale dieser Schicht konnte daher nicht mehr völlig aufgelöst werden.

Bei den chromhaltigen Proben wurden zur Analyse die teilweise überlappenden Peaks (Cr- und O-Peaks) mittels des geräteeigenen Softwareprogramms (Version 3.60) durch eine holographische Peak Dekonvolutionsanalyse (HDP) ausgewertet.

ESCA/XPS-Messungen:

Weiterhin wurden die Pigmente des Beispiels 8 und des Beispiels 9 mittels ESCA hinsichtlich ihrer Elementzusammensetzung und ihres Metall- und Sauerstoffgehaltes untersucht. Zur Probenpräparation wurden die Pigmente in Aceton aufgenommen und auf einem Glasträger eingetrocknet. Auf diese Weise wird eine weitgehende planparallele Orientierung der Pigmente erreicht. Die Messungen wurden mit dem Gerät ESCALAB 250 der Fa. Thermo VG Scientific durchgeführt. Für die Anregung wurde monochromatische Al $K_\alpha$. Röntgenstrahlung verwendet (15 kV, 150 W, 500 $\mu$m Spotgröße). Die Transmissionsfunktion des Gerätes wurde an einer Kupferprobe gemessen. Zur Ladungskompensation kam eine "Flood-Gun" mit einer Elektronenenergie von 6 eV/0,05 mA Strahlstrom zum Einsatz.

Es wurde zunächst ein Übersichtsspektrum (Pass-Energie: 80 eV) aufgenommen. Anschließend wurden Hochauflösungsspektren mit einer Pass-Energie von 30 eV gemessen. Die Proben wurden mit Ar-Ionen abgesputtert und anschließend ein Hochauflösungsspektrum mit dem. ESCALAB 250 gemessen. Auf diese Weise wurden Tiefenprofile der Zusammensetzung bestimmt.

Die mittleren Schichtdicken der Proben wurden durch Auszählung einer signifikanten Stichprobe (> 20 Teilchen) unter einem Raster-Elektronen-Mikroskop (REM) ermittelt. Hierbei wurden die azimuthalen Winkel der einzelnen Pigmentteilchen relativ zur Beobachterebene geschätzt und in die Berechnung mit einbezogen. Es wurden nur Teilchen vermessen, die weitgehend senkrecht zur Beobachterebene standen.

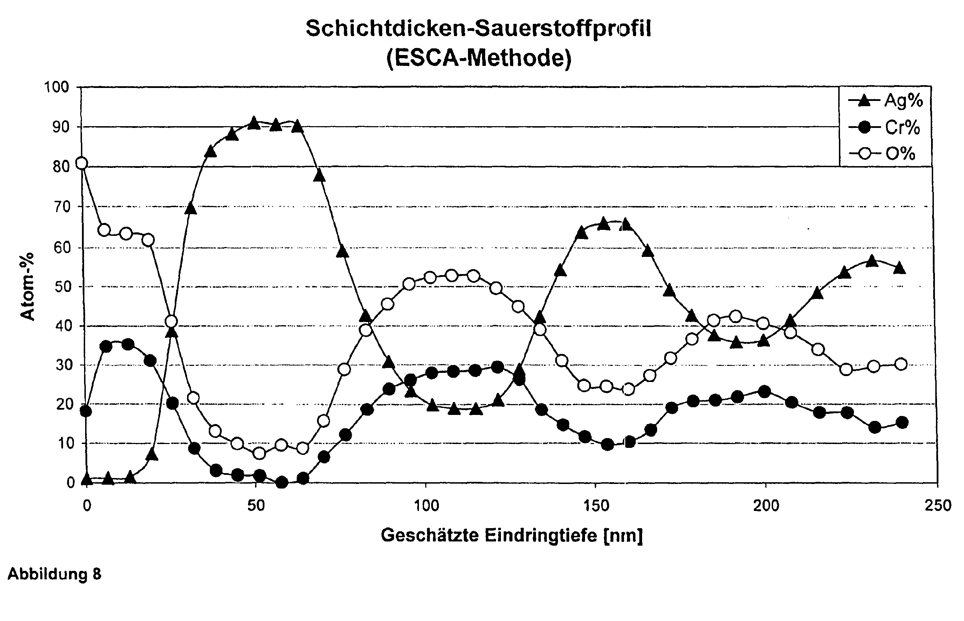

In Abbildung 8 sind die Konzentrationen der Elemente Silber, Chrom und Sauerstoff als Funktion der Sputtertiefe dargestellt. Die Sputtertiefe kann ist nur annähernd wiedergegeben bzw. grob geschätzt werden, da eine absolut genaue Eichung nicht möglich war. Daher stimmt diese Tiefenskala nicht unbedingt mit den aus REM-Messungen ermittelten Schichtdicken überein. Deutlich erkennt man zunächst eine Cr-O-Schicht. Bei ca. 50 nm Sputtertiefe ist das Chromsignal praktisch auf den Nullpunkt gesunken und korrespondierend dazu das Silbersignal von fast Null bis auf über 90 Atom-% angestiegen (Schicht B). Anschließend (bei ca. 100 nm Sputtertiefe) sinkt das Silbersignal wieder ab und das Chromsowie das Sauerstoffsignal steigen wieder an, wobei jedoch nicht die Werte wie am Anfang erreicht werden; insbesondere sinkt das Silbersignal nicht so stark ab.

Es wird vermutet, dass Schwankungen bei der Meßmethode, dem Sputtern und/oder die statistischen Verteilungen der Schichtdicken der Pigmente zu einer "Verschmierung" der Signale führen. Die Anfangswerte stellen jedoch verläßliche Angaben zur mittleren Zusammensetzung der Schichten A und B dar.

Der nachfolgende Anstieg des Silbersignals, gefolgt von einem Anstieg des Chromsignals ist darauf zurückzuführen, dass bereits die nächste Lage an Pigmenten vermessen und die erste Pigmentlage bereits weitgehend weggesputtert wurde. Insgesamt nivellieren sich mit zunehmender Anzahl die gemessenen Elementkonzentrationen naturgemäß immer mehr.

Der Schichtaufbau der Pigmente kann ferner durch REM-Querschliffe gezielt analysiert werden.

In Abbildung 7 ist der mittels ESCA/XPS-Messungen ermittelte Schichtaufbau des Pigmentes gemäß dem erfindungsgemäßen Beispiel 9 dargestellt. In den äußeren Schichten liegt im Mittel eine Aluminiumkonzentration von 53 Atom-% und eine korrespondierende Sauerstoffkonzentration von 47 Atom-% vor. Nach ca. 50 nm Sputtertiefe beginnt das Aluminiumsignal zu wachsen und das Sauerstoffsignal dementsprechend zu sinken. Im Maximum erreicht die Al-Konzentration 71 Atom-%.

**Beispiel 12:** (Verfahren mit drei Verdampfungsquellen)

Es wurde die gleiche Versuchsapparatur wie bei den erfindungsgemäßen Beispielen 10 und 11 benutzt. Zur Herstellung der Schicht B wurde Aluminium mit kontinuierlicher Drahtzufuhr widerstandsbeheizt verdampft. Zusätzlich wurden jedoch dicht neben der Verdampferkeramik zwei Molybdänschiffe zur Verdampfung von Cr angebracht (Abbildung 4b). Als Sauerstoffquelle wurden angefeuchtete Kartons in die Vakuumkammer gelegt.

Die Schichtdicke der mittigen Al-Schicht A wurde über die von außen in die Vakuumkammer geführte Al-Drahtmenge, die Größe des mittigen Blendenausschnitts, die Heizleistung der verwendeten Verdampferkeramik und der Bandgeschwindigkeit gesteuert.

Die Schichtstärke und der Oxidgehalt der Schichten A und C aus Cr wurde durch die Blendenanordnung und den variabel einstellbaren Widerstand der Molybdänschiffe, sowie der zugeführten Sauerstoffmenge kontrolliert. Nach abgeschlossener Beschichtung wurde die Vakumkammer belüftet, die metallisierte PET-Folie entnommen und wie in den anderen Beispielen beschrieben weiterverarbeitet.

Man erhält hellgold glänzende Metallpigmente mit einem außerordentlich starken Hell-Dunkel-Flop.

**Vergleichsbeispiel 14:** Reichgold 62926/G; kommerziell erhältliches

Messingpigment (Hersteller: Fa. ECKART GmbH).

**Vergleichsbeispiel 15:** Variocrom Magic Gold (Fa. BASF)

Kommerziell erhältliches fünfschichtiges Interferenzeffektpigment mit einem Kern aus Aluminium, einer beidseitigen $SiO_2$-Schich und einer semitransparenten Eisenoxidschicht. Die mittlere Teilchengröße beträgt 17 $\mu$m.

**Vergleichsbeispiel 16:** Kommerziell erhältliches Blattgold.

Bestes Rosenobel Doppelgold 23 ¾ Karat; Firma Noris Blattgold).

**Vergleichsbeispiel 17**: Auf einen Objektträger wurde Gold in einer Schichtdicke von ca. 80 nm aufgesputtert.

Schließlich wurden nach einheitlichen Zerkleinerungsverfahren mittels üblicher Laserbeugungsmethoden (Gerät Cilas 1064) die Größenverteilung der Pigmente der Beispiele 1-8, 10-13, 23, 24 und der Vergleichsbeispiele bestimmt und in üblicher Weise aus der Summendurchgangsverteilung der $D_{50}$-Wert als Maß für die mittlere Größe ermittelt (s. Tabelle 5).

**Tabelle 2**: Verfahrensparameter und berechnete Schichtdicken für Beispiele 7 bis 13 (Bandbeschichtungsanlage widerstandsbeheiztes Verdampfungsverfahren)

| Probe | Schichtenfolge/ aufgedampfte Stoffe | Kammervakuum [$1*10^{-4}$ mbar] | Massenbelegung [$\mu$g*cm$^{-2}$] | Schichtdicke über den Schwingquarz berechnet [nm] | Bandgeschwindigkeit [m*min$^{-1}$] |
|---|---|---|---|---|---|
| Beispiel 7 | A: Oxidhaltiges Cr | 6,5 | 10,5 | 28 | 0,8 |
| | B: Al | 0,9 | 11,0 | 41 | 0,8 |
| | C: Oxidhaltiges Cr | 5,0 | 10,5 | 28 | 0,8 |
| Beispiel 8 | A: Oxidhaltiges Cr | 4,0 | 9,8 | 25 | 0,64 |
| | B: Ag | 1,0 | 113 | 110 | 3 |
| | C: Oxidhaltiges Cr | 3,5 | 9,8 | 25 | 0,64 |
| Beispiel 9 | A: Oxidhaltiges Al | 2,0 | 19,6 | 73 | 0,64 |
| | B: Al | 1,43 | 17,3 | 64 | 0,64 |
| | C: Oxidhaltiges Al | 1,8 | 23 | 86 | 0,64 |
| Beispiel 10 | A: Oxidhaltiges Al | | | | |
| | B: Al | 3,0 | | | 1,2 |
| | C: Oxidhaltiges Al | | | | |
| Beispiel 11 | A: Oxidhaltiges Al | | | | |
| | B: Al | 25 | | | 20 |
| | C: Oxidhaltiges Al | | | | |
| Beispiel 12 | A: Oxidhaltiges Cr | | | | |
| | B: Al | 13 | | | 8,0 |
| | C: Oxidhaltiges Cr | | | | |

(fortgesetzt)

| Probe | Schichtenfolge/ aufgedampfte Stoffe | Kammervakuum [$1*10^{-4}$ mbar] | Massenbelegung [$\mu$g*cm$^{-2}$] | Schichtdicke über den Schwingquarz berechnet [nm] | Bandgeschwindigkeit [m*min$^{-1}$] |
|---|---|---|---|---|---|
| Beispiel 13 | A: Oxidhaltiges Al | | | | |
| | | | | | |
| | B: Al | 500 | 68 | | 20 |
| | C: Oxidhaltiges Al | | | | |

**Tabelle 3**: Verfahrensparameter für Beispiele 20 bis 24 (Bandbeschichtungsanlage -Verdampfungsverfahren mit Elektronenstrahlverdampfung)

| Probe | Schichtenfolge / aufgedampfte Stoffe | Kammervakuum [$1 \times 10^{-4}$ mbar] | Massenbelegung [$\mu g \cdot cm^{-2}$] | Gasfluss [$sl \cdot min^{-1}$] | Emissionsstrom [mA] bei 10 kV | Bandgeschwindigkeit [$m \cdot min^{-1}$] |
|---|---|---|---|---|---|---|
| Beispiel 20 | A: Oxidhaltiges Fe/Cr | 5,5 | 15,3 | 1,1 | 460 | 22 |
|  | B: Al | 1,2 | 10,8 | 0 | 400 | 22 |
|  | C: Oxidhaltiges Fe/Cr | 3,7 | 15,3 | 1,1 | 480 | 22 |
| Beispiel 21 | A: Oxidhaltiges Ti | 2 | 15,4 | 0,1 | 440 | 3,2 |
|  | B: Al | 0,9 | 16,2 | 0 | 275 | 3,2 |
|  | C: Oxidhaltiges Ti | 1,5 | 15,4 | 0,1 | 520 | 3,2 |
| Beispiel 22 | A: Oxidhaltiges Cr | 1,7 | 55,5 | 0,65 | 450 | 6,5 |
|  | B: Oxidhaltiges Cr |  |  |  |  |  |
|  | C: Oxidhaltiges Cr |  |  |  |  |  |
| Beispiel 23 | A: Oxidhaltiges Ag | 2,2 | 16,3 | 0,3 | 340 | 6,5 |
|  | B: Oxidhaltiges Ag |  |  |  |  |  |
|  | C: Oxidhaltiges Ag |  |  |  |  |  |
| Beispiel 24 | A: Oxidhaltiges Cu | 10 | 21,9 | 0,7 | 350 | 4 |
|  | B: Oxidhaltiges Cu |  |  |  |  |  |
|  | C: Oxidhaltiges Cu |  |  |  |  |  |

**Tabelle 4**: geometrische Maße der Draufsicht der Abbildungen 11 bzw. 17 bei einem Abstand der Verdampfungsquelle zum Substrat von 40 cm.

| | Längsblende für Zone I | Querblende I | Längsblende für zentrale Zone | Querblende II | Längsblende für Zone II |
|---|---|---|---|---|---|
| Beispiel 10 | Offen 19 cm | Zu 15 cm | Zu 8cm | Zu 10 cm | Zu 10 cm |
| | Zu 19 cm | Zu 15 cm | Offen 8 cm | Zu 10 cm | Zu 10 cm |
| | Zu 19 cm | Zu 15 cm | Zu 8cm | Zu 10 cm | Offen 10 cm |
| Komplette Schichtabfolge Beispiel 10 | Offen 19 cm | Zu 15 cm | Offen 8 cm | Zu 10 cm | Offen 10 cm |
| Beispiel 22 | Offen 5 cm | Zu 7,5 cm | Zu 15 cm | Zu 7,5 cm | Zu 5 cm |
| | Zu 5 cm | Zu 7,5 cm | Offen 15 cm | Zu 7,5 cm | Zu 5 cm |
| | Zu 5cm | Zu 7,5 cm | Zu 15 cm | Zu 7,5 cm | Offen 5cm |
| Komplette Schichtabfolge Beispiel 22 | Offen 5cm | Zu 7,5cm | Offen 15 cm | Zu 7,5 cm | Offen 5 cm |
| Beispiel 23 | Offen 9 cm | Zu 7,5 cm | Zu 7,5 cm | Zu 7,5 cm | Zu 9 cm |
| | Zu 9 cm | Zu 7,5 cm | Offen 7,5 cm | Zu 7,5 cm | Zu 9 cm |
| | Zu 9cm | Zu 7,5 cm | Zu 7,5 cm | Zu 7,5 cm | Offen 9cm |
| Komplette Schichtabfolge Beispiel 23 | Offen 9cm | Zu 7,5cm | Offen7,5 cm | Zu 7,5 cm | Offen 9 cm |
| Beispiel 24 | Offen 9 cm | Zu 7,5 cm | Zu 7,5 cm | Zu 7,5 cm | Zu 9 cm |
| | Zu 9 cm | Zu 7,5 cm | Offen 7,5 cm | Zu 7,5 cm | Zu 9 cm |
| | Zu 9cm | Zu 7,5 cm | Zu 7,5 cm | Zu 7,5 cm | Offen 9cm |
| Komplette Schichtabfolge Beispiel 24 | Offen 9cm | Zu 7,5cm | Offen7,5 cm | Zu 7,5 cm | Offen 9 cm |

Farbmetrische Eigenschaften anhand von Rakelabzügen:

im Folgenden soll das farbmetrische Verhalten der Pigmente der erfindungsgemäßen Beispielen und der Vergleichsbeispiele dargestellt werden.

Hierzu wurde das jeweilige Pigment in jeweils 2 g eines konventionellen Nitrocelluloselackes (Dr. Renger Erco Bronzemischlack 2615e; Fa. Morton) eingerührt. Dabei wurde das Effektpigment vorgelegt und anschließend mit einem Pinsel in den Lack dispergiert.
Der fertige Lack wurde auf einem Rakelabzugsgerät mit einer Nassfilmdicke von 50 $\mu$m auf Prüfkarten Nr. 2853 der Fa. Byk Gardner (Schwarz-Weiß-Kontrastpapier) appliziert.

Die Pigmentierungshöhe wurde so gewählt, dass sich eine deckende Schicht ergab. Hierdurch geht in die farbmetrischen Daten der Einfluss des Untergrundes nicht ein.

Die Rakelabzüge wurden farbmetrisch gemäß Herstellerangaben vermessen (Gerät Optronic Multiflash, Berlin). Dabei wurde unter einem konstanten Winkel von 45° eingestrahlt und die CIELAB L*-, a*- und b*-Werte bei Beobachtungswinkeln von 15°, 20°, 25°, 45°,55°, 70°, 75° und 110° relativ zum Glanzwinkel bestimmt (Lichtquelle: D65).

In Tabelle 5 sind zusätzlich zu analytischen Daten die farbmetrischen Daten der Rakelabzüge der erfindungsgemäßen Beispiele und der Vergleichsbeispiele eingetragen.

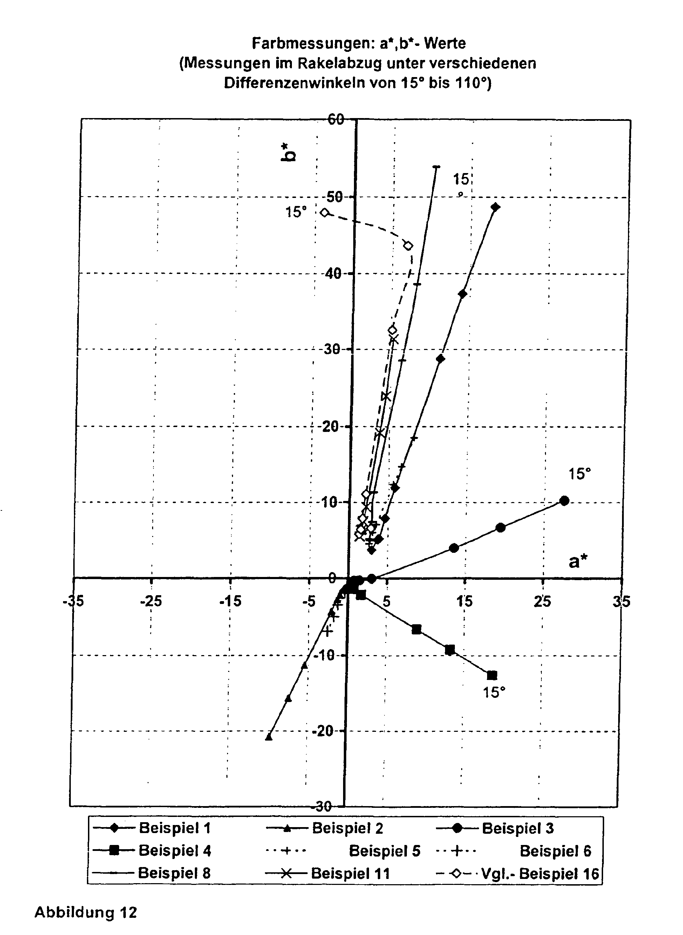

Abbildung 12 zeigt in einer CIELAB a*, b*- Darstellung die farbmetrische Darstellung erfindungsgemäßer Pigmente und Pigmente aus Vergleichsbeispiele. Zusätzlich wurde ein Blattgoldmuster (Vergleichsbeispiel 16, Bestes Rosenobel Doppelgold 23 ¾ Karat; Firma Noris Blattgold) vermessen.
Die Pigmente der Beispiele 1, 8 und 11 zeigen metallisch goldfarbene Effekte. Entsprechend erscheint Beispiel 2 blau, Beispiel 3 rot und Beispiel 4 violett mit Farbflopeffekte.

Die Proben der erfindungsgemäßen Beispiele 5 und 6 zeigen ein dezenteres Chroma als jene der erfindungsgemäßen Beispiele 1 und 2.

Das Vergleichsbeispiel 16 (Blattgold) zeigt ebenfalls ein starkes Chroma, jedoch einen leichten Farbflop (Messwinkel 15° nach 20°) und daher mithin eine geringere Farbtonreinheit als die erfindungsgemäßen Pigmente.

Das Pigment gemäß Beispiel 11, das sich durch Beschichtung in einem Bedampfungsschritt hergestellt wurde, zeigt in seinem Verlauf ein Anschmiegen an den Verlauf des Blattgoldmusters im gelben Bereich (Vergleichsbeispiel 16), erreicht jedoch nicht das Chroma des Blattgoldes. Beispiel 8 zeigt ebenfalls ein Anschmiegen des Kurvenverlaufs an das Blattgoldmuster und zeigt darüber hinaus ein deutlich stärkeres Chroma als das Blattgoldmuster. Die Pigmente der Beispiele 5 und 6 weisen ein dezenteres Chroma auf.

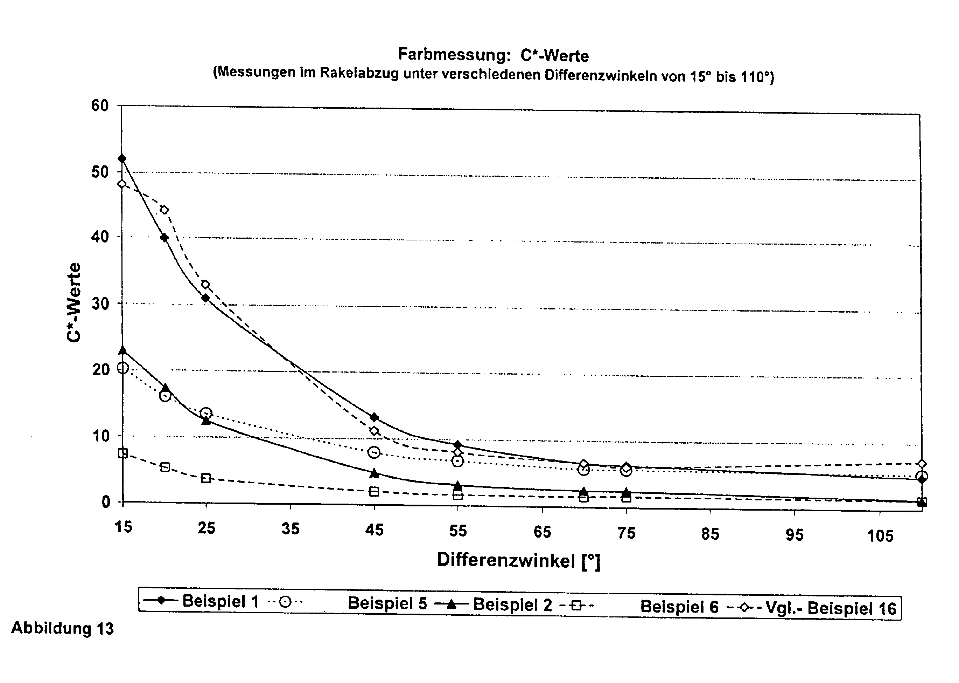

In Abbildung 13 ist das Chroma verschiedener goldfarbener (Beispiel 1, Beispiel 5) und blauer (Beispiel 2 und Beispiel 6) metallischer Effektpigmente gegen den Glanzwinkel dargestellt. Diese Darstellung zeigt, dass das starke Farbchroma von Blattgold auch durch das erfindungsgemäße Pigment des Beispiels 1 erreicht wird. Die Beispiele 5 und 6 (Cr/Al/Cr) weisen ein dezenteres Chroma auch nahe dem Glanzwinkel (15°) von höchstens 20 Einheiten auf.

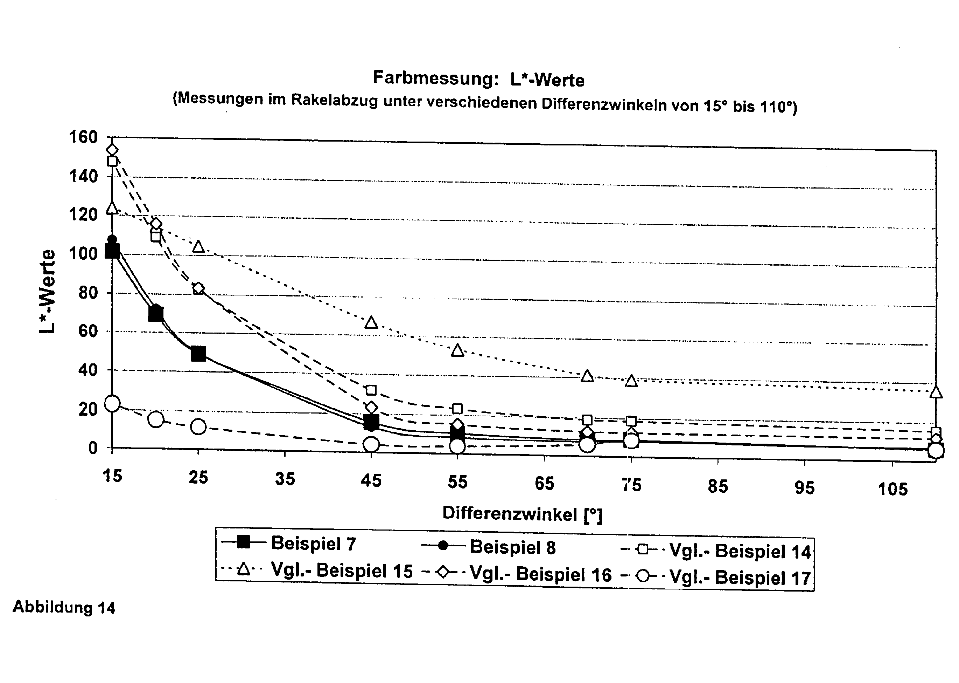

In Abbildung 14 sind die Helligkeitswerte über verschiedene Beobachtungswinkel verschiedener goldfarbiger Beispiele und Vergleichsbeispiele graphisch dargestellt. Hierbei wurden die erfindungsgemäßen Beispiele 7 und 8 sowie im Stand der Technik bekannte Proben wie Messingpigmente (Vergleichsbeispiel 14), Variochrom (Vergleichsbeispiel 15) und das Blattgoldmuster (Vergleichsbeispiel 16) verwendet. Zudem wurde ein Objektträger in einem Sputterverfahren mit Gold bedampft und dadurch verspiegelt (Vergleichsbeispiel 17) und dieser vermessen.

Die Helligkeitskurven der Beispiele 7 und 8 zeigen einen extrem starken Abfall zu niedrigen Werten bei höheren Betrachtungswinkeln. Dieser Effekt ist vor allem auf den hohen Sauerstoffgehalt der äußeren Schichten A und C dieser Pigmente zurückzuführen. Diese Schichten weisen eine dunkle Färbung auf. Die Pigmente der Vergleichsbeispiele 14 bis 16 zeigen bei 15° höhere L*-Werte, erreichen bei höheren Winkeln jedoch nicht die niedrigen Werte der erfindungsgemäßen Beispiele. Das auf den Objektträger aufgesputterte Gold (Vergleichsbeispiel 17) erscheint als Goldspiegel und weist dementsprechend einen außerordentlich hohen Glanz auf. Die gemessene Helligkeit bei 15° dieser Probe ist sehr gering, da in diesem Fall praktisch alles einfallende Licht im Glanzwinkel reflektiert wurde und kaum noch Streustrahlung vorhanden ist. Umso bemerkenswerter erscheinen daher die mit dem Goldspiegel vergleichbar geringen Helligkeiten der erfindungsgemäßen Beispiele bei hohen Messwinkeln.

Der extrem starke Helligkeitswechsel von Hell ins Dunkle der erfindungsgemäßen Pigmente spiegeln auch die sehr hohen Flopwerte (Tabelle 5) wieder.

**Tabelle 5:**

| Probe | | Schichtabfolge des verdampften Metalls | | | Messmethodik | | Pigmentierungshöhe [%] | D 50: [$\mu$m] | Helligkeiten | | | Flop | Subjektiver Farbeindruck |
|---|---|---|---|---|---|---|---|---|---|---|---|---|---|
| | | | | | EDX | ESCA | | | L15 | L45 | L110 | | |
| Beispiel 1 | | Cr | Ag | Cr | | | 8,8 | 15 | 86,8 | 14,2 | 5,9 | 36 | Sehr farbintensiver gold-braun glänzender 50 $\mu$m Rakelabzug |
| | Schichtdicken [nm] | 23 | 50 | 23 | | | | | | | | | |
| Beispiel 2 | | Cr | Ag | Cr | | | 11,3 | ~17 | 74,8 | 8,9 | 3,0 | 47,7 | Blau glänzender ins schwarze abkippender 50 $\mu$m Rakelabzug |
| | Schichtdicken [nm] | 41 | 50 | | | | | | | | | | |
| Beispiel 3 | | Cr | Ag | Cr | | | 9,6 | ~17 | 45,4 | 4,1 | 1,9 | 53,9 | Rot glänzender ins schwarze abkippender 50 $\mu$m Rakelabzug |
| | Schichtdicken [nm] | 32 | 50 | 32 | | | | | | | | | |
| Beispiel 4 | | Cr | Ag | Cr | | | 10,2 | 20 | 44,6 | 3,9 | 1,9 | 53,9 | Violett glänzender ins schwarze abkippender 50 $\mu$m Rakelabzug |
| | Schichtdicken [nm] | 38 | 50 | | | | | | | | | | |
| Beispiel 5 | | Cr | Al | Cr | | | 4,6 | 17 | 92 | 22,7 | | 23 | Braun glänzender 50 $\mu$m Rakelabzug |
| | Sthichtdicken [nm] | 24 | 60 | 24 | | | | | | | | | |
| Beispiel 6 | | Cr | Al | Cr | 40 | | 6,9 | 18 | 69 | 9,15 | 3,8 | 41 | Blau glänzender Rakelabzug 36 $\mu$m Rakelabzug |
| | Schichtdicken [nm] | 40 | 50 | 40 | | | | | | | | | |
| Beispiel 7 | | Cr | Al | Cr | Messung Einzelschichten | | 4,6 | ~17 | 101 | 16 | 6 | 39 | Sehr farbintensiver gold - glänzender 50 $\mu$m Rakelabzug |
| | REM-Schichtdicken [nm] Sauerstoff | 28 | 40 | 28 | | | | | | | | | |
| | O [Atom-%] | 34 | 13 | 34 | | | | | | | | | |

(fortgesetzt)

| Probe | | Schichtabfolge des verdampften Metalls | | | Messmethodik | | Pigmentierungshöhe [%] | D 50: [μm] | Helligkeiten | | | Flop | Subjektiver Farbeindruck |
|---|---|---|---|---|---|---|---|---|---|---|---|---|---|
| | | | | | EDX | ESCA | | | L15 | L45 | L110 | | |
| Bespiel 8 | | Cr | Ag | Cr | | Messung am Pigment | 14,5 | 24 | 107 | 13,5 | 6,5 | 48 | Sehr farbintensiver gold - glänzender 50 μm Rakelabzug |
| | REM-Schichtdicken [nm] | 25 | 110 | 25 | | | | | | | | | |
| | Sauerstoff O (Atom-%] | ~35 | ~10 | ~35 | | | | | | | | | |
| Beispiel 9 | | Al | Al | Al | | Messung am Pigment | | | | | | | blaufarbene Pigmente |
| | Schichtdicken [nm] | 72 | 64 | 86 | | | | | | | | | |
| | Sauerstoff O [Aom-%] Beispiel | 48 | 30 | 48 | | | | | | | | | |
| Beispiel 10 | | Al | Al | Al | Messung Einzeischicht und am Pigment | | 4,3 | 22 | 105 | 21 | 12,1 | | Pigmente mit goldfarbenen Erscheinungsbi |
| | REM-Schichtdicken [nm] | 38 | 100 | 38 | | | | | | | | | |
| | Sauerstoff O [Atom-%] | 50 | 18 | 55 | | | | | | | | | |
| Beispiel 11 | | Al | Al | Al | Messung am Pigment | | 4 | 24 | 103 | 14 | 5,5 | 45 | Gold glänzender ins Dunkle abkippender 50 μm Rakelabzug |
| | REM-Schichtdicken [nm] Sauerstoff | ~50 | 50 | ~50 | | | | | | | | | |
| | O [Atome-%] | 40 | 20 | 40 | | | | | | | | | |
| Beispiel 12 | | Cr | Al | Cr | | | ~ 4 | ~ 17 | 101 | 12 | 5,4 | 51 | Hell-Gold glänzender ins Dunkle glänzender 50 μm Rakelabzug |
| | Schichtdicken [nm] | ~15 | ~40 | ~15 | | | | | | | | | |

(fortgesetzt)

| Probe | | Schichtabfolge des verdampften Metalls | | | Messmethodik | | Pigmentierungshöhe [%] | D 50: [μm] | Helligkeiten | | | Flop | Subjektiver Farbeindruck |
|---|---|---|---|---|---|---|---|---|---|---|---|---|---|
| | | | | | EDX | ESCA | | | L15 | L45 | L110 | | |
| Beispiel 13 | | Al | | | | | 9 | 24 | 49 | 3,84 | 2,11 | 61 | Schwarzer rot/blau flopender 50 μm Rakelabzug |
| | REM-Schichtdicken [nm] | 252 | | | | | | | | | | | |
| Beispiel 20 | | Fe/Cr | Al | Fe/C r | Messung Einzeischichten | | | | | | | | Sehr farbintensiver goldfarbene- magnetische Farbpigmente |
| | REM-Schichtdicken | 40 | 40 | 40 | | | | | | | | | |
| | Sauerstoff [nm] O [Atom%] | 53 | 17 | 52 | | | | | | | | | |
| | | | | | | | | | | | | | |
| Beispiel 21 | | Ti | Al | Ti | Messung Einzeischichten | | | | | | | | Blauglänzende Farbpigmente |
| | REM-Schichtdicken [nm] | 50 | 60 | 50 | | | | | | | | | |
| | Sauerstoff O [Atom-%] | 66 | 17 | 66 | | | | | | | | | |
| | | | | | | | | | | | | | |
| Beispiel 22 | | Cr | Cr | Cr | Messung Einzeischichten | | | | | | | | Glänzend türkisfarbene Farbpigmente |
| | REM-Schichtdicken [nm] | 40 | 70 | 40 | | | | | | | | | |
| | Sauerstoff O [Atom-%] | 44 | 59,6 | 44 | | | | | | | | | |

(fortgesetzt)

| Probe | | Schichtabfolge des verdampften Metalls | | | Messmethodik | | Pigmentierungshöhe [%] | D 50: [$\mu$m] | Helligkeiten | | | Flop | Subjektiver Farbeindruck |
|---|---|---|---|---|---|---|---|---|---|---|---|---|---|
| | | | | | EDX | ESCA | | | L15 | L45 | L110 | | |
| Beispiel 23 | | Ag | Ag | Ag | Messung Einzeischichten | | | 12,2 | | | | | Glänzend hellgoldfarbene Farbpigmente |
| | REM-Schichtdicken [nm] | 20 | 21 | 19 | | | | | | | | | |
| | Sauerstoff O [Atom-%] | 11 | 9 | 11 | | | | | | | | | |
| Beispiel 24 | | Cu | Cu | Cu | Messung Einzeischichten | | | 8,6 | | | | | Glänzend violettkupferfarben Farbpigmente |
| | REMSchichtdicken [nm] | 43 | 43 | 37 | | | | | | | | | |
| | Sauerstoff O [Atom-%] | 23 | 19 | 24 | | | | | | | | | |

## Charakterisierung durch Elektronenbeugtung

An ausgewählten Proben wurden TEM-Aufnahmen (Gerät: Scanning Transmissionselektronenmikroskop, Hersteller: Fa. Jeol (Japan), Typ: 2010) erstellt. Der präparierte Querschnitt der Proben wurde mit einem Elektronenstrahl von 200 KV durchstrahlt und die Strukturbilder sowie die Beugungsbilder mit Hilfe einer CCD-Kamera aufgenommen. In hellen Bildbereichen wird der Elektronenstrahl wenig gestreut, während dunklere Bereiche durch eine starke Wechselwirkung der Elektronen mit der Probe verursacht werden. Es kann sich dabei sowohl um Streuung an dichten und schweren Bereichen als auch um Beugungseffekte an Kristallflächen handeln.

Die Auswertung der Beugungsbilder erlaubt Aussagen über den kristallographischen Aufbau der Probe. Trifft der Elektronenstrahl auf einen Einkristall, so wird er an den Netzebenen des Kristalls gebeugt und erzeugt in der Brennebene des TEM ein diskretes Beugungsspektrum. Besteht der abgebildete Bereich aus vielen kleinen, zufällig orientierten Kristalliten (polykristallines Material), so bilden sich anstelle einzelner Beugungspunkte konzentrischen Kreise, die Debye-Scherrer-Ringe aus. Bei amorphen Proben besteht das Beugungsmuster aus diffusen Ringen. Diese Strukturen sind materialspezifisch und für viele Stoffe in der Literatur dokumentiert.

Folgende Proben wurden zur näheren Charakterisierung der Schicht A oder C der erfindungsgemäßen Pigmente exemplarisch mittels Elektronenbeugung charakterisiert:

**Beispiel 7**: Cr-Al-Cr Erfindungsgemäßes Pigment mit gesamter Schichtabfolge

**Beispiel 18**: Einzelschicht Cr (Schicht A): Bei der Herstellung der Beispiels 7 wurde die erste Schicht A nach nur einmaligem Bedampfen des Release-coats mit Cr in Sauerstoffhaltiger Umgebung isoliert.

**Beispiel 19:** Einzelschicht Al (Schicht A): Bei der Herstellung der Beispiels 10 wurde nur die erste Schicht A (Al in Sauerstoffhaltiger Umgebung) bei dem einstufigen Bedampfungsverfahren durch geschickte Wahl der Blenden (s. Abbildung 11) auf einen Teil des Release-coats isoliert aufgetragen und später getrennt von der Folie gelöst.

Probenpräparation:

Die Proben wurden in ein Epoxidharz eingebunden. Nach Aushärtung wurden mittels Ultramikrotomie ca. 100 nm dicke Scheibchen präpariert und auf TEM-Netzchen aufgebracht.

Auswertung:

Die TEM-Präparate wurden mit einem Elektronenstrahl von 200 kV durchleuchtet. Durch eine Abbildung der Brennebene wurden die Beugungsmuster der mit Elektronenstrahlen durchstrahlten Proben sichtbar gemacht. Hier wurden für die Einzelschicht von Beispiel 18 als Beugungssignale zwei unterscheidbare Ringe detektiert (CCD-Kamera). Bei der Probe von Beispiel 7 wurden scharfe Reflexe sowie Ringe detektiert.
Die Lage der einzelnen Reflexe bzw. Ringe wird mit charakteristischen Beugungskonstanten aus der Literatur verglichen. In Tabelle 6 sind die experimentellen Werte (nur die intensivsten Signale wurden ausgewertet), Literaturdaten sowie die Zuordnungen der experimentellen Daten dargestellt.

**Tabelle 6: Ergebnisse Elektronenbeugungsversuche**

| Probe | d (Å) gemessen | Reflexcharakter | Zuordnung | Literaturwerte | |
|---|---|---|---|---|---|
| | | | | d (Å) | rel. Intensität |
| Beispiel 18 | 2,488 | Ring 1 | $Cr_2O_3$ (Schicht A) | **Cr (Metall):** | |
| | 2,56 | Ring 1 | $Cr_2O_3$ (Schicht A) | 2,039 | 100 |
| | 2,072 | Ring 2 | Cr (Schicht A) | **$Cr_2O_3$:** | |
| | 2,089 | Ring 2 | Cr (Schicht A) | 2,665 | 100 |
| | | | | 2,480 | 93 |
| Beispiel 7 | 2,326 | Reflex 1 | Al (Schicht B) | **Cr (Metall):** | |
| | 2,320 | Reflex 2 | Al (Schicht B) | 2,039 | 100 |
| | 2,020 | Reflex 3 | Al (Schicht B) | **$Cr_2O_3$:** | |
| | 2,019 | Reflex 4 | Al (Schicht B) | 2,665 | 100 |
| | 2,488 | Ring 1 | $Cr_2O_3$ (Schicht A/C) | 2,480 | 93 |

(fortgesetzt)

| Probe | d (Å) gemessen | Reflexcharakter | Zuordnung | Literaturwerte | |
|---|---|---|---|---|---|
| | | | | d (Å) | rel. Intensität |
| | 2,56 | Ring 1 | $Cr_2O_3$(Schicht A / C) | Al (Metall): | |
| | 2,072 | Ring 2 | Cr(Schicht A / C) | 2,338 | 100 |
| | 2,089 | Ring 2 | Cr(Schicht A 1 C) | 2,024 | 47 |
| Beispiel 19 | 2,349 | Reflex 1 | Al (Schicht A) | Al (Metall): | |
| | 2,339 | Reflex 2 | Al (Schicht A) | 2,338 | 100 |
| | 2,043 | Reflex 3 | Al (Schicht A) | 2,024 | 47 |

Bei Beispiel 18 konnten lediglich zwei Ringe und keine scharfen Reflexe detektiert werden. Dieses Ergebnis zeigt dafür, dass kristalline Strukturen in einem Größenbereich unterhalb von 10 nm vorliegen.

Beim Vergleich der aus der Lage der Ringe berechneten Gitterkonstanten mit den Literaturwerten für elementares Chrom sowie für $Cr_2O_3$ zeigt das Ring 1 $Cr_2O_3$ und Ring 2 metallischem Cr zugeordnet werden können. Mithin liegen in dieser einen Schicht, die ein Beispiel der Schichten A und/oder C der erfindungsgemäßen metallischen Effektpigmente darstellt, das abgeschiedene Cr sowohl in einer äußerst feinteiligen (nanometrischen) metallischen Form als auch einer Oxidform vor.

Hätte die Schicht einen rein oxidischen Charakter, so hätten keine Signale entsprechend des metallischen Chroms detektiert werden dürfen.

Dennoch ist diese Schicht im Sinne dieser Erfindung weitgehend homogen zusammengesetzt, da derartige Phasen, die auf einer Nanometerebene existieren, nicht mehr mit den anderen Methoden (EDX, XPS) aufgelöst werden können.

Offensichtlich sind das metallische Chrom und das oxidische Chrom auch weitgehend miteinander zu einer Schicht vermischt und liegen nicht etwas als zwei getrennte, aufeinander folgende Schichten vor, da man in diesem Fall mittels der anderen Meßmethode entsprechende Konzentrationsunterschiede detektiert hätte.

Bei Beispiel 7 wurden zusätzlich zu den Ringsignalen aus Beispiel 18 scharfe Reflexe detektiert, die sich dem metallischen Aluminium der mittleren Schicht B zuordnen lassen.

Bei Beispiel 19 wurden Reflexe erhalten, die sich eindeutig metallischem Aluminium zuordnen lassen. Im Beugungsbild waren jedoch auch schwache Ringstrukturen zu detektieren. Es wurden keine Signale gefunden, die kristallinen Aluminiumoxiden entsprochen hätten. Daher liegen hier offenbar metallische Aluminiumteilchen vor, die in einer amorphen Aluminiumoxidschicht eingebettet sind.

Die Größe dieser metallischen Aluminiumteilchen ist größer als die der Chromteilchen in Beispiel 18, da hier Reflexe neben Ringstrukturen vorliegen. Die Größe liegt da in einem Bereich von 10 bis 20 nm.

**Beispiele kosmetischer Formulierungen:**

**Erfindungsgemäßes Beispiel 25: Nagellackzusammensetzung**

Zuvor wird die Pigmentsuspension aus Beispiel 8 in Ethylacetat umgenetzt und auf 20 Gew.-%- Festkörpergehalt eingestellt.

**Tab. 7: Nagellackzusammensetzung**

| Nr. | Stoff | Konzentration in Gew.-% |
|---|---|---|
| 1 | Erfindungsgemäßes Beispiel 8: (20 Gew.-% Metalleffektpigment in Ethylacatat) | 20 |
| 2 | Hostaphat CS 120 * | 1 |
| 3 | Methylethylketon | 20 |
| 4 | Methylisobutylketon | 20 |
| 5 | CAB 381.2 | 9 |

(fortgesetzt)

| Nr. | Stoff | Konzentration in Gew.-% |
|---|---|---|
| 6 | Butylacetat 98/100 | 30 |
| * Stearylphosphorsäureester | | |

Herstellung:

Die Pigmentsuspension des erfindungsgemäßen Beispiels 8 wurde durch vorsichtiges Abdampfen von Aceton auf einen Pigmentgehalt von 25 Gew.-% gebracht. Das Butylacetat wird vorgelegt und das CAB-Pulver (Cellulose-Acetylbutyrat) unter Rühren portionsweise zugegeben.
Die Komponenten 2 - 4 werden nacheinander zu der Metallpigmentdispersion gegeben und schonend eingearbeitet.
Man erhält einen Nagellack, der nach Auftrag auf einen Fingernagel einen intensiven goldmetallischen Eindruck hinterlässt.

**Erfindungsgemäßes Beispiel 26: Lip Gloss**

Zuvor wird die Pigmentsuspension aus Beispiel 8 in Isononylnonanoat umgenetzt und auf 20 Gew.-%- Festkörpergehalt eingestellt.

| INCI Name | Product Name | %W/W | Supplier |
|---|---|---|---|
| A | | 100.00 | |
| Hydriertes Polyisobuten (und) Ethylen/Propylen/ Styrol-Copolymer (und) Butylen/Ethylen/Styrol-Copolymer | Versagel ME 750 | 74.10 | www penreco.com |
| Simmondsia Chinensis (Jojoba) Samenöl | Jojoba Oil - Natural/Golden | 2.00 | www.biochemica.com |
| Caprylyltrimethicon | Silcare Silicone 31M50 | 7.00 | www.clariant.com |
| Stearyldimethicon | Silcare Silicone 41 M65 | 3.20 | www.clariant.com |
| hydriertes Polydecen | Nexbase 2002 | 4.00 | www.jandekker.com |
| Isopropylmyristat | Isopropyl Myristate | 4.50 | www.vwr.com |
| B | | | |
| Erfindungsgemäßes Beispiel 8: (20 Gew.-% Metalleffektpigment in sononylnonanoat) | ---- | 5.00 | |
| Propylparaben | Propyl-4-hydroxybenzoat | 0.20 | www.sig.maaldrich.com |

**Herstellungsverfahren:**

1. Phase A wird auf 85°C erwärmt

2. Phase B wird zu Phase A hinzugefügt und gemischt, bis Homogenität erhalten wird

3. Befüllen eines Lip-Gloss-Gefäßes

Man erhält einen Lip Gloss, der nach Auftrag auf die Lippen einen goldmetallischen Eindruck hinterlässt.

**Erfindungsgemäßes Beispiel 27: Lip Stick**

| INCI Name | Product Name | %W/W | Supplier |
|---|---|---|---|
| A | | 100.00 | |
| Carnauba-Wachs | Ewacera 34 | 5.04 | www.wagnerlanolin.de |
| Bienenwachs | Ewacera 12 | 3.92 | www.wagnerlanolin.de |
| Candelilla-Wachs | Ewacera 42 | 4.48 | www.wagnerlanolin.de |
| Mikrokristallines Wachs | Parcera MW | 8.07 | www.paramelt.com |
| Cetylpalmitat | Walrath synthetic | 2.24 | www.kahlwax.de |
| Hydriertes Coco-Glyceride | Softisan 100 | 5.60 | www.sasolwax.com |
| Petrolatum | Penreco Blond | 6.50 | www.penreco.com |
| Cetearyloctanoat | Luvitol EHO | 11.99 | www.basf.com |
| Tocopherylacetat | D,L-Alpha-Tocopherolacetat | 0.56 | www.dsm.com |
| Castoröl | Castor Oil | 44.37 | www.riedeldehaen.com |
| B | | | |
| Erfindungsgemäßes Beispiel 8: (20 Gew.-% Metalleffektpigment in sononylnonanoat) | ---- | 7.00 | |
| Methylparaben, Propylparaben | Rokonsal SSH-1 | 0.22 | www.biochema.com |

**Herstellungsverfahren:**

    1. Phase A wird auf 85°C erwärmt
    2. Phase B wird zu Phase A gegeben und gemischt
    3. Befüllen einer Lippenstift-Form bei 75°C

Man erhält einen Lip Stick, der nach Auftrag auf die Lippen einen goldmetallischen Eindruck hinterlässt.

**Erfindungsgemäßes Beispiel 28: Flüssiger Eyeliner:**

| INCI Name | Product Name | %W/W | Supplier |
|---|---|---|---|
| A | | 100.00 | |
| Wasser | Aqua | 64.70 | |
| Wasser/ Ruß-Dispersion | MBD 201 | 3.00 | www.geotech.nl |
| Acrylatcopolymer | Covacryl E14 | 10.00 | www.lcw.fr |
| Magnesium Aluminium Silicat | Veequm HV | 1.00 | www.cherbsloeh.de |
| B | | | |
| Propyleneglycol | 1,2 Propandiol | 3.00 | www.vwr.com |
| Triethanolamin | Triethanolamine | 1.40 | www.vwr.com |

(fortgesetzt)

| C | | | |
|---|---|---|---|
| Xanthangummi | Keltrol T | 0.30 | |
| D | | | |
| Mica | Silk Mica | 2.00 | www.vwr.com |
| E | | | |
| Erfindungsgemäßes Beispiel 8: (20 Gew.-% Metalleffektpigment in sononylnonanoat) | ------ | 5.00 | |
| Stearinsäure | Kortacid 1895 | 2.80 | www.akzonobel.de |
| Glycerylstearat | Aldo MS K FG | 0.80 | www.lonza.com |
| Oleylalkohol | HD-Ocenol 90/95 V | 0.50 | www.biesterfeld.com |
| Phenoxyethanol (und) Methylparaben (und) Ethylparaben (und) Butylparaben | Uniphen P-23 | 0.50 | www.induchem.com |
| F | | | |
| Dimethicon (und) Trisiloxan | Dow Corning 2-1184 Fluid | 5.00 | www.dowchemicals.com |

**Herstellungsverfahren:**

1. Dispergieren von Veegum in Phase A
2. Rühren für 15 min.
3. Phase B wird zu Phase A gegeben
4. Phase C wird zu Phase AB gegeben
5. Rühren für 10 min.
6. Phase D wird zu Phase ABC gegeben und auf 75°C erwärmt
7. Phase E wird auf 75°C erwärmt
8. Phase E wird zu Phase ABCD gegeben
9. Abkühlen auf 60°C und zugeben von Phase F
10. Gießen in ein entsprechendes Behältnis

Man erhält einen Liquid Eyeliner, der nach Auftrag auf das Augenlid einen goldmetallischen Eindruck hinterlässt.

**Legende**

**Abbildung 3:**

1. Verdampfungsquelle
2. Präparierter Drehteller
3. Blende
4. Stationär angeordneter Schwingquarz
5. Vakuumkammer
6. Drehachse

**Abbildung 4a:**

1. Mit Releasecoat beschichtete Quellrolle
2. und 3. Umlenkrolle
4. Rollenaufnehmer
5. und 6. Tränsmissionsmessung
7. Schwingquarzmessung
8. Verdampferschiff
9. und 10. Blendeneingang und Blendenausgang

**Abbildung 4b:**

1. Mit Releasecoat beschichtete Quellrolle
2. und 3. Umlenkrolle
4. Rollenaufnehmer
5. und 6. Transmissionsmessung
7. Schwingquarzmessung
8. Verdampferschiff A, B und C
9., 10., 11. und 12. Trennwände

**Abbildung 9:**

1. Sauerstoffquelle 1
2. Sauerstoffquelle 2
3. Metallverdampfungsquelle

**Abbildung 15:**

1. Sauerstoffquelle
2. Metallverdampfungsquelle

**Bevorzugte Ausführungsformen der Erfindung**

Gemäß einer bevorzugten Ausführungsform des metallischen Effektpigmentes weisen die Schichten A und/ oder C eine weitgehend homogene chemische Zusammensetzung hinsichtlich Sauerstoff $O_A$ und/oder $O_c$ bzw. Metall $M_A$ und/oder $M_c$ auf.

Gemäß einer bevorzugten Ausführungsform des metallischen Effektpigmentes weisen die Schichten A und C des metallischen Effektpigmentes einen durchschnittlichen Gehalt an Sauerstoff $O_{AC}$ von 30 bis 57 Atom-% auf.

Gemäß einer bevorzugten Ausführungsform des metallischen Effektpigmentes weisen die Schichten A und C des metallischen Effektpigmentes einen durchschnittlichen Gehalt an Sauerstoff $O_{AC}$ von 35 bis 56 Atom-% auf.

Gemäß einer bevorzugten Ausführungsform des metallischen Effektpigmentes beträgt in den Schichten A und/ oder C des metallischen Effektpigmentes der Gesamtgehalt an $M_A$ und an $O_A$ 90 bis 100 Atom-%, bezogen auf alle Komponenten der Schicht A, bzw. der Gesamtgehalt an $M_C$ und an $O_C$ 90 bis 100 Atom-%, bezogen auf alle Komponenten der Schicht C.

Gemäß einer bevorzugten Ausführungsform des metallischen Effektpigmentes besitzen die Schicht A und/ oder C unabhängig voneinander eine durchschnittliche Dicke von 10 bis 250 nm.

Gemäß einer bevorzugten Ausführungsform des metallischen Effektpigmentes ist das wenigstens eine Metall $M_B$ aus der Gruppe, bestehend aus Aluminium, Chrom, Silber, Kupfer, Gold, Zink, Zinn, Mangan, Eisen, Kobalt, Nickel, Titan, deren Mischungen und deren Legierungen, ausgewählt.

Gemäß einer bevorzugten Ausführungsform des metallischen Effektpigmentes nach Anspruch 4 hat die Schicht B einen metallischen Charakter und besitzt eine durchschnittliche Dicke von 10 bis 200 nm.

Gemäß einer bevorzugten Ausführungsform des metallischen Effektpigmentes nach Anspruch 5 hat die Schicht B einen weitgehend metallischen oder oxidischen Charakter und besitzt eine durchschnittliche Dicke von 50 bis 2000 nm.

Gemäß einer bevorzugten Ausführungsform des metallischen Effektpigmentes nach einem der Ansprüche 1 bis 5 besitzt das gesamte metallische Effektpigment eine durchschnittliche Dicke von 30 bis 550 nm.

Gemäß einer bevorzugten Ausführungsform des metallischen Effektpigmentes nach einem der Ansprüche 1 bis 5 besitzt das metallische Effektpigment eine durchschnittliche Dicke von 50 bis 300 nm.

Gemäß einer bevorzugten Ausführungsform sind die Metalle $M_A$, $M_B$ und $M_c$ gleich.

Gemäß einer bevorzugten Ausführungsform des metallischen Effektpigmentes sind die mittleren Schichtdicken der Schichten A und C im Wesentlichen gleich.

Gemäß einer bevorzugten Ausführungsform des metallischen Effektpigmentes sind die Schichten A, B und C unmittelbar aufeinander folgend angeordnet.

Gemäß einer bevorzugten Ausführungsform des metallischen Effektpigmentes ist das metallische Effektpigment mit einer Korrosionsschutzschicht, vorzugsweise umhüllend, beschichtet.

Gemäß einer bevorzugten Ausführungsform des metallischen Effektpigmentes umfasst die, vorzugsweise umhüllende, Korrosionsschutzschicht $SiO_2$ oder besteht aus $SiO_2$.

Gemäß einer bevorzugten Ausführungsform des Verfahrens zur Herstellung des erfindungsgemäßen metallischen Effektpigmentes ist die wenigstens eine Sauerstoffquelle in Form von Wasser, Wasser-abgebenden Substanzen, Sauerstoff-abgebenden Substanzen und/oder Sauerstoffgas in der Vakuumkammer angeordnet.

Gemäß einer bevorzugten Ausführungsform des Verfahrens zur Herstellung eines metallischen Effektpigmentes erfolgt während Schritt a) und Schritt c) und optional während Schritt b) eine kontrollierte Zudosierung von Sauerstoffgas in die Vakuumkammer.

Gemäß einer bevorzugten Ausführungsform des Beschichtungsmittels enthält das Beschichtungsmittel erfindungsgemäße metallische Effektpigmente.

**Patentansprüche**

1. Metallisches Effektpigment,
   **dadurch gekennzeichnet,**
   **dass** das metallische Effektpigment plättchenförmig ist und wenigstens drei Schichten umfasst:

   A) eine Schicht A, welche wenigstens ein Metall $M_A$ und einen durchschnittlichen Gehalt an Sauerstoff $O_A$, bezogen auf den Gesamtgehalt an $M_A$ und $O_A$ in der Schicht A aufweist,
   B) eine Schicht B mit wenigstens einem Metall $M_B$ und einem durchschnittlichen Gehalt an Sauerstoff $O_B$ von 0 bis 77 Atom-%, insbesondere von 0 bis 58 Atom-%, bezogen auf den Gesamtgehalt an $M_B$ und $O_B$ in der Schicht B,
   C) eine Schicht C, welche wenigstens ein Metall $M_c$ und einen durchschnittlichen Gehalt an Sauerstoff $O_c$, bezogen auf den Gesamtgehalt an $M_c$ und $O_c$ in der Schicht C, aufweist,

   wobei der durchschnittliche Sauerstoffgehalt $O_{AC}$ in den Schichten A und C gemäß der Formel (1)

   $$O_{AC} = \frac{1}{2}\left( \frac{O_A}{M_A + O_A} + \frac{O_C}{M_C + O_C} \right) \qquad (\text{I})$$

   bestimmt wird und in einem Bereich von 2 bis 77 Atom-%, insbesondere von 25 bis 58 Atom-%, liegt, wobei die Schichten A und C keine reinen stöchiometrischen Oxidschichten sind.

2. Metallisches Effektpigment nach Anspruch 1,
   **dadurch gekennzeichnet**, . ' _ _
   dass der durchschnittliche Gehalt an Sauerstoff $O_A$, bezogen auf den Gesamtgehalt an $M_A$ und $O_A$ in der Schicht A, sowie der durchschnittliche Gehalt an Sauerstoff $O_c$, bezogen auf den Gesamtgehalt an $M_C$ und $O_C$ in der Schicht C, unabhängig voneinander jeweils in einem Bereich von 25 bis 58 Atom-%, bevorzugt von 30 bis 57 Atom-%, liegt.

3. Metallisches Effektpigment nach einem der vorstehenden Ansprüche,
   **dadurch gekennzeichnet,**
   **dass** das wenigstens eine Metall $M_A$ und/oder $M_c$ aus der Gruppe, bestehend aus Aluminium, Magnesium, Chrom, Silber, Kupfer, Gold, Zink, Zinn, Mangan, Eisen, Kobalt, Nickel, Titan, Tantal, Molybdän, deren Mischungen und deren Legierungen, ausgewählt ist.

**4.** Metallisches Effektpigment nach einem der vorstehenden Ansprüche,
**dadurch gekennzeichnet,**
**dass** der durchschnittliche Gehalt an Sauerstoff $O_B$, bezogen auf den Gesamtgehalt an $M_B$ und $O_B$ in der Schicht B in einem Bereich von 0 bis unter 25 Atom-% liegt.

**5.** Metallisches Effektpigment nach einem der Ansprüche 1 bis 3,
**dadurch gekennzeichnet,**
**dass** der durchschnittliche Gehalt an Sauerstoff $O_B$, bezogen auf den Gesamtgehalt an Mg und $O_B$ in der Schicht B bei 25 bis 58 Atom-% liegt.

**6.** Metallisches Effektpigment nach einem der vorstehenden Ansprüche,
**dadurch gekennzeichnet,**
**dass** das Metall $M_B$ Aluminium und/oder Silber ist.

**7.** Metallisches Effektpigment nach einem der vorstehenden Ansprüche,
**dadurch gekennzeichnet,**
**dass** das Metall $M_A$ und $M_C$ gleich sind.

**8.** Metallisches Effektpigment nach einem der vorstehenden Ansprüche,
**dadurch gekennzeichnet,**
**dass** das Metall $M_A$ und/oder $M_C$ im wesentlichen Chrom ist und in der Schicht A und/oder C unabhängig voneinander der durchschnittliche Gehalt an Sauerstoff $O_A$ bzw. $O_c$ im Bereich von 35 bis 48 Atom-%, bezogen auf den jeweiligen Gesamtgehalt an Chrom und Sauerstoff in der Schicht A bzw. C, liegt.

**9.** Metallisches Effektpigment nach einem der Ansprüche 1 bis 7,
**dadurch gekennzeichnet,**
**dass** das Metall $M_A$ und/oder $M_C$ im wesentlichen Aluminium ist und in der Schicht A und/oder C unabhängig voneinander der durchschnittliche Gehalt an Sauerstoff $O_A$ bzw. $O_c$ im Bereich von 30 bis 55 Atom-%, bezogen auf den jeweiligen Gesamtgehalt an Aluminium und Sauerstoff in der Schicht A bzw. C, liegt.

**10.** Verfahren zur Herstellung eines metallischen Effektpigmentes nach einem der Ansprüche 1 bis 9,
**dadurch gekennzeichnet,**
**dass** die einzelnen Schichten A, B und C durch PVD-Verfahren nacheinander durch Aufdampfen vom $M_A$, $M_B$ und $M_C$ angeordnet werden, wobei zumindest die Schichten A und C in Gegenwart wenigstens einer Sauerstoff-abgebenden Sauerstoffquelle aufgedampft werden, wobei das Verfahren folgende Schritte umfasst:

a) Bedampfen eines, vorzugsweise beweglichen, Substrates in einer Vakuumkammer mittels physical vapour deposition (PVD) mit wenigstens einem Metall $M_A$ in Gegenwart von Sauerstoff unter Bildung der Schicht A auf dem Substrat,
b) Bedampfen der Schicht A in einer Vakuumkammer mittels physical vapour deposition (PVD) mit wenigstens einem Metall $M_B$ in Gegenwart oder Abwesenheit von Sauerstoff unter Bildung der Schicht B,
c) Bedampfen der Schicht B in einer Vakuumkammer mittels physical vapour deposition (PVD) mit wenigstens einem Metall $M_c$ In Gegenwart von Sauerstoff unter Bildung der Schicht C,
d) Ablösen des metallischen Schichtpaketes vom Substrat,
e) Zerkleinern des metallischen Schichtpaketes zu metallischen Effektpigmenten,
f) optional Überführen der metallischen Effektpigmente in eine Dispersion oder Paste.

**11.** Verfahren zur Herstellung eines metallischen Effektpigmentes nach Anspruch 10,
**dadurch gekennzeichnet,**
**dass** das Verfahren folgende Schritte umfasst:

a) Bedampfen eines, vorzugsweise beweglichen, Substrates, vorzugsweise eines umlaufenden oder sich bewegenden Bandes, in einer Vakuumkammer mit wenigstens dem Metall $M_A$ aus einer Verdampferquelle $VQ_A$ in Gegenwart einer Sauerstoff-abgebenden Sauerstoffquelle unter Bildung der Schicht A,
b) Bedampfen der Schicht A auf dem, vorzugsweise beweglichen, Substrat, vorzugsweise eines umlaufenden oder sich bewegenden Bandes, in einer Vakuumkammer mit wenigstens dem Metall $M_B$ aus einer Verdampferquelle $VQ_B$ in Gegenwart oder Abwesenheit einer Sauerstoffquelle unter Bildung der Schicht B,
c) Bedampfen der Schicht B auf dem, vorzugsweise beweglichen, Substrat, vorzugsweise eines umlaufenden oder sich bewegenden Bandes, in einer Vakuumkammer mit wenigstens dem Metall $M_C$ aus einer Verdampferquelle $VQ_C$ in Gegenwart einer Sauerstoffquelle unter Bildung der Schicht C,
d) Ablösen des metallischen Schichtpaketes von dem, vorzugsweise beweglichen, Substrat, vorzugsweise umlaufenden oder sich bewegenden Bandes,
e) Zerkleinern des metallischen Schichtpaketes zu metallischen Effektpigmenten,
f) optional Überführen der metallischen Effektpigmente in eine Dispersion oder Paste.

**12.** Verfahren zur Herstellung eines metallischen Effektpigmentes nach Anspruch 11,
**dadurch gekennzeichnet,**
**dass** die einzelnen Verdampferquellen $VQ_A$, $VQ_B$ und $VQ_C$ voneinander oder jeweils paarweise voneinander abgetrennt sind.

**13.** Verfahren zur Herstellung eines metallischen Effektpigmentes nach einem der Ansprüche 10 bis 12,
**dadurch gekennzeichnet,**
**dass** $M_A$, $M_B$ und $M_C$ gleich, paarweise gleich oder verschieden voneinander sind und aus einer Metallverdampfungsquelle oder mehreren Metallverdampfungsquellen ein bewegliches, Substrat, vorzugsweise ein umlaufendes oder sich bewegendes Band, in einer Vakuumkammer in Gegenwart von einer oder mehreren Sauerstoff-abgebenden Sauerstoffquellen mit Metall bedampft wird, wobei sich zwischen der Metallverdampfungsquelle, der Sauerstoffquelle und dem beweglichen Substrat dreidimensionale Konzentrationsbereiche aus Metalldampf und Sauerstoff in der Vakuumkammer ausbilden, wodurch auf dem beweglichen Substrat durch physikalische Dampfabscheidung die wenigstens drei Schichten A, B und C mit voneinander unterscheidbaren, Metall- und Sauerstoffgehalten nacheinander abgeschieden werden.

**14.** Verfahren zur Herstellung eines metallischen Effektpigmentes nach Anspruch 13,
**dadurch gekennzeichnet,**
**dass** zwischen Metallverdampfungsquelle, Sauerstoffquelle und beweglichem Substrat jeweils eine Abdeckvorrichtung oder mehrere Abdeckvorrichtungen angeordnet sind, durch die die mögliche Ausbildung von Übergangsschichten zwischen den Schichten A, B und C unterdrückt werden, so dass die wenigstens drei aufeinanderfolgenden Schichten A, B und C mit jeweils voneinander unterscheidbaren Gehalten an Metall und Sauerstoff abgeschieden werden.

**15.** Verwendung der metallischen Effektpigmente nach einem der Ansprüche 1 bis 9 in Coatings, Lacken, Automobillacken, Pulverlacken, Druckfarben, Digitaldruckfarben, Kunststoffen oder kosmetischen Formulierungen.

**16.** Beschichtungsmittel,
**dadurch gekennzeichnet,**
**dass** das Beschichtungsmittel metallische Effektpigmente nach einem der Ansprüche 1' bis 9 enthält.

**17.** Beschichteter Gegenstand,
dadurch gekenntzeichnet
dass der Gegenstand mit metallischen Effektpigmenten nach einem der Ansprüche 1 bis 9 oder mit einem Beschichtungsmittel nach Anspruch 16 versehen ist.

**Claims**

**1.** Metallic effect pigment,
**characterised in that**
the metallic effect pigment is platelet shaped and comprises at least three layers:

A) a layer A containing at least one metal $M_A$ and an average content of oxygen $O_A$ relative to the total content of $M_A$ and $O_A$ in layer A,
B) a layer B with at least one metal $M_B$ and an average content of oxygen $O_B$ of 0 to 77 atom %, in particular 0 to 58 atom %, relative to the total content of $M_B$ and $O_B$ in layer B,
C) a layer C containing at least one metal $M_C$ and an average content of oxygen $O_C$ relative to the total content of $M_C$ and $O_C$ in layer C,

and the average oxygen content $O_{AC}$ in layers A and C is determined on the basis of formula (I)

$$O_{AC} = \frac{1}{2}\left(\frac{O_A}{M_A + O_A} + \frac{O_C}{M_C + O_C}\right) \quad \text{(I)}$$

and lies within a range of from 2 to 77 atom %, in particular 25 to 58 atom %, and layers A and C are not purely stoichiometric oxide layers.

2. Metallic effect pigment as claimed in claim 1,
   **characterised in that**
   the average content of oxygen $O_A$ relative to the total content of $M_A$ and $O_A$ in layer A, as well as the average content of oxygen $O_C$ relative to the total content of $M_C$ and $O_C$ in layer C lies, independently of one another, within a range of from 25 to 58 atom %, preferably 30 to 57 atom %.

3. Metallic effect pigment as claimed in one of the preceding claims,
   **characterised in that**
   the at least one metal $M_A$ and/or $M_C$ is selected from the group comprising aluminium, magnesium, chromium, silver, copper, gold, zinc, tin, manganese, iron, cobalt, nickel, titanium, tantalum, molybdenum, mixtures thereof and alloys thereof.

4. Metallic effect pigment as claimed in one of the preceding claims,
   **characterised in that**
   the average content of oxygen $O_B$ relative to the total content of $M_B$ and $O_B$ in layer B lies within a range of from 0 to less than 25 atom %.

5. Metallic effect pigment as claimed in one of claims 1 to 3,
   **characterised in that**
   the average content of oxygen $O_B$ relative to the total content of $M_B$ and $O_B$ in layer B lies between 25 and 58 atom %.

6. Metallic effect pigment as claimed in one of the preceding claims,
   **characterised in that**
   metal $M_B$ is aluminium and/or silver.

7. Metallic effect pigment as claimed in one of the preceding claims,
   **characterised in that**
   metals $M_A$ and $M_C$ are the same.

8. Metallic effect pigment as claimed in one of the preceding claims,
   **characterised in that**
   metal $M_A$ and/or $M_C$ is essentially chromium and, independently of one another, the average content of oxygen $O_A$ respectively $O_C$ in layer A and/or C lies within the range of 35 to 48 atom % relative to the respective total content of chromium and oxygen in layer A respectively C.

9. Metallic effect pigment as claimed in one of claims 1 to 7,
   **characterised in that**
   metal $M_A$ and/or $M_C$ is essentially aluminium and, independently of one another, the average content of oxygen $O_A$ respectively $O_C$ in layer A and/or C lies within a range of from 30 to 55 atom % relative to the respective total content of aluminium and oxygen in layer A respectively C.

10. Method of producing a metallic effect pigment as claimed in one of claims 1 to 9,
    **characterised in that**
    the individual layers A, B and C are applied in succession by vapour deposition of $M_A$, $M_B$ and $M_C$, and at least layers A and C are vapour deposited in the presence of at least one oxygen-donating oxygen source, and the method comprises the following steps:

    a) applying vapour to a preferably moving substrate in a vacuum chamber by means of physical vapour deposition (PVD) with at least one metal $M_A$ in the presence of oxygen to form layer A on the substrate,
    b) applying vapour to layer A in a vacuum chamber by means of physical vapour deposition (PVD) with at least one metal $M_B$ in the presence or absence of oxygen to form layer B,

c) applying vapour to layer B in a vacuum chamber by means of physical vapour deposition (PVD) with at least one metal $M_C$ in the presence of oxygen to form layer C,

d) detaching the metallic layer stack from the substrate,

e) grinding the metallic layer stack to form metallic effect pigments,

f) optionally converting the metallic effect pigments into a dispersion or paste.

**11.** Method of producing a metallic effect pigment as claimed in claim 10,
**characterised in that**
the method comprises the following steps:

a) applying vapour to a preferably moving substrate, preferably a circulating or moving belt, in a vacuum chamber with at least metal $M_A$ from a vaporiser source $VQ_A$ in the presence of an oxygen-donating oxygen source to form layer A,

b) applying vapour to layer A on the preferably moving substrate, preferably a circulating or moving belt, in a vacuum chamber with at least metal $M_B$ from a vaporiser source $VQ_B$ in the presence or absence of an oxygen-donating source to form layer B,

c) applying vapour to layer B on the preferably moving substrate, preferably a circulating or moving belt, in a vacuum chamber with at least metal $M_C$ from a vaporiser source $VQ_C$ in the presence of an oxygen source to form layer C,

d) detaching the metallic layer stack from the preferably moving substrate, preferably a circulating or moving belt,

e) grinding the metallic layer stack to form metallic effect pigments,

f) optionally converting the metallic effect pigments into a dispersion or paste.

**12.** Method of producing a metallic effect pigment as claimed in claim 11,
**characterised in that**
the individual vaporiser sources $VQ_A$, $VQ_B$ and $VQ_C$ are separate from one another or arranged in respective pairs.

**13.** Method of producing a metallic effect pigment as claimed in one of claims 10 to 12,
**characterised in that**
$M_A$, $M_B$ and $M_C$ are the same, the same based on pairs or different from one another and are vaporised from a metal vaporiser source or several metal vaporiser sources and deposited on a moving substrate, preferably a circulating or moving belt, in a vacuum chamber in the presence of one or more oxygen-donating oxygen sources with metal, and three-dimensional areas of concentrated metal vapour and oxygen form in the vacuum chamber between the metal vaporiser source, oxygen source and moving substrate, as a result of which the at least three layers A, B and C with mutually variable metal and oxygen contents are deposited in succession on the moving substrate by physical vapour deposition.

**14.** Method of producing a metallic effect pigment as claimed in claim 13,
**characterised in that**
a cover device or several cover devices is or are disposed respectively between the metal vaporiser source, oxygen source and moving substrate, by means of which the possible formation of transition layers between layers A, B and C can be prevented so that the at least three consecutive layers A, B and C are each deposited with mutually variable contents of metal and oxygen.

**15.** Use of the metallic effect pigments as claimed in one of claims 1 to 9 in coatings, varnishes, automobile paints, powder coatings, printing inks, digital printing inks, plastics or cosmetic formulations.

**16.** Coating substance,
**characterised in that**
the coating substance contains metallic effect pigments as claimed in one of claims 1 to 9.

**17.** Coated object,
**characterised in that**
the object is coated with metallic effect pigments as claimed in one of claims 1 to 9 or with a coating substance as claimed in claim 16.

**Revendications**

1. Pigment métallisé à effet, **caractérisé en ce que**
le pigment métallisé à effet est en forme de plaquettes et comprend au moins trois couches :

   A) une couche A, laquelle comporte au moins un métal $M_A$ et présente une teneur moyenne en oxygène $O_A$, rapportée à la teneur totale en $M_A$ et $O_A$ dans la couche A,
   B) une couche B, avec au moins un métal $M_B$ et une teneur moyenne en oxygène $O_B$ de 0 à 77 % en atomes, en particulier de 0 à 58 % en atomes, rapportée à la teneur totale en $M_B$ et $O_B$ dans la couche B,
   C) une couche C, laquelle comporte au moins un métal $M_C$ et présente une teneur moyenne en oxygène $O_C$, rapportée à la teneur totale en $M_C$ et $O_C$ dans la couche C,

   sachant que la teneur moyenne en oxygène $O_{AC}$ dans les couches A et C est déterminée d'après la formule (I)

   $$O_{AC} = \frac{1}{2}\left( \frac{O_A}{M_A + O_A} + \frac{O_C}{M_C + O_C} \right) \qquad (I)$$

   et est comprise dans un intervalle de 2 à 77 % en atomes, en particulier de 25 à 58 % en atomes, les couches A et C n'étant pas des couches d'oxyde purement stoechiométriques.

2. Pigment métallisé à effet selon la revendication 1,
**caractérisé en ce que**
la teneur moyenne en oxygène $O_A$, rapportée à la teneur totale en $M_A$ et $O_A$ dans la couche A, ainsi que la teneur moyenne en oxygène $O_C$, rapportée à la teneur totale en $M_C$ et $O_C$ dans la couche C, sont comprises respectivement, indépendamment l'une de l'autre, dans un intervalle de 25 à 58 % en atome, de préférence de 30 à 57 % en atome.

3. Pigment métallisé à effet selon l'une quelconque des revendications précédentes,
**caractérisé en ce que**
ledit au moins un métal $M_A$ et/ou $M_C$ est choisi parmi le groupe constitué de l'aluminium, du magnésium, du chrome, de l'argent, du cuivre, de l'or, du zinc, de l'étain, du manganèse, du fer, du cobalt, du nickel, du titane, du tantale, du molybdène, de leur mélanges et de leurs alliages.

4. Pigment métallisé à effet selon l'une quelconque des revendications précédentes,
**caractérisé en ce que**
la teneur moyenne en oxygène $O_B$, rapportée à la teneur totale en $M_B$ et en $O_B$ dans la couche B, est comprise dans un intervalle de 0 à moins de 25 % en atome.

5. Pigment métallisé à effet selon l'une quelconque des revendications 1 à 3,
**caractérisé en ce que**
la teneur moyenne en oxygène $O_B$, rapportée à la teneur totale en $M_B$ et en $O_B$ dans le couche B, est de 25 à 58 % en atome.

6. Pigment métallisé à effet selon l'une quelconque des revendications précédentes,
**caractérisé en ce que**
le métal $M_B$ est de l'aluminium et/ou de l'argent.

7. Pigment métallisé à effet selon l'une quelconque des revendications précédentes,
**caractérisé en ce que**
les métaux $M_A$ et $M_C$ sont les mêmes.

8. Pigment métallisé à effet selon l'une quelconque des revendications précédentes,
**caractérisé en ce que**
le métal $M_A$ et/ou $M_C$ est essentiellement du chrome et **en ce que** dans la couche A et/ou C, la teneur moyenne en oxygène $O_A$, respectivement $O_C$, indépendamment l'une de l'autre, se trouve dans l'intervalle de 35 à 48 % en atome, rapportée à la teneur respective totale en chrome et en oxygène dans les couches A, respectivement C.

**9.** Pigment métallisé à effet selon l'une quelconque des revendications 1 à 7,
**caractérisé en ce que**
le métal $M_A$ et/ou $M_C$ est essentiellement de l'aluminium et **en ce que** dans les couches A et/ou C, les teneurs moyennes en oxygène $O_A$, respectivement $O_C$, sont comprises indépendamment l'une de l'autre dans l'intervalle de 30 à 55 % en atome, rapportées à la teneur respective totale en aluminium et en oxygène dans les couches A, respectivement C.

**10.** Procédé de fabrication d'un pigment métallisé à effet selon l'une quelconque des revendications 1 à 9,
**caractérisé en ce que**
les différentes couches A, B et C sont disposées l'une après l'autre par dépôt de $M_A$, $M_B$ et $M_C$ depuis la phase vapeur moyennant un procédé PVD, lors de quoi au moins les couches A et C sont déposées depuis la phase vapeur en présence d'au moins une source d'oxygène dégageant de l'oxygène, le procédé comprenant les étapes suivantes :

a) dépôt depuis la phase vapeur sur un substrat, de préférence mobile, dans une chambre à vide par dépôt physique depuis la phase vapeur (PVD), d'au moins un métal $M_A$ en présence d'oxygène moyennant la formation de la couche A sur le substrat,
b) dépôt sur la couche A depuis la phase vapeur, dans une chambre à vide par dépôt physique depuis la phase vapeur (PVD), d'au moins un métal $M_B$ en présence ou en l'absence d'oxygène moyennant la formation de la couche B,
c) dépôt sur la couche B depuis la phase vapeur, dans une chambre à vide par dépôt physique depuis la phase vapeur (PVD), d'au moins un métal $M_C$ en présence d'oxygène moyennant la formation de la couche C,
d) décollement du pack de couches métalliques depuis le substrat,
e) fragmentation du pack de couches métalliques pour donner des pigments métallisés à effet,
f) en option, transfert des pigments métallisés à effet dans une dispersion ou dans une pâte.

**11.** Procédé de fabrication d'un pigment métallisé à effet selon la revendication 10,
**caractérisé en ce que**
le procédé comprend les étapes suivantes:

a) dépôt depuis la phase vapeur sur un substrat, de préférence mobile, de préférence un ruban en circuit fermé ou en mouvement, dans une chambre à vide, d'au moins un métal $M_A$ provenant d'une source d'évaporation $VQ_A$ en présence d'une source d'oxygène dégageant de l'oxygène moyennant la formation de la couche A,
b) dépôt depuis la phase vapeur sur la couche A sur le substrat, de préférence mobile, de préférence un ruban en circuit fermé ou en mouvement, dans une chambre à vide, d'au moins un métal $M_B$ provenant d'une source d'évaporation $VQ_B$ en présence ou en l'absence d'une source d'oxygène moyennant la formation de la couche B,
c) dépôt depuis la phase vapeur sur la couche B, sur le substrat, de préférence mobile, de préférence un ruban en circuit fermé ou en mouvement, dans une chambre à vide, d'au moins un métal $M_C$ provenant d'une source d'évaporation $VQ_C$ en présence d'une source d'oxygène moyennant la formation de la couche C,
d) décollement du pack de couches métalliques depuis le substrat, de préférence mobile, de préférence un ruban en circuit fermé ou en mouvement,
e) fragmentation du pack de couches métalliques pour donner des pigments métallisés à effet,
f) en option, transfert des pigments métallisés à effet dans une dispersion ou dans une pâte.

**12.** Procédé de fabrication d'un pigment métallisé à effet selon la revendication 11,
**caractérisé en ce que**
les différentes sources de vapeur $VQ_A$, $VQ_B$ et $VQ_C$ sont séparées les unes des autres ou respectivement par paires les unes des autres.

**13.** Procédé de fabrication d'un pigment métallisé à effet selon l'une quelconque des revendications 10 à 12,
**caractérisé en ce que**
$M_A$, $M_B$ et $M_C$ sont identiques, que deux sont identiques, ou sont différents les uns des autres et **en ce qu'**un métal est déposé depuis la phase vapeur depuis une source d'évaporation de métal ou plusieurs sources d'évaporation de métal, sur un substrat mobile, de préférence un ruban en circuit fermé ou en mouvement, dans une chambre à vide, en présence d'une ou de plusieurs sources d'oxygène dégageant de l'oxygène, lors de quoi il se forme dans la chambre à vide entre la source d'évaporation de métal, la source d'oxygène et le substrat mobile des zones tridimensionnelles de concentration en vapeur métallique et en oxygène, moyennant quoi lesdites au moins trois couches A, B et C, avec des teneurs en métal et en oxygène distinctes les unes des autres, sont déposées les unes après les autres sur le substrat mobile par dépôt physique depuis la vapeur.

14. Procédé de fabrication d'un pigment métallisé à effet selon la revendication 13,
**caractérisé en ce que**
entre la source d'évaporation de métal, la source d'oxygène et le substrat mobile sont disposés respectivement un dispositif de recouvrement ou plusieurs dispositifs de recouvrement, par lesquels la formation possible de couches de transition entre les couches A, B et C est inhibée, de telle sorte que lesdites au moins trois couches successives A, B et C sont déposées respectivement avec des teneurs en métal et en oxygène différentes les unes des autres.

15. Utilisation des pigments métallisés à effet selon l'une quelconque des revendications 1 à 9 dans des enduits, des peintures, des peintures d'automobile, des peintures en poudre, des encres d'imprimerie, des encres d'imprimantes numériques, des plastiques ou des formulations cosmétiques.

16. Produit de revêtement,
**caractérisé en ce que**
le produit de revêtement contient un pigment métallisé à effet selon l'une quelconque des revendications 1 à 9.

17. Objet revêtu,
**caractérisé en ce que**
l'objet est doté de pigments métallisés à effet selon l'une quelconque des revendications 1 à 9 ou d'un produit de revêtement selon la revendication 16.

**Abbildung 1**

**Elektronenstrahl 2 kV** **Elektronenstrahl 2,8 kV** **Elektronenstrahl 3,4 kV**

A

B

C

40 nm 100 nm 140 nm

**Abbildung 2**

**Abbildung 3**

**Abbildung 4a**

**Abbildung 4b**

| Freie Beschichtungs-fläche des Bandes zur Erzeugung der Bandabfolge | Präpariertes Band zur Erzeugung der Einzelschichten | Erzeugte Bandsequenz |
|---|---|---|
| | Erzeugung Schicht A | Schicht A |
| | Erzeugung Schicht B | Schicht A+B |
| | Erzeugung Schicht C | Schicht A+B+C |

Bandrichtung →

Freie Beschichtungsfläche des Bandabschnitts

Abgedeckte Beschichtungsfläche des Bandabschnitts

Erzeugte Bandsequenzen

**Abbildung 5**

Schichtdicken-Sauerstoffprofil (EDX-Methode)

Abbildung 6

Schichtdicken-Sauerstoffprofil (ESCA-Methode)

Abbildung 7

Abbildung 8

**Abbildung 9**

Blendeneingang

Blende
I

Blende
II

Blendenausgang

Schicht
A

Schicht
B

Schicht
C

Bandrichtung

**Abbildung 10**

**Abbildung 11**

Bandrichtung

## Farbmessungen: a*,b*- Werte
### (Messungen im Rakelabzug unter verschiedenen
### Differenzenwinkeln von 15° bis 110°)

| | | | |
|---|---|---|---|
| ◆ Beispiel 1 | ▲ Beispiel 2 | ● Beispiel 3 |
| ■ Beispiel 4 | ··+·· Beispiel 5 | ··+·· Beispiel 6 |
| Beispiel 8 | ✕ Beispiel 11 | –◇–·Vgl.- Beispiel 16 |

Abbildung 12

EP 2 176 359 B1

## Farbmessung: C*-Werte
### (Messungen im Rakelabzug unter verschiedenen Differenzwinkeln von 15° bis 110°)

**Abbildung 13**

Farbmessung: L*-Werte

(Messungen im Rakelabzug unter verschiedenen Differenzwinkeln von 15° bis 110°)

L*-Werte

Differenzwinkel [°]

15  25  35  45  55  65  75  85  95  105

160  140  120  100  80  60  40  20  0

■— Beispiel 7
●— Beispiel 8
··△·· Vgl.- Beispiel 15
·□· Vgl.- Beispiel 14
◇ Vgl.- Beispiel 16
○ Vgl.- Beispiel 17

Abbildung 14

**Abbildung 15**

1. Sauerstoffquelle 1
2. Metallverdampfungsquelle

| Blenden-eingang | | Blende I | Schicht B | Blende II | | Blenden-ausgang |

Schicht A

Bandrichtung

**Abbildung 16**

**metallische Zone I Bildung Schicht A**

**Zentrale oxidischere metallische Zone Bildung Schicht B**

**metallische Zone II Bildung Schicht C**

Blende I

Blende II

Bandrichtung

**Abbildung 17**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2839378 A
- US 2941894 A
- US 4321087 A
- WO 2004026971 A
- WO 2004026972 A
- US 3438796 A
- US 5571624 A
- DE 102004063433 A1
- WO 2004052999 A2
- EP 1522606 A1
- US 4430366 A
- WO 9935194 A
- EP 0259592 B1

**In der Beschreibung aufgeführte Nicht-Patentliteratur**

- Pigment Handbook. Wiley-Interscience, 1973, vol. 1, 807 ff
- **A.B.J. RODRIGUEZ.** *JOCCA,* 1992, vol. 4, 150-153